# EUROPÄISCHE PATENTSCHRIFT

(11) **EP 0 799 238 B1**
(45) Veröffentlichungstag und Bekanntmachung des Hinweises auf die Patenterteilung: **24.05.2000**
(21) Anmeldenummer: 95943195.8
(22) Anmeldetag: 23.12.1995
(51) Int. Cl.: C07J 53/00, C07J 71/00, A61K 31/56, A61K 31/58

(54) **14ALPHA,17ALPHA-C2-ÜBERBRÜCKTE 19-NOR-PROGESTERONDERIVATE**
14ALPHA,17ALPHA-C2-BRIDGED 19-NOR-PROGESTERONE DERIVATIVES
DERIVES DE 19-NOR-PROGESTERONE A PONTAGE 14ALPHA,17ALPHA-C2

(30) Priorität: 23.12.1994 DE 4447401
(43) Veröffentlichungstag der Anmeldung: 08.10.1997
(73) Patentinhaber: SCHERING AKTIENGESELLSCHAFT, 13342 Berlin (DE)
(72) Erfinder: SCHÖLLKOPF, Klaus, D-14129 Berlin (DE); HALFBRODT, Wolfgang, D-13505 Berlin (DE); KUHNKE, Joachim, D-14089 Berlin (DE); SCHWEDE, Wolfgang, D-13467 Berlin (DE); FRITZEMEIER, Karl-Heinrich, D-13505 Berlin (DE); KRATTENMACHER, Rolf, D-13467 Berlin (DE); MUHN, Hans-Peter, D-13465 Berlin (DE)
(86) Internationale Anmeldenummer: EP9505107
(87) Internationale Veröffentlichungsnummer: WO9620209

(56) Entgegenhaltungen:
- STEROIDS: STRUCTURE, FUNCTION, AND REGULATION, Bd. 18, Nr. 3, September 1971, MA US, Seiten 251-259, XP000569729 A. J. SOLO ET AL: "Ring-D-bridged steroid analogs. IX. 19-nor-14alpha,17alpha-ethano-16alpha-carb omethoxy-4-pregnene-3,20-dione."
- JOURNAL OF MEDICINAL CHEMISTRY, Bd. 16, Nr. 3, März 1973, WASHINGTON US, Seiten 270-273, XP000056778 A. J. SOLO & J. N. KAPOOR: "Ring D bridged steroid analogs. 11. The high clauberg activity of 19-nor-14alpha,17alpha-ethano-4-pregnene-3 ,20-dione."
- SOUTH AFRICAN JOURNAL OF CHEMISTRY, Bd. 44, Nr. 3, September 1991, Seiten 87-94, XP000569150 J. R. BULL & R. I. THOMSON: "Influence of bridgehead functionality on reactions of 14alpha,17alpha-etheno-19-norsteroids."
- JOURNAL OF PHARMACEUTICAL SCIENCES, Bd. 62, Nr. 9, September 1973, WASHINGTON US, Seiten 1471-1475, XP000568770 A. J. SOLO & B. SINGH: "Bridged D-ring steroid analogs XII: effect of D-ring substituents on chemical shift of angular methyl protons."

## Beschreibung

Die vorliegende Erfindung betrifft 14,17-C₂-überbrückte Steroide der allgemeinen Formel (**I**), 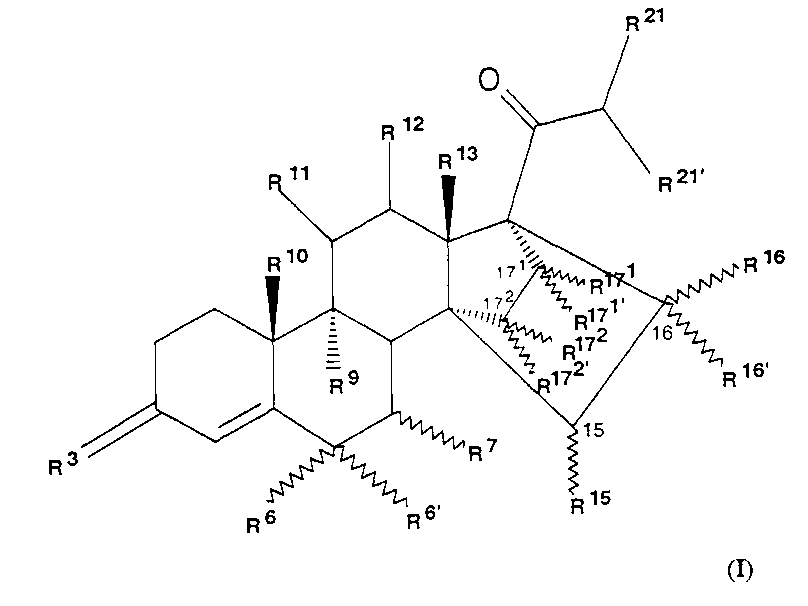 worin
- R³: für ein Sauerstoffatom, die Hydroxyiminogruppe oder zwei Wasserstoffatome,
- R⁶: für ein Wasserstoff-, Fluor-, Chlor- oder Bromatom oder für einen α- oder β -ständigen C₁-C₄-Alkylrest, wobei dann R^{6'} und R⁷ Wasserstoffatome darstellen, oder aber
- R⁶: für ein Wasserstoff-, Fluor-, Chlor- oder Bromatom oder für einen C₁-C₄-Alkylrest, wobei dann R^{6'} und R⁷ eine gemeinsame zusätzliche Bindung darstellen,
- R⁷: für einen α- oder β-ständigen C₁-C₄-Alkylrest, wobei dann R⁶ und R^{6'} Wasserstoffatome darstellen, oder aber
- R⁶ und R⁷: gemeinsam für eine α- oder β-ständige Methylengruppe und R^{6'} für ein Wasserstoffatom oder
- R⁶ und R^{6'}: gemeinsam für eine Ethylen- oder Methylengruppe und R⁷ für ein Wasserstoffatom,
- R⁹ und R¹⁰: jeweils für ein Wasserstoffatom oder eine gemeinsame Bindung,
- R¹¹ und R¹²: jeweils für ein Wasserstoffatom oder eine gemeinsame Bindung,
- R¹³: für eine Methyl- oder Ethylgruppe,
- R¹⁵: für ein Wasserstoffatom oder einen C₁-C₃-Alkylrest,
- R¹⁶ und R^{16'}: unabhängig voneinander für ein Wasserstoffatom, einen C₁-C₃-Alkylrest
oder einen C₂-C₄-Alkenylrest oder gemeinsam für eine C₁-C₃-Alkylidengruppe
- R¹⁵ und R¹⁶: für eine gemeinsame Bindung sowie R¹⁶ für ein Wasserstoffatom oder
einen C₁-C₃-Alkylrest oder
- R¹⁵ und R¹⁶: gemeinsam für einen Ring der Teilformel 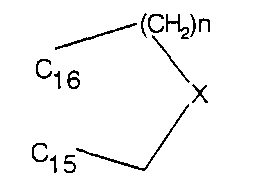 worin n = 1 und 2 und X eine Methylengruppe oder ein Sauerstoffatom bedeuten sowie
R^{16'} für ein Wasserstoffatom,
- R^{17¹}: für ein Wasserstoffatom oder einen C₁-C₃-Alkylrest,
- R^{17²}: für ein Wasserstoffatom, einen C₁-C₃-Alkylrest'oder einen C₂-C₄-Alkenylrest
- R^{17^{1'}} und R^{17^{2'}}: jeweils für ein Wasserstoffatom oder für eine gemeinsame Bindung,
- R²¹: für ein Wasserstoffatom oder einen C₁-C₃-Alkylrest,
- R^{21'}: für ein Wasserstoffatom, einen C₁-C₃-Alkylrest oder eine Hydroxygruppe stehen,
ausgenommen der Verbindung 14,17-Ethano-19-norpregn-4-en-3,20-dion.

Die Schlangenlinien 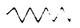 in den allgemeinen Formeln der vorliegenden Erfindung bedeuten, daß der betreffende Suhstituent sich in der α- oder β-Position an dem entsprechenden Kohlenstoffatom befinden kann.

Bei den vorstehend als mögliche Substituenten genannten C₁-C₃-Alkylgruppen kann es sich um eine Methyl-, Ethyl-, n-Propyl- oder i-Propyl- und bei den C₁-C₄-Alkylgruppen zusätzlich um eine n-Butyl-, i-Butyl- oder tert.-Butylgruppe handeln. Eine Methyl- oder Ethylgruppe ist in allen Fällen bevorzugt.

Im Falle des C₂-C₄-Alkenylrestes für R¹⁶, R^{16'} und/oder R^{17²} handelt es sich um einen Vinyl-, Allyl- oder But-3-enylrest; der Vinylrest ist bevorzugt.

Bevorzugt gemäß vorliegender Erfindung sind solche Verbindungen der allgemeinen Formel (**I**), in denen
- R³: für ein Sauerstoffatom oder zwei Wasserstoffatome, und/oder
- R⁶: für ein Wasserstoffatom oder für einen α- oder β-ständigen C₁-C₄-Alkylrest, wenn R^{6'} und R⁷ Wasserstoffatome darstellen, oder aber
- R^{6'}: für ein Wasserstoff-, Chlor- oder Bromatom oder für einen C₁-C₄-Alkylrest, wenn R^{6'} und R⁷ eine gemeinsame zusätzliche Bindung darstellen und/oder
- R¹⁶ und R^{16'}: jeweils für ein Wasserstoffatom, jeweils für eine Methylgruppe oder der
eine dieser beiden Substituenten für eine C₁-C₄-Alkyl- oder eine Vinylgruppe und der andere dieser beiden Substituenten für ein Wasserstoffatom stehen,
oder
beide gemeinsam eine C1-C3-Alkylidengruppe bilden und/oder
- R^{17¹} und R^{17²}: unabhängig voneinander für ein Wasserstoffatom oder eine Methylgruppe und/oder
- R^{17^{1'}} und R^{17^{2'}}: jeweils für ein Wasserstoffatom oder eine gemeinsame Bindung und/oder
- R²¹: für ein Wasserstoffatom oder einen C₁-C₃-Alkylrest sowie R^{21'} für ein Wasserstoffatom oder eine Hydroxygruppe
stehen,
sowie die anderen Substituenten alle die in Formel (**I**) angegebenen Bedeutungen haben können.

Die nachstehend genannten Verbindungen sind erfindungsgemäß insbesondere bevorzugt:
14,17-Ethano-19-norpregna-4,9-dien-3,20-dion;
14,17-Ethano-19-norpregna-4,6-dien-3,20-dion;
14,17-Ethano-19-norpregna-4,15-dien-3,20-dion
14,17-Ethano-19-norpregna-4,6,15-trien-3,20-dion
14,17-Ethano-19-norpregna-4,9,15-trien-3,20-dion
21-Methyl-14,17-ethano-19-norpregn-4-en-3,20-dion;
21-Methyl-14,17-ethano-19-norpregna-4,9-dien-3,20-dion;
21-Methyl-14,17-ethano-19-norpregna-4,6-dien-3,20-dion;
21-Methyl-14,17-ethano-19-norpregna-4,15-dien-3,20-dion
21-Methyl-14,17-ethano-19-norpregna-4,9,15-trien-3,20-dion
14,17-Etheno-19-norpregn-4-en-3,20-dion;
14,17-Etheno-19-norpregna-4,6-dien-3,20-dion;
14,17-Etheno-19-norpregna-4,9-dien-3,20-dion;
21-Methyl-14,17-etheno-19-norpregn-4-en-3,20-dion
21-Methyl-14,17-etheno-19-norpregna-4,6-dien-3,20-dion
21-Methyl-14,17-etheno-19-norpregna-4,9-dien-3,20-dion;
21-Methyl-14,17-etheno-19-norpregna-4,9,11-trien-3,20-dion
21-Hydroxy-14,17-etheno-19-norpregn-4-en-3,20-dion
21-Hydroxy-14,17-etheno-19-norpregna-4,9-dien-3,20-dion
17¹-Methyl-14,17-etheno-19-norpregn-4-en-3,20-dion
17¹-Methyl-14,17-etheno-19-norpregna-4,6-dien-3,20-dion
17²-Methyl-14,17-etheno-19-norpregn-4-en-3,20-dion
17²-Methyl-14,17-etheno-19-norpregna-4,9-dien-3,20-dion
15β,16α-Dimethyl-14,17-etheno-19-norpregn-4-en-3,20-dion
6-Methyl-14,17-ethano-19-norpregna-4,6-dien-3,20-dion;
6-Chlor -14,17-ethano-19-norpregna-4,6-dien-3,20-dion;
6α-Methyl-14,17-ethano-19-norpregn-4-en-3,20-dion;
6,21-Dimethyl-14,17-ethano-19-norpregna-4,6-dien-3,20-dion;
15β,16α-Dimethyl-14,17-ethano-19-norpregn-4-en-3,20-dion
6-Chlor-21-methyl-14,17-ethano-19-norpregna-4,6-dien-3,20-dion;
16α-Methyl-14,17-ethano-19-norpregn-4-en-3,20-dion;
16α-Methyl-14,17-ethano-19-norpregna-4,6-dien-3,20-dion;
16α-Methyl-14,17-ethano-19-norpregna-4,9-dien-3,20-dion;
16α,21-Dimethyl-14,17-ethano-19-norpregna-4,9-dien-3,20-dion
21-Hydroxy-16α-methyl-14,17ethano-19-norpregn-4-en-3,20-dion
16α-Ethyl-14,17-ethano-19-norpregn-4-en-3,20-dion;
16α-Ethenyl-14,17-ethano-19-norpregn-4-en-3,20-dion;
16-Methyl-14,17-ethano-19-norpregna-4,15-dien-3,20-dion
(17¹*R*)-17¹-Methyl-14,17-ethano-19-norpregn-4-en-3,20-dion
(17¹*S*)-17¹-Methyl-14,17-ethano-19-norpregn-4-en-3,20-dion
(17¹*R*)-17¹-Methyl-14,17-ethano-19-norpregna-4,9-dien-3,20-dion
(17¹*S*)-17¹-Methyl-14,17-ethano-19-norpregna-4,9-dien-3,20-dion
(17²*R*)-17²-Methyl-14,17-ethano-19-norpregn-4-en-3,20-dion
(17²*R*)-172-Methyl-14,17-ethano-19-norpregna-4,6-dien-3,20-dion
(17²*R*)-17²-Methyl-14,17-ethano-19-norpregna-4,9-dien-3,20-dion
(17²R)-17²,21-Dimethyl-14,17-ethano-19-norpregna-4,6-dien-3,20-dion
(17²R)-17²,21-Dimethyl-14,17-ethano-19-norpregna-4,9-dien-3,20-dion
(17²*R*)-172,21-Dimethyl-14,17-ethano-19-norpregna-4,9,11-trien-3,20-dion
16-Methylen-14,17-ethano-19-norpregn-4-en-3,20-dion
16-Methylen-14,17-ethano-19-norpregna-4,6-dien-3,20-dion
16-Methylen-14,17-ethano-19-norpregna-4,9-dien-3,20-dion
21-Hydroxy-14,17-ethano-19-norpregn-4-en-3,20-dion;
21-Hydroxy-14,17-ethano-19-norpregna-4,6-dien-3,20-dion;
21-Hydroxy-14,17-ethano-19-norpregna-4,9-dien-3,20-dion;
21-Hydroxy-14,17-ethano-19-norpregna-4,9,15-trien-3,20-dion
(21*R*)-21-Hydroxy-21-methyl-14,17-ethano-19-norpregn-4-en-3,20-dion;
(21*S*)-21-Hydroxy-21-methyl-14,17-ethano-19-norpregn-4-en-3,20-dion;
(21*R*)-21-Hydroxy-21-methyl-14,17-ethano-19-norpregna-4,9-dien-3,20-dion;
(21*S*)-21-Hydroxy-21-methyl-14,17-ethano-19-norpregna-4,9-dien-3 ,20-dion;
(21*R*)-21-Hydroxy-21-methyl-14,17-ethano-19-norpregna-4,6-dien- 3,20-dion;
(21*S*)-21-Hydroxy-21-methyl-14,17-ethano-19-norpregna-4,6-dien- 3,20-dion;
(21*R*)-21-Hydroxy-21-methyl-14,17-ethano-19-norpregna-4,9,15-trien-3,20-dion
(21*S*)-21-Hydroxy-21-methyl-14,17-ethano-19-norpregna-4,9,15-trien-3,20-dion
14,17-Ethano-18a-homo-19-norpregn-4-en-3,20-dion
14,17-Ethano-18a-homo-19-norpregna-4,6-dien-3,20-dion
14,17-Ethano-18a-homo-19-norpregna-4,9-dien-3,20-dion
14,17-Ethano-18a-homo-19-norpregna-4,15-dien-3,20-dion
21-Methyl-14,17-ethano-18a-homo-19-norpregn-4-en-3,20-dion
21-Methyl-14,17-ethano-18a-homo-19-norpregna-4,6-dien-3,20-dion
21-Methyl-14,17-ethano-18a-homo-19-norpregna-4,9-dien-3,20-dion
(21*R*)-21-Hydroxy-21-methyl-14,17-ethano-18a-homo-19-norpregn-4-en-3,20-dion
(21*S*)-21-Hydroxy-21-methyl-14,17-ethano-18a-homo-19-norpregn-4-en-3,20-dion
(21*R*)-21-Hydroxy-21-methyl-14,17-ethano-18a-homo-19-norpregna-4,9-en-3,20-dion
(21*S*)-21-Hydroxy-21-methyl-14,17-ethano-18a-homo-19-norpregna-4,9-en-3,20-dion
(21*R*)-21-Hydroxy-21-methyl-14,17-ethano-18a-homo-19-norpregna-4,6-en-3,20-dion
(21*S*)-21-Hydroxy-21-methyl-14,17-ethano-18a-homo-19-norpregna-4,6-en-3,20-dion

Im Gestagenrezeptor-Bindungstest auf gestagene Wirkung unter Verwendung von Cytosol aus Kaninchenuterushomogenat und von ³H-Progesteron als Bezugssubstanz zeigen die neuen Verbindungen eine sehr starke Affinität zum Gestagenrezeptor. Im Schwangerschaftserhaltungstest an der Ratte zeigen die erfindungsgemäßen Verbindungen der allgemeinen Formel (I) eine sehr hohe gestagene Wirksamkeit.
Die Verbindungen der allgemeinen Formel (I) zeigen auch Wirkungen an anderen Steroidrezeptoren.

14,17-Ethano-19-norpregn-4-en-3,20-dion, die aus dem Umfang der allgemeinen Formel I disclaimte Verbindung, wurde bereits von A. J. Solo und J. N. Kapoor in J. Med. Chem. 16, 270 (1973) beschrieben. Diese Verbindung besitzt im Endometriumstransformationstest (Clauberg-Test) auf gestagene Wirkung nach subcutaner Applikation gute, nach oraler Gabe aber nur noch eine geringe Wirkung. Der Faktor zwischen subcutaner und peroraler Wirkung liegt laut der genannten Literaturstelle über 20.

Zusätzlich zur sehr hohen gestagene Wirksamkeit im Schwangerschaftserhaltungstest, die größtenteils sogar die der disclaimtem Verbindung übertrifft, zeigen die erfindungsgemäßen Verbindungen der allgemeinen Formel I im Gegensatz zur bereits bekannten Verbindung 14,17-Ethano-19-norpregn-4-en-3,20-dion aber größtenteils auch nach oraler Gabe eine gute gestagene Wirkung. Der Faktor zwischen subcutaner und peroraler Wirkung liegt für die erfindungsgemäßen Verbindungen ungefähr zwischen 3 und 5. Die erfindungsgemäßen Verbindungen unterscheiden sich also von der disclaimten Verbindung durch ein deutlich verbessertes Wirkungsspektrum.

Aufgrund ihrer hohen gestagenen Wirksamkeit können die neuen Verbindungen der allgemeinen Formel (I) beispielsweise allein oder in Kombination mit Estrogenen in Präparaten zur Kontrazeption verwendet werden. Aber auch alle anderen heutzutage für Gestagene bekannten Verwendungsmöglichkeiten stehen den neuen Verbindungen offen.

Geeignete Dosierungen können routinemäßig bestimmt werden, z. B. durch Bestimmung der Bioequivalenz gegenüber einem bekannten Gestagen für eine bestimmte Verwendung, beispielsweise eine Menge, die bioequivalent zu 30 bis 150 µg Levonorgestrel für die Kontrazeption ist.

Die Dosierung der erfindungsgemäßen Verbindungen in Kontrazeptionspräparaten soll vorzugsweise 0,01 bis 2 mg pro Tag betragen.

Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen vorzugsweise synthetische Estrogene wie Ethinylestradiol, 14α,17α-Ethano-1,3,5(10)-estratrien-3,17β-diol (WO 88/01275) oder 14α,17α-Ethano-1,3,5(10)-estratrien-3,16α,17β-triol (WO 91/08219) in Betracht.
Das Estrogen wird in einer Menge verabfolgt, die der von 0,01 bis 0,05 mg Ethinylestradiol entspricht.

Die neuen Verbindungen der allgemeinen Formel (**I**) können auch in Präparaten zur Behandlung gynäkologischer Störungen und zur Substitutionstherapie eingesetzt werden. Wegen ihres günstigen Wirkungsprofils sind die erfindungsgemäßen Verbindungen besonders gut geeignet zur Behandlung prämenstrueller Beschwerden, wie Kopfschmerzen, depressive Verstimmung, Wasserretention und Mastodynie. Die Tagesdosis bei der Behandlung prämenstrueller Beschwerden liegt bei etwa 1 bis 20 mg.

Schließlich können die neuen Verbindungen auch als gestagene Komponente in den neuerdings bekannt gewordenen Zusammensetzungen für die weibliche Fertilitätskontrolle, die sich durch die zusätzliche Verwendung eines kompetitiven Progesteronantagonisten auszeichnen, zum Einsatz kommen (H.B. Croxatto und A.M. Salvatierra in Female Contraception and Male Fertility Regulation, ed. by Runnebaum, Rabe & Kiesel - Vol. 2, Advances in Gynecological and Obstetric Research Series, Parthenon Publishing Group - 1991, Seite 245).
Die Dosierung liegt im bereits angegebenen Bereich, die Formulierung kann wie bei konventionellen OC-Präparaten erfolgen. Die Applikation des zusätzlichen, kompetitiven Progesteronantagonisten kann dabei auch sequentiell vorgenommen werden.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.
Für die bevorzugte orale Applikation kommen insbesondere Tabletten, Dragees, Kapseln, Pillen, Suspensionen oder Lösungen in Frage.
Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.
Die Verbindungen der allgemeinen Formel (**I**) können auch kontinuierlich durch ein intrauterines Freisetzungssystem (IUD) appliziert werden; die Freisetzungsrate der aktiven Verbindung(en) wird dabei so gewählt, daß die täglich freigesetzte Dosis innerhalb der bereits angegebenen Dosierungsbereiche liegt.
Es ist auch möglich, die erfindungsgemäßen Substanzen in ein transdermales System einzuarbeiten und sie damit transdermal zu applizieren.
Die zur Herstellung der Verbindungen der allgemeinen Formel (**I**) zunächst benötigten Ausgangsverbindungen sind gemäß nachstehender Syntheseroute zugänglich: 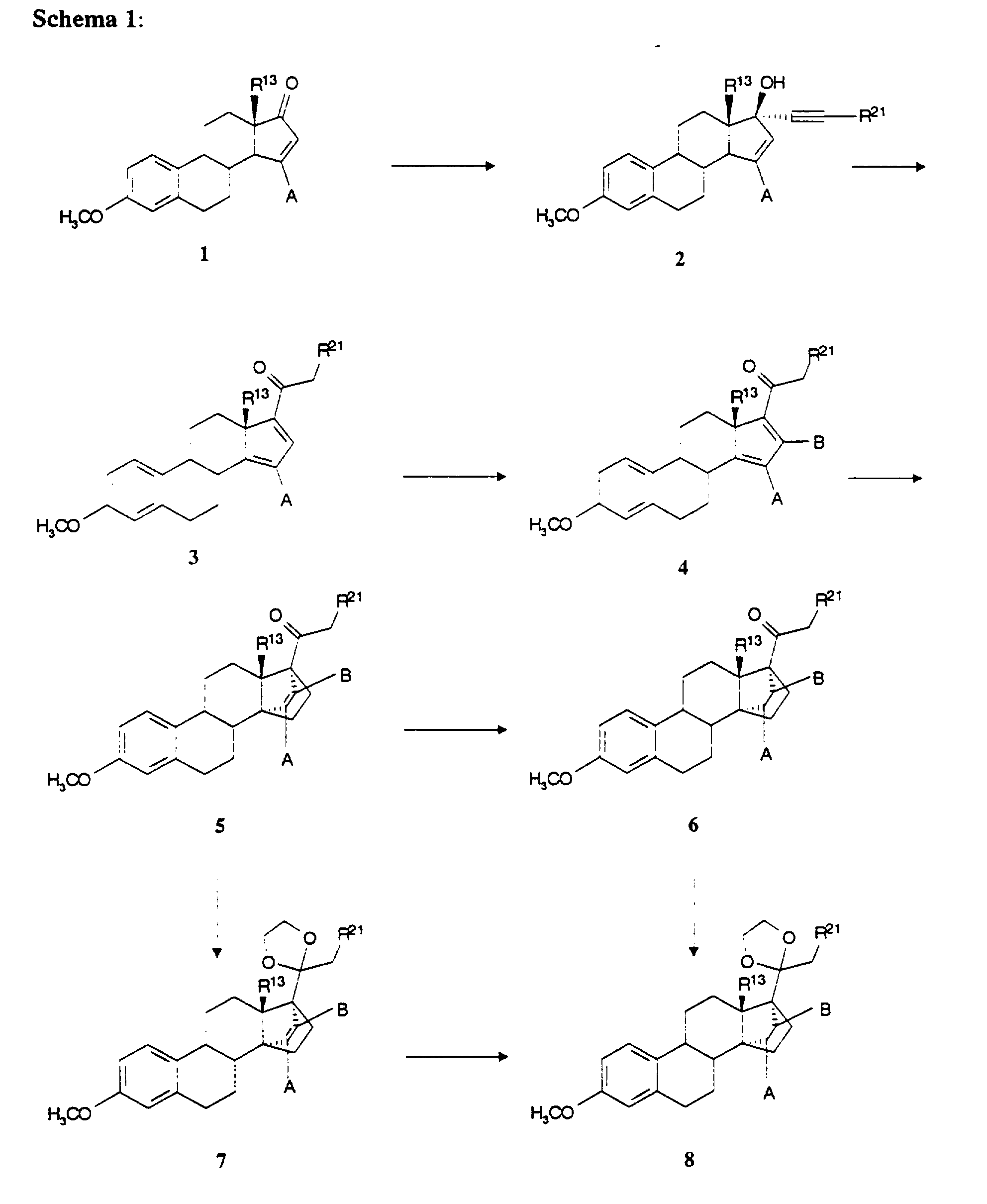 R¹³ = -CH₃, -C₂H₅; R²¹ = Wasserstoff, C₁-C₃-Alkyl; A und B = unabhängig voneinander Wasserstoff oder C₁-C₃-Alkyl
Gemäß Schema 1 kann beispielsweise eine an sich bekannte Verbindung der allgemeinen Formel **1** (siehe beispielsweise DE 43 26 240 A1) durch Addition des Anions eines terminalen Alkins in eine an sich bekannte Verbindung der allgemeinen Formel **2** überführt werden. Diese wird durch Reaktion mit einer Säure wie beispielsweise Schwefelsäure, Salzsäure, p-Toluolsulfonsäure, Ameisensäure oder Essigsäure in Gegenwart oder Abwesenheit von inerten Lösungsmitteln wie zum Beispiel Toluol, Tetrahydrofuran oder Dichlormethan in eine Verbindung der allgemeinen Formel **3** überführt (siehe beispielsweise D.K. Phillips, P.P. Wickham, G.O. Potts und A. Arnold, *J. Med. Chem.,* **11**, 924 (1968)). Falls gewünscht, kann eine Verbindung der allgemeinen Formel **3** mit geeigneten Nucleophilen, beispielsweise Dialkylkupferverbindungen, gefolgt von einer Oxidation, beispielsweise einer modifizierten Saegusa-Oxidation (vergleiche I. Minami et al., *Tetrahedron* **42**, 2971 (1986) oder EP-A 0299913) in eine Verbindung der allgemeinen Formel **4** überführt werden, wobei B dann für einen Alkylrest steht. Ansonsten steht B für Wasserstoff.
Die Verbindung der allgemeinen Formel **4** kann dann mit Ethen unter Druck und bei erhöhter Temperatur nach an sich bekannten Verfahren in einer Cycloaddition in eine Verbindung der allgemeinen Formel **5** überführt werden. Diese läßt sich dann nach Standardverfahren durch eine Hydrierung der 17¹,17²-Doppelbindung (Kohlenstoffatom 17¹ bzw. 17² bezeichnet das Kohlenstoffatom, an dem sich der Substituent R^{17¹} bzw. R^{17²} befindet) mit Edelmetallkatalysatoren wie zum Beispiel Platin oder Palladium in eine Verbindung der allgemeinen Formel **6** überführen. Die Verbindungen der allgemeinen Formel **5** und **6**, in denen R²¹ für ein Wasserstoffatom steht, können auch nach Standardverfahren alkyliert werden und so in die entsprechenden Verbindungen der allgemeinen Formel **5** und **6**, in denen R²¹ für eine C₁-C₃-Alkylgruppe steht, überführt werden (siehe beispielsweise R. Bloch *Tetrahedron 39,* 639 (1983)). Die Verbindungen der allgemeinen Formel **5** lassen sich nach Standardmethoden zu Verbindungen der allgemeinen Formel **7** ketalisieren, die durch Hydrierung in die Verbindungen der allgemeinen Formel **8** überführt werden können. Diese Verbindungen lassen sich auch durch die Ketalisierung der Verbindung der allgemeinen Formel **6** erhalten. Dabei sind anstelle der 1,2-Ethandiylbis(oxy)-Schutzgruppe am Kohlenstoffatom 20 generell auch andere bekannte Ketoschutzgruppen wie beispielsweise die 2,2-Dimethyl-1,3-propandiylbis(oxy)-Gruppe erfindungsgemäß geeignet. Weitere Schutzgruppen, die im Rahmen vorliegender Erfindung verwendet werden können, lassen sich bei "Protective Groups in Organic Synthesis", Theodora W. Greene, Peter G. N. Wuts, John Wiley and Sons, Inc., New York, 1991, S. 178 - 210, entnehmen.

Diejenigen Verbindungen der allgemeinen Formeln **5** und **6**, in denen R¹³ eine Ethylgruppe und R²¹ ein Wasserstoffatom oder eine C₁-C₃-Alkylgruppe oder R¹³ eine Methylgruppe und R²¹ eine C₁-C₃-Alkylgruppe bedeuten, gehören zusammengenommen als Zwischenverbindungen der allgemeinen Formel II zum Gegenstand der vorliegenden Erfindung: 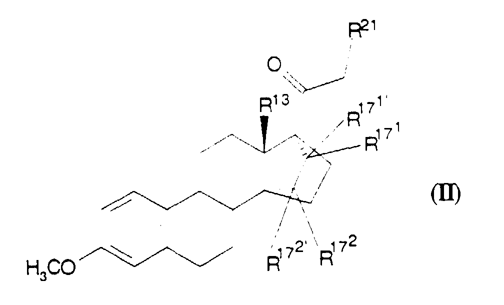 worin
R¹³ = -C₂H₅; R²¹ = Wasserstoff, C₁-C₃-Alkyl oder
R¹³ = -CH₃; R²¹ = C₁-C₃-Alkyl und
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung bedeuten.

Die durch Ketalisierung der Verbindungen der allgemeinen Formeln **5** bzw. **6** erhaltenen Verbindungen der allgemeinen Formeln **7** und **8** sind alle neu und gehören zusammengenommen als Zwischenverbindungen der allgemeinen Formel III ebenfalls zum Gegenstand der vorliegenden Erfindung: 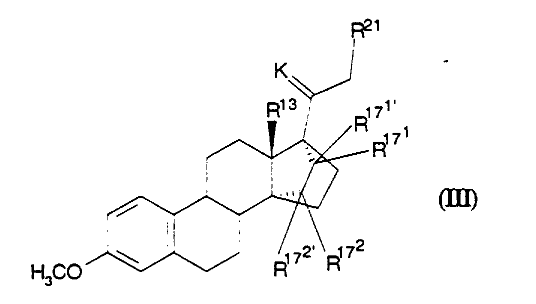 worin
R¹³ = -CH₃, -C₂H₅,
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = eine Ketalschutzgruppe,
R²¹ = Wasserstoff, C₁-C₃-Alkyl
bedeuten. 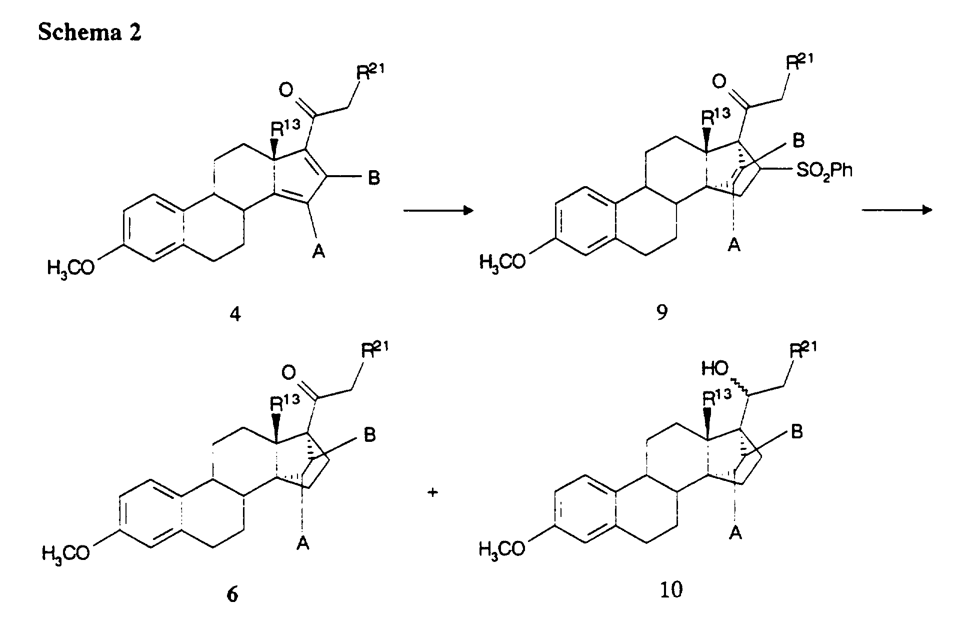 R¹³ = -CH₃, -C₂H₅; R²¹ = Wasserstoff, C₁-C₃-Alkyl; A und B = unabhängig voneinander Wasserstoff oder C₁-C₃-Alkyl

Gemäß Schema 2 gelingt die Umsetzung einer Verbindung der allgemeinen Formel **4** auch nach an sich bekannten Verfahren mit Phenylvinylsulfon in inerten Lösungsmitteln zu einer Verbindung der allgemeinen Formel **9** (J.R. Bull und R.I. Thomson *S.Afr.J.Chem.* **44**, 87 (1991)). Die Reduktion dieser Verbindung durch Metalle wie Raney-Nickel oder Magnesium in niederen Alkoholen wie Methanol oder Ethanol führt zu Verbindungen der allgemeinen Formeln **6** und **10**, die durch Oxidations- beziehungsweise Reduktionsverfahren, beispielsweise mit Pyridiniumdichromat oder unter den Bedingungen einer Oppenauer-Oxidation bzw. mit Natriumborhydrid oder Lithiumaluminiumhydrid, ineinander überführt werden können.

Die Herstellung der erfindungsgemäßen Verbindungen, die in den Positionen 15 und/oder 16 substituiert sind, erfolgt durch die Umsetzung einer Verbindung der allgemeinen Formel **4** mit geeigneten Olefinen wie zum Beispiel Propen, 2-Methylpropen, 2-Buten, Cyclopenten, Cyclohexen oder 2,5-Dihydrofuran und gegebenenfalls der Hydrierung der entstandenen 17¹,17²-Doppelbindung. Die weiteren Umsetzungen der so erhaltenen Verbindungen erfolgen analog zu den weiteren Reaktionen der Verbindungen der allgemeinen Formel **6**.

Zur Herstellung der erfindungsgemäßen Verbindungen, die in Position 16 einen Alkyl- oder Alkenylrest tragen, kann eine Verbindung der allgemeinen Formel **4** auch mit einem Acrylsäureester der Formel H₂C=CH-COOAlkyl (Alkyl = C₁-C₄-Alkyl) gemäß Schema 3 umgesetzt werden. 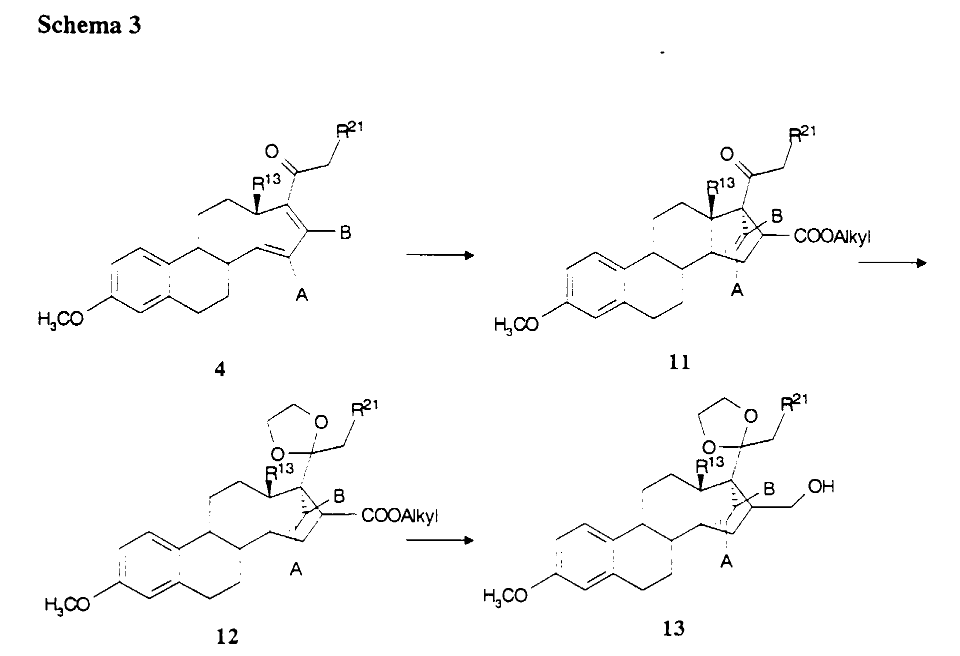 R¹³ = -CH₃, -C₂H₅; R²¹ = Wasserstoff, C₁-C₃-Alkyl; A und B = unabhängig voneinander Wasserstoff oder C₁-C₃-Alkyl

Die so erhaltenen Verbindungen der allgemeinen Formel **11** werden nach Ketalisierung der 20-Ketogruppe und Hydrierung der entstandenen 17¹,17²-Doppelbindung zu den Verbindungen der allgemeinen Formel **12** umgesetzt, welche mit Lithiumaluminiumhydrid zu den 16-Hydroxymethylverbindungen der allgemeinen Formel 13 umgesetzt werden können.

Die Verbindungen der allgemeinen Formel **13** lassen sich nach Standardverfahren (siehe beispielsweise J. Hooz und S. S. Gilani, *Can. J. Chem.* **46**, 86 (1968)) in die entsprechenden 16-Brommethylverbindungen überführen, welche unter den Bedingungen einer Birch-Reduktion zu den 16-Methylverbindungen reduziert werden. Dabei wird auch der aromatische A-Ring unter Ausbildung der 2,5(10)-Dienstruktur reduziert.

Die Verbindungen der allgemeinen Formel **13** lassen sich durch Oxidation nach an sich bekannten Verfahren, beispielsweise mit Pyridiniümdichromat, in die entsprechenden 16-Aldehyde überführen, welche nach Umsetzung mit entsprechenden Phosphoryliden zu den erfindungsgemäßen 16-Alkenylverbindungen führen, die sich durch eine Hydrierung in die 16-Alkylverbindungen überführen lassen.

Die 16-Aldehyde können durch Erwärmen mit Arylhydrazinen nach an sich bekannten Verfahren (vergleiche beispielsweise M. Pieper et al., *Liebigs Ann. Chem.,* 1334 (1986)) in die Arylhydrazone überführt werden, welche bei Basenbehandlung im Sinne einer Shapiro- bzw. Bamford-Stevens Reaktion zu den 16-Exomethylenverbindungen fragmentieren. Alternativ lassen sich die 16-Aldehyde durch Umsetzung mit Sulfonsäure-Derivaten wie etwa Sulfonsäurehalogeniden oder Sulfonsäureanhydriden in Gegenwart von Basen wie etwa Lithiumdiisopropylamid oder auch Kaliumhexamethyldisilazid in inerten Lösungsmitteln wie z.B. Tetrahydrofuran in die Enolsulfonsäureester überführen, die durch reduktive Spaltung, beispielsweise durch Behandlung mit Ammoniumformiat in Gegenwart katalytischer Mengen eines Palladium(II)-Katalysators wie etwa Palladium(II)-acetat in geeigneten Lösungsmitteln, beispielsweise Acetonitril, in die 16-Exomethylenverbindung übergehen.

Die Verbindungen der allgemeinen Formeln **11**, **12** und **13** zusammen mit den im Text beschriebenen Derivaten sind alle neu und gehören als Zwischenverbindungen der allgemeinen Formel IV zum Gegenstand vorliegender Erfindung: 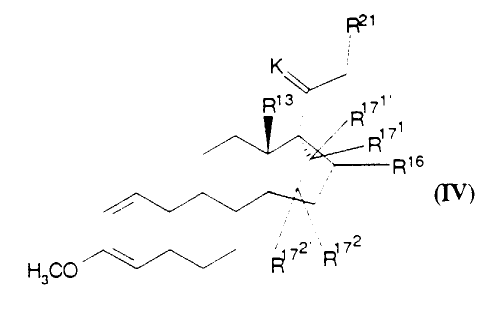 worin
R¹³ = CH₃, -C₂H₅,
R¹⁶ = -COOAlkyl wobei Alkyl ein C₁-C₄-Alkylresi ist, oder -CH₂OH, oder CHO, oder Methylen,
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff, C₁-C₃-Alkyl
bedeuten.

Die Verbindungen der allgemeinen Formel **12** lassen sich durch alkalische Hydrolyse in die entsprechenden Carbonsäuren überführen, welche durch Decarboxylierung und Oxidation, beispielsweise durch Erhitzen mit Bleitetraacetat und Kupfer(II)-acetat in Toluol (siehe beispielsweise J. D. Bacha und J. K. Kochi, *Tetrahedron* **24**, 2215 (1968)) zu Derivaten mit einer 15,16-Doppelbindung führen. 14,17-C₂-überbrückte Derivate mit einer 15,16-Doppelbindung sind auch auf anderen Wegen zugänglich:
1. Die Umsetzung einer Verbindung der allgemeinen Formel **4** mit Maleinsäureanhydrid zum Diels-Alder Produkt, gefolgt von der katalytischen Hydrierung der 17¹,17²-Doppelbindung ergibt nach Erhitzen mit Bistriphenylphosphinnickeldicarbonyl in geeigneten Lösungsmitteln wie Diglyme das entsprechende 15,16-Doppelbindungsderivat (siehe beispielsweise K. Wiesner et al., *Can. J. Chem.* **52**, 640 (1974)). Alternativ kann ausgehend vom 17¹,17²-gesättigten Anhydrid mit Basen, wie etwa wässriger Natronlauge, zur 15,16-Dicarbonsäure umgesetzt werden, welche via doppelter Decarboxilierung in das entsprechende 15,16-Doppelbindungsderivat überführt wird (siehe beispielsweise C. M. Cimarusti und J. Wolinsky, *J. Am. Chem. Soc.* **90**, 113 (1968)). Beispielsweise wird die Dicarbonsäure mit Bleitetraacetat in geeigneten Lösungsmitteln, beispielsweise Pyridin, auf Temperaturen von 30-100°C erwärmt.
   Das Diels-Alder Addukt läßt sich auch für die Synthese anderer Derivate nutzen: Reduktion des Diels-Alder Produktes zum Lacton mit geeigneten Reduktionsmitteln, wie etwa Natriumborhydrid (siehe beispielsweise D. M. Bailey und R. F. Johnson, *J. Org. Chem.* **35**, 3574 (1970)), Oxidation des entstandenen 20-Alkoholes, beispielsweise mit Pyridiniumchlorochromat und Schutz des Ketons als Ketal führt nach Reduktion des Lactons mit geeigneten Reduktionsmitteln, wie etwa Lithiumaluminiumhydrid, zur 15,16-Bishydroxymethylverbindung. Die Hydroxyfunktionen lassen sich zum Beispiel unter geeigneten Bedingungen zu einem cyclischen Ether kondensieren. Dies wird bevorzugt unter basischen Bedingungen, wie etwa durch Behandlung mit Sulfonsäurederivaten wie Sulfonsäurehalogeniden oder Sulfonsäureanhydriden in Gegenwart von Basen wie beispielsweise Pyridin, durchgeführt.
2. Die Umsetzung einer Verbindung der allgemeinen Formel **4** mit Vinylencarbonat (zu Diels-Alder Reaktionen mit Vinylencarbonat siehe beispielsweise Y. Shizuri et al., J. *Chem. Soc., Chem. Commun.* 292 (1985) oder G. H. Posner et al., *Tetrahedron Lett.* **32,** 5295 (1991)) im Sinne einer Diels-Alder Reaktion gemäß Schema 4 führt zu einem Cycloadditionsprodukt der Formel **14**. Nach Hydrierung der 17¹,17²-Doppelbindung und Spaltung des cyclischen Carbonates nach Standardverfahen, wie etwa der Umsetzung des Carbonates in einem geeigneten Lösungsmittel wie z.B. Methanol mit einer Base wie z.B. Kaliumcarbonat, wird ein Diol der Formel **17** erhalten. Die Reihenfolge von Hydrierung und Carbonatspaltung ist beliebig. 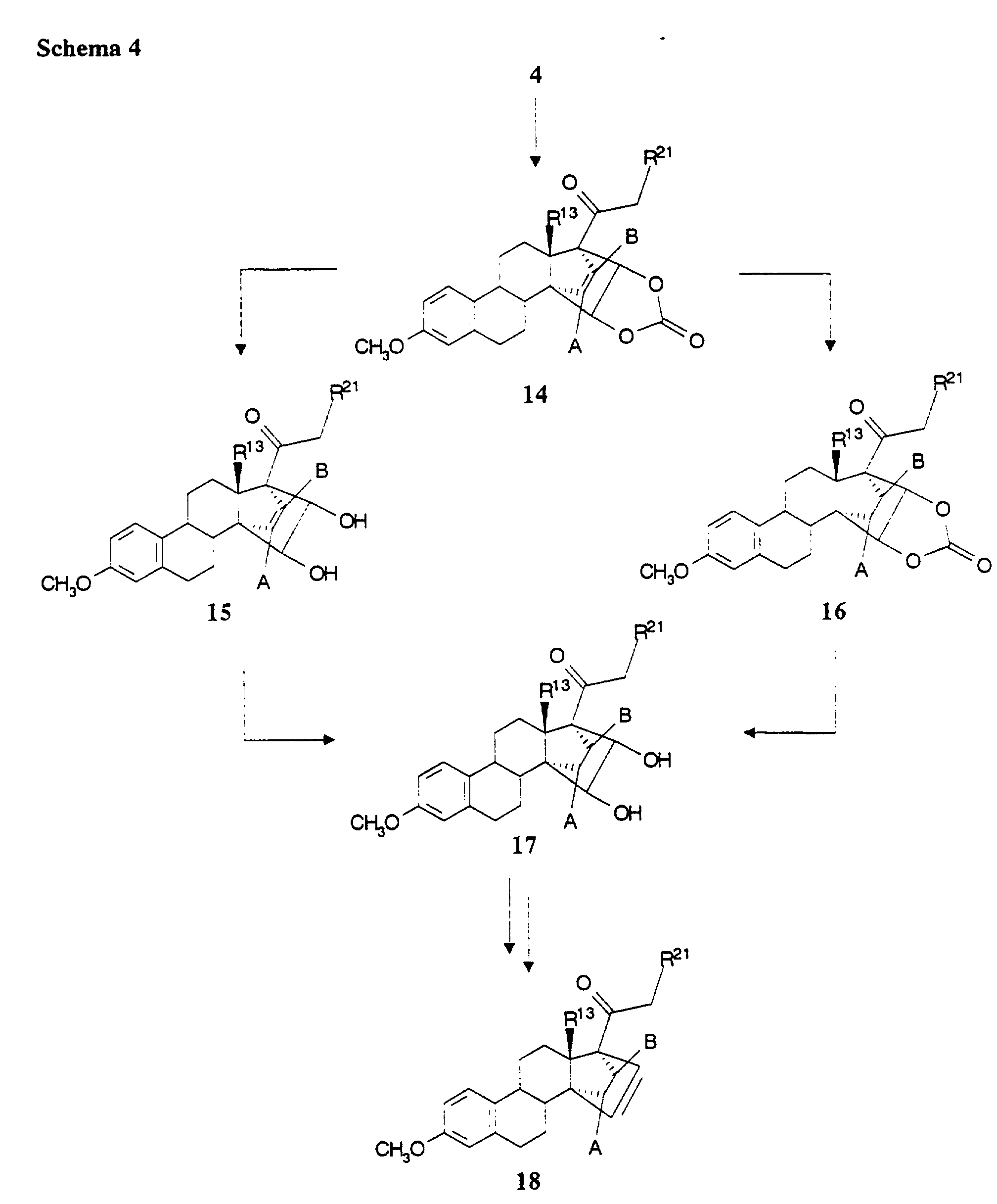
R¹³ = -CH₃, -C₂H₅; R²¹ = Wasserstoff, C₁-C₃-Alkyl; A und B = unabhängig voneinander Wasserstoff oder C₁-C₃-Alkyl

Zur Umwandlung von vicinalen Diolen in Olefine stehen eine ganze Reihe dem Fachmann geläufiger Methoden zur Auswahl (vergleiche beispielsweise M. Ando et al., *Chemistry Leiters* 879 (1986)). Beispielsweise läßt sich ein Diol der allgemeinen Formel **17** mit einem Orthoester, wie beispielsweise Trimethylorthoformiat mit Säurekatalyse, beispielsweise mit Pyridiniumparatoluolsulfonat, in einem geeigneten Lösungsmittel, hier sei als Beispiel Dichlormethan genannt, oder ohne Lösungsmittel zu dem entsprechenden Orthoester umsetzen, der bei Erwärmung in geeigneten Lösungsmitteln wie z.B. Acetanhydrid zu einem Olefin der allgemeinen Formel **18** fragmentiert.

Die Verbindungen der allgemeinen Formeln **14, 15, 16, 17** und **18** zusammen mit den im Text beschriebenen Derivaten sind alle neu und gehören als Zwischenverbindungen der allgemeinen Formel V zum Gegenstand vorliegender Erfindung: 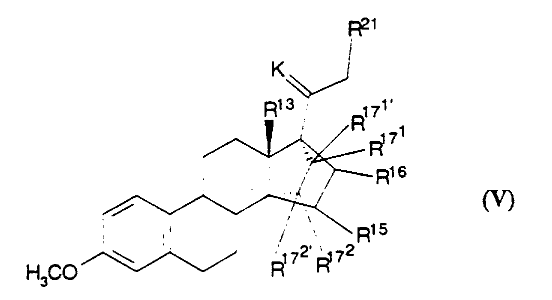 worin
R¹³ = CH₃, -C₂H₅,
R¹⁵ und R¹⁶ = gemeinsam einen Ring der Teilformeln 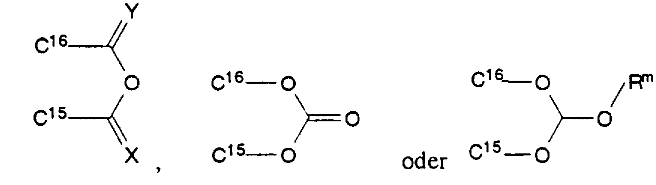 bedeuten,
   worin
X und Y = unabhängig voneinander jeweils ein Sauerstoffatom oder zwei Wasserstoffatome und
R^{m} = C₁-C₃-Alkyl
bedeuten, oder
R¹⁵ und R¹⁶ = jedes für sich eine -OH Gruppe oder
R¹⁵ und R¹⁶ = gemeinsam eine Bindung und
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff oder C₁-C₃-Alkyl
bedeuten.

Weitere Substitutionsmuster am D-Ring der 14,17-C2-überbrückten Steroide lassen sich z.B. ausgehend von den Diels-Alder Produkten der Formel **19**, darstellbar durch Umsetzung eines Diens der allgemeinen Formel **4** mit einem Acetylencarbonsäurealkylester (Alkyl = C₁-C₄-Alkyl), erzeugen: 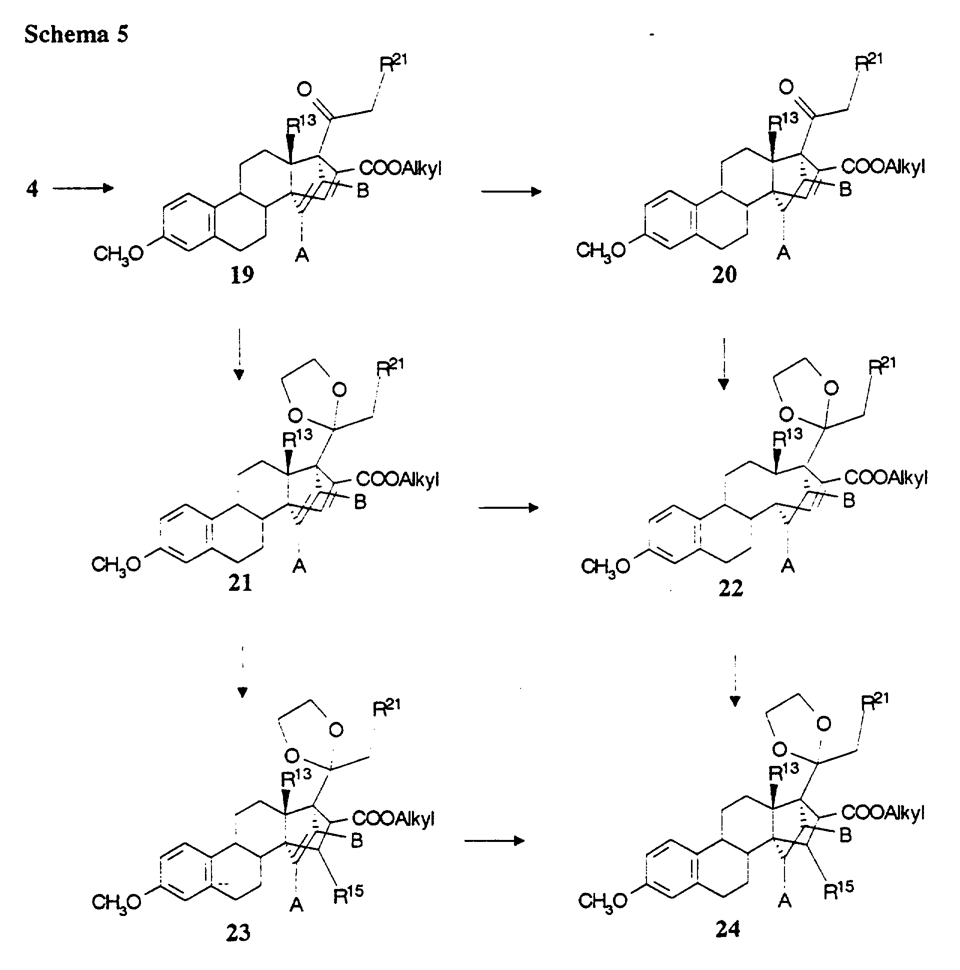 R¹³ = -CH₃, -C₂H₅; R²¹ = Wasserstoff, C₁-C₃-Alkyl; A und B = unabhängig voneinander Wasserstoff oder C1-C3-Alkyl; R¹⁵ = Wasserstoff, C₁-C₃-Alkyl

Ketalisierung des Cycloadditionsproduktes **19** ergibt eine Verbindung der allgemeinen Formel **21**. Die selektive Reduktion der 15,16-Doppelbindung gelingt mit Magnesium in einem geeigneten Lösungsmittel, bevorzugt einem Alkohol wie etwa Methanol, und ergibt eine Verbindung der Formel **23,** worin R¹⁵ dann ein Wasserstoffatom bedeutet, 1,4-Additionen an Verbindungen der Formel **21** werden nach an sich bekannten Methoden durchgeführt. So ergibt beispielsweise die Umsetzung mit Dimethylkupfer in geeigneten Lösungsmitteln wie etwa Tetrahydrofuran eine Verbindung der allgemeinen Formel **23**, worin R¹⁵ dann eine Methylgruppe bedeutet. Durch katalytische Hydrierung an Edelmetallkatalysatoren läßt sich bei Bedarf auf jeder Zwischenstufe die 17¹,17²-Doppelbindung selektiv entfernen. Die Esterfunktion an C¹⁶ kann in vielfältiger Weise modifiziert werden. Zusätzlich zu den bereits für die Folgechemie der Cycloaddition mit Acrylsäurealkylestern beschriebenen Möglichkeiten seien hier die folgenden erwähnt:
α,β-gesättigte Ester, wie etwa Verbindungen der allgemeinen Formeln **23** und **24**, ergeben nach Reduktion mit Lithiumaluminiumhydrid, Überführung des entstandenen Alkoholes in eine Abgangsgruppe, wie etwa einen Sulfonsäureester, der z.B. durch Umsetzung mit einem Sulfonsäurehalogenid unter Verwendung geeigneter Basen wie etwa Pyridin mit oder ohne Zuhilfenahme eines inerten Lösungsmittels wie etwa Dichlormethan erhalten wird und anschließender Reduktion mit geeigneten Reduktionsmitteln, beispielsweise Lithiumtriethylborhydrid, die 16-Methylderivate
α,β-ungesättigte Ester, wie etwa Verbindungen der allgemeinen Formeln **21** und **22,** ergeben bei Behandlung mit geeigneten Reduktionsmitteln wie etwa Diisobutylaluminiumhydrid, gegebenenfalls unter Zuhilfenahme von Lewissäuren, beispielsweise Zinkchlorid, die 15,16-ungesättigten 16-Hydroxymethylderivate. Die Überführung in die entsprechenden Carbonsäureester beziehungsweise Sulfonsäureester gelingt nach an sich bekannten Methoden. Beispielsweise wird der Allylalkohol mit Acetylchlorid in Pyridin zum entsprechenden Essigsäureester umgesetzt. Unter den Bedingungen einer Birch-Reduktion wird dann das entsprechende 15,16-ungesättigte 16-Methylderivat erhalten (zur Birch-Reduktion von Allylacetaten vergleiche beispielsweise R. T. Jacobs et al., *J. Org. Chem.* **55**, 4051 (1990)). Dabei wird auch der aromatische A-Ring unter Ausbildung der 2,5(10)-Dienstruktur reduziert.

Die Verbindungen der allgemeinen Formeln **19, 20, 21, 22, 23** und **24** zusammen mit den im Text beschriebenen Derivaten sind alle neu und gehören als Zwischenverbindungen der allgemeinen Formel VI zum Gegenstand vorliegender Erfindung: 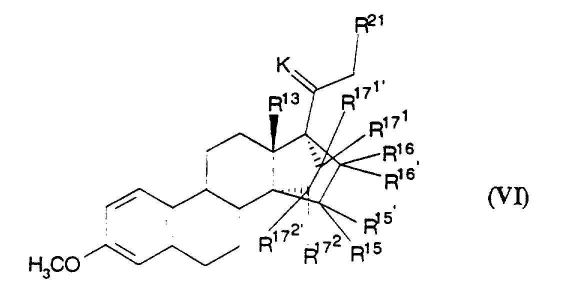 worin
R¹³ = -CH₃, -C₂H₅,
R¹⁵ und R¹⁶ = jeweils Wasserstoff oder gemeinsam eine Bindung,
R^{15'} = Wasserstoff oder C₁-C₃-Alkyl,
R^{16'} = -COOAlkyl, wobei Alkyl ein C₁-C₄-Alkylrest ist, oder CH₂OH, oder CHO, oder einen C₁-C₃-Alkylrest,
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff oder C₁ - C₃ -Alkyl
bedeuten.

In den vorstehenden Verbindungen der allgemeinen Formeln III, IV, V und VI steht K, wenn es sich um eine Ketalschutzgruppe handelt, vorzugsweise für eine 1,2-Ethandiylbis(oxy)- oder 2,2-Dimethyl-1,3-propandiylbis(oxy)gruppe.

Die Reduktion der so erhaltenen Verbindungen der allgemeinen Formeln **6, 7, 8** und **9** als auch der entsprechenden Derivate, die in den Positionen 15, 16, 17¹ oder 17² substituiert sind unter den an sich bekannten Bedingungen einer Birch-Reduktion (siehe beispielsweise J. Fried, J.A. Edwards, Organic Reactions in Steroid Chemistry, von Nostrand Reinhold Company 1972, S. 1-60) führt zu den entsprechenden 3-Methoxy-Δ2,Δ5(10)-Derivaten. Diese können durch Reaktion mit verdünnten Mineralsäuren und gegebenenfalls einer anschließenden Oxidation der 20-Hydroxygruppe nach Standardverfahren, wie zum Beispiel mit Pyridiniumdichromat, zu den erfindungsgemäßen Δ4-3-Ketonen der allgemeinen Formel (I) umgesetzt werden. Die 3-Methoxy-Δ2,Δ5(10)-Derivate können aber auch nach Standardverfahren (siehe beispielsweise D. Burn und V. Petrow *J. Chem. Soc.,* 364 (1962)) zu Δ5(10)-3-Ketonen umgesetzt werden, die sich durch eine Bromierung-Dehydrobromierung und gegebenenfalls einer anschließenden Oxidation der 20-Hydroxygruppe in die erfindungsgemäßen Δ4,Δ9-3-Ketone der allgemeinen Formel (**I**) überführen lassen (siehe beispielsweise J. Fried, J.A. Edwards, Organic Reactions in Steroid Chemistry, von Nostrand Reinhold Company 1972, S. 265-374). Ketalisierung der Δ4,Δ9-3-Ketone nach Standardverfahren führt zu den Δ5(10),Δ9(11)-3-Ketalen, die sich unter mild sauren Bedingungen, beispielsweise mit wässriger Essigsäure, zu den Δ5(10),Δ9(11)-3-Ketonen spalten lassen. Die Dekonjugation der Δ4,Δ9-3-Ketone kann gegebenenfalls auch durch Behandlung mit Säuren, beispielsweise wässrige Salzsäure unter Zusatz eines Lösungsvermittlers wie etwa Aceton, durchgeführt werden. Die Umsetzung der erhaltenen dekonjugierten Dienone mit Oxidationsmitteln (vergleiche beispielsweise DE 2748250 C2), wie etwa 2,3-Dichlor-5,6-dicyano-p-benzochinon in geeigneten Lösungsmitteln, beispielsweise Dichlormethan, führt nach Entfernung gegebenenfalls noch vorhandener Schutzgruppen zu den erfindungsgemäßen Δ4,Δ9,Δ11-3-Ketonen der allgemeinen Formel (**I**).

Die nächsten Schritte gelten in aller Regel dem Aufbau der Reste R⁶, R^{6'} und R⁷. Die Einführung einer 6,7-Doppelbindung gelingt über eine Dienoletherbromierung und anschließende Bromwasserstoffabspaltung (siehe beispielsweise J. Fried, J.A. Edwards, Organic Reactions in Steroid Chemistry, von Nostrand Reinhold Company 1972, S. 265-374) oder auch durch Umsetzung mit Chloranil oder 2,3-Dichlor-5,6-dicyano-p-benzochinon.
Die Dienoletherbromierung kann zum Beispiel analog der Vorschrift in *Steroids* **1**, 233 (1965) erfolgen. Die Bromwasserstoffabspaltung gelingt durch Erhitzen der 6-Bromverbindung mit basischen Mitteln, wie zum Beispiel Lithiumbromid oder Lithiumcarbonat in aprotischen Lösungsmitteln wie Dimethylformamid bei Temperaturen von 50-150°C oder aber indem die 6-Bromverbindungen in Collidin oder Lutidin erhitzt werden.

Für Verbindungen mit einer 6,7-Methylenfunktion erfolgt die Einführung ebenfalls aus dem Dienon durch Umsetzung mit Dimethylsulfoxoniummethylid, wobei hier allerdings ein Gemisch der α- und β-Isomeren auftritt (das Verhältnis ist abhängig von den verwendeten Substraten und liegt bei ca. 1:1), die zum Beispiel über Säulenchromatographie getrennt werden können.

Verbindungen mit R⁷ gleich Alkyl werden aus den 4,6-Dien-3-on-verbindungen durch 1,6-Addition nach bekannten Methoden hergestellt (J. Fried, J.A. Edwards: Organic Reactions in Steroid Chemistry, von Nostrand Reinhold Company 1972, Seite 75-82; A. Hosomi und H. Sakurai, *J. Am. Chem. Soc.* **99**, 1673 (1977)). Die Einführung der 7-Alkylfunktionen erfolgt hierbei in der Regel über die Dialkylkupferlithiumverbindungen.

Verbindungen in denen R⁶ ein Chloratom darstellt und R^{6'} und R⁷ eine gemeinsame zusätzliche Bindung bilden, werden ebenfalls ausgehend von den 4,6-Dien-3-on-verbindungen dargestellt. Hierzu wird zunächst die 6,7-Doppelbindung unter Verwendung organischer Persäuren, wie zum Beispiel meta-Chlorperbenzoesäure in Methylenchlorid, gegebenenfalls in Gegenwart von Natriumhydrogencarbonatlösung, epoxidiert (siehe W. Adam, J.-C. Liu und O. Rodriguez, *J. Org. Chem.* **38**, 2269 (1973)). Die Öffnung dieses Epoxids und die Eliminierung der primär gebildeten 7a-Hydroxygruppe erfolgt zum Beispiel durch Umsetzung mit Chlorwasserstoffgas in Eisessig (siehe u.a. DE-A 11 58 966 und DE-A 40 06 165).

Die Einführung einer 6-Methylengruppe kann zum Beispiel ausgehend von einem 3-Amino-3,5-dien-derivat durch Umsetzung mit Formalin in alkoholischen Lösungen unter Bildung einer 6a-Hydroxymethylgruppe und anschließender saurer Wasserabspaltung zum Beispiel mit Salzsäure in Dioxan/Wasser erfolgen. Die Wasserabspaltung kann aber auch in der Weise erfolgen, daß zunächst eine Fluchtgruppe eingeführt und dann eliminiert wird. Als Fluchtgruppen sind zum Beispiel das Mesylat, Tosylat oder Benzoat geeignet (siehe DE-A 34 02 329, EP-A 150157, US 4.584.288(86); K. Nickisch, S. Beier, D. Bittler, W. Elger, H. Laurent, W. Losert, Y. Nishino, E. Schillinger und R. Wiechert, *J. Med. Chem.* **34**, 2464 (1991)).
Eine weitere Möglichkeit zur Darstellung der 6-Methylenverbindungen besteht in der direkten Umsetzung der 4(5) ungesättigten 3-Ketone mit Acetalen des Formaldehyds in Gegenwart von Natriumacetat mit zum Beispiel Phosphoroxychlorid oder Phosphorpentachlorid in geeigneten Lösungsmitteln wie Chloroform (siehe zum Beispiel K. Annen, H. Hofmeister, H. Laurent und R. Wiechert, *Synthesis* 34, (1982)). Eine zusätzliche Möglichkeit zur Einführung der 6-Methylengruppe besteht in der Umsetzung eines Δ4-3-Ketons zu einem Dienolether, dessen Umsetzung mit Dimethylformamid und Phosphoroxychlorid zum Aldehyd und dessen Reduktion mit komplexen Borhydriden und anschließender Wasserabspaltung mit Mineralsäuren nach an sich bekannten Verfahren (siehe WO 90/12027).
Die 6-Methylenverbindungen können zur Darstellung von Verbindungen der allgemeinen Formel (**I**), in denen R⁶ gleich Methyl ist und R⁶ und R⁷ eine gemeinsame zusätzliche Bindung bilden, genutzt werden.
Hierzu kann man zum Beispiel ein von D. Burn, D.N. Kirk und V. Petrow in *Tetrahedron* **21**,1619 (1965) beschriebenes Verfahren nutzen, bei dem eine Isomerisierung der Doppelbindung durch Erwärmen der 6-Methylenverbindungen in Ethanol mit 5% Palladium-Kohle als Katalysator, der entweder mit Wasserstoff oder durch Erwärmen mit einer geringen Menge Cyclohexen vorbehandelt wurde, erreicht wird. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.
Die Darstellung von 6-Methyl-4,6-dien-3-on-derivaten kann aber auch direkt erfolgen (siehe K. Annen, H. Hofmeister, H. Laurent und R. Wiechert, *Liebigs Ann. Chem.* 712, (1983)).

Verbindungen, in denen R⁶ eine α-Methylfunktion darstellt, können aus den 6-Methylenverbindungen durch Hydrierung unter geeigneten Bedingungen dargestellt werden, Die besten Ergebnisse (selektive Hydrierung der exo-Methylenfunktion) werden durch Transfer-Hydrierung erreicht (E.A. Brande, R.P. Linstead und P.W.D. Mitchell, *J. Chem. Soc.* 3578 (1954)). Erhitzt man die 6-Methylenderivate in einem geeigneten Lösungsmittel, wie zum Beispiel Ethanol, in Gegenwart eines Hydriddonators, wie zum Beispiel Cyclohexen, und eines Edelmetallkatalysators, beispielsweise Platin oder Palladium, so kommt man in sehr guten Ausbeuten zu 6a -Methylderivaten. Geringe Anteile an 6β-Methylverbindungen können sauer isomerisiert werden (siehe zum Beispiel D. Burn, D.N. Kirk und V. Petrow, *Tetrahedron* **21**, 1619 (1965)).

Die Alkylierung von 17-Acetylderivaten zu den homologen Ketonen kann nicht nur, wie bereits beschrieben, an Verbindungen mit aromatischem A-Ring erfolgen, sondern auch im weiteren Verlauf der Synthese an geeignet geschützten Derivaten.

Die Einführung eines 21-OH-Substituenten erfolgt an geeignet geschützten 20-Ketoverbindungen nach an sich bekannten Verfahren, wie der direkten Oxidation eines Enolats (siehe beispielsweise E. Vedejs, D. A. Engler und J. E. Telschow, *J. Org. Chem.* **43**, 188 (1978) und J. C. Anderson und S. C. Smith, *Synlett* 1990, 107) oder der Umsetzung des Enolats zum entsprechenden Iodid, Substitution des Iodids durch Acetat und Hydrolyse des Acetats. Die dabei gegebenenfalls entstehenden Diastereomerengemische können durch Chromatographie getrennt werden.

Nach Einführung aller Reste werden noch vorhandene Schutzgruppen nach Standardverfahren abgespalten.

Die erhaltenen Verbindungen der allgemeinen Formel (**I**) mit R³ gleich Sauerstoff können gewünschtenfalls durch Umsetzung mit Hydroxylaminhydrochlorid in Gegenwart von tert. Aminen bei Temperaturen zwischen -20 und +40°C in die Oxime überführt werden (allgemeine Formel (**I**) mit R³ in der Bedeutung von N-OH, wobei die Hydroxygruppe syn- oder anti-ständig sein kann).

Die Entfernung der 3-Oxogruppe zu einem Endprodukt der allgemeinen Formel (**I**) mit R³ in der Bedeutung von zwei Wasserstoffatomen kann beispielsweise nach der in DE-A-2805490 angegebenen Vorschrift durch reduktive Spaltung des Thioketals erfolgen.

Die nachfolgenden Beispiele dienen der näheren Erläuterung der Erfindung:

### Beispiel 1

### 14,17-Etheno-19-norpregn-4-en-3,20-dion

### a) 3-Methoxy-19-norpregna-1,3,5(10),14,16-pentaen-20-on

84,2 g 3-Methoxy-19-nor-17α-pregna-1,3,5(10),15-tetraen-20-in-17β-ol *(J. Med. Chem.,* **11**, 924 (1968)) werden in 875 ml 86-prozentiger Ameisensäure unter Rühren auf 110 °C erhitzt. Nach 2 Stunden läßt man unter Zugabe von 1000 ml Wasser abkühlen. Der ausgefallene Feststoff wird abfiltriert, getrocknet und an Kieselgel mit einem Gemisch aus Ethylacetat und Hexan chromatographiert. Es werden 47,8 g 1a) erhalten.
Fp.: 152-155 °C
¹H-NMR (CDCl₃): δ = 1,22 ppm (s, 3H, H-18); 2,35 (s, 3H, H-21); 3,78 (s, 3H, 3-OCH₃); 6,08 (m, 1H, H-15); 6,68 (d, J = 3 Hz, 1H, H-4); 6,74 (dd, J = 9, 3 Hz, 1H, H-2); 7,23 (d, J = 9 Hz, 1H, H-1); 7,27 (d, J = 3 Hz, 1H, H-16)

### b) 3-Methoxy-14,17-etheno-19-norpregna-1,3,5(10)-trien-20-on

Eine Lösung von 200 g der unter 1a) beschriebenen Substanz in 2,5 l Benzol wird unter einem Ethylendruck von 300 bar für 240 Stunden auf 160 °C erhitzt. Nach dem Erkalten wird das Reaktionsgemisch eingeengt und der Rückstand an Kieselgel mit einem Gemisch aus Ethylacetat und Hexan chromatographiert. Es werden 175 g 1b) erhalten.
¹H-NMR (CDCl₃): d = 0,91 ppm (s, 3H, H-18); 2,22 (s, 3H, H-21); 3,78 (s, 3H, 3-OCH₃); 6,07 und 6,14 (2d, J = 6 Hz, je 1H, H-17¹ und H-17²); 6,65 (d, J = 3 Hz, 1H, H-4); 6,73 (dd, J = 9, 3 Hz, 1H, H-2); 7,22 (d, J = 9 Hz, 1H, H-1)

### c) 20,20-[1,2-Ethandiylbis(oxy)]-3-methoxy-14,17-etheno-19-norpregna-1,3,5(10)-trien

Zu einer Lösung von 25 g der unter 1b) beschriebenen Verbindung in 175 ml Dichlormethan werden bei Raumtemperatur unter Rühren 75 ml Ethylenglykol, 63 ml Trimethylorthoformiat und 1,25 g p-Toluolsulfonsäure gegeben. Nach 90 Minuten werden 15 ml Triethylamin und 100 ml Dichlormethan zugesetzt und das Reaktionsgemisch dreimal mit konzentrierter Natriumhydrogencarbonatlösung gewaschen. Die organische Phase wird über Kaliumcarbonat getrocknet, abfiltriert und eingeengt. Es werden 31 g 1c) erhalten.
¹H-NMR (CDCl₃): d = 0.98 ppm (s, 3H, H-18); 1,37 (s, 3H, H-21); 3,78 (s, 3H, 3-OCH₃); 3,95-4,05 (m, 4H, 20-OCH₂CH₂O-): 5,97 und 6,01 (2d, J = 6 Hz, je 1H, H-17¹ und H-17²): 6,65 (d, J = 3 Hz, 1H, H-4); 6,72 (dd, J = 9, 3 Hz, 1H, H-2); 7,22 (d, J = 9 Hz, 1H, H-1)

### d) 20,20-[1,2-Ethandiylbis(oxy)]-3-methoxy-14,17-etheno-19-norpregna-2,5(10)-dien

Zu 2,2 l flüssigem Ammoniak wird bei -70 °C eine Lösung von 31 g der unter 1c) beschriebenen Verbindung in einem Gemisch aus 400 ml Tetrahydrofuran und 70 ml tert.-Butanol gegeben. Zu diesem Gemisch werden unter Rühren 16 g Lithium portionsweise zugegeben. Man läßt auf -40 °C erwärmen, tropft nach 5,5 Stunden 350 ml Ethanol zu, läßt die Mischung dann auf Raumtemperatur erwärmen, verdünnt mit Wasser und extrahiert mit Ethylacetat. Die organische Phase wird mit Wasser und konzentrierter Natriumchloridlösung gewaschen, über Natriumsulfat getrocknet, abfiltriert und im Vakuum eingeengt. Es werden 23,1 g kristallines 1d) erhalten, welches ohne weitere Reinigung in Folgestufen umgesetzt wird.
¹H-NMR (CDCl₃): d = 0.96 ppm (s, 3H, H-18); 1,33 (s, 3H, H-21); 3,55 (s, 3H, 3-OCH₃); 3,88-4,03 (m, 4H, 20-OCH₂CH₂O-); 4,63-4,67 (m, 1H, H-2); 5,93 und 6,07 (2d, J = 6 Hz, je 1H, H-17¹ und H-17²)

### e) 14,17-Etheno-19-norpregn-4-en-3,20-dion

Eine Lösung von 2,7 g der unter 1d) beschriebenen Verbindung in 30 ml Tetrahydrofuran und 150 ml Aceton wird unter Rühren mit 7,8 ml 4-normaler Salzsäure versetzt. Nach 2 Stunden wird das Lösungsmittel entfernt und der Rückstand aus Diisopropylether umkristallisiert. Es werden 1,72 g 1e) erhalten.
Fp.: 139 - 143 °C
¹H-NMR (CDCl₃): d = 0,92 ppm (s, 3H, H-18); 2,18 (s, 3H, H-21); 5,88 (s breit, 1H, H-4); 6,04 (s, 2H, H-17¹ und H-17²)

### Beispiel 2

### 14,17-Etheno-19-norpregna-4,6-dien-3,20-dion

### a) 3-Ethoxy-14,17-etheno-19-norpregna-3,5-dien-20-on

Zu einer Lösung von 2,02 g der unter 1e) beschriebenen Verbindung in 80 ml Tetrahydrofuran werden unter Rühren 6,1 ml Ethanol, 6,1 ml Triethylorthoformiat und 145 mg p-Toluolsulfonsäure gegeben. Nach 2 Stunden bei Raumtemperatur werden 2,5 ml Triethylamin zugegeben, mit Natriumhydrogencarbonatlösung verdünnt und das Gemisch mit Ethylacetat extrahiert. Die organische Phase wird mit Wasser und konzentrierter Natriumchloridlösung gewaschen, über Natriumsulfat getrocknet, abfiltriert und eingeengt. Es werden 3,3 g 2a) als farbloses Öl erhalten, welches ohne weitere Reinigung in der Folgestufe umgesetzt wird.

### b) 14,17-Etheno-19-norpregna-4,6-dien-3,20-dion

Eine Lösung von 3,3 g der unter 2a) beschriebenen Verbindung in 41 ml Dioxan und 10 ml Wasser wird mit 16 ml einer 10-prozentigen Natriumacetatlösung und dann bei 0 °C unter Rühren mit 890 mg 1,3-Dibrom-5,5-dimethylhydantoin versetzt. Nach 15 Minuten wird das Reaktionsgemisch auf Eiswasser gegossen und mit Ethylacetat extrahiert. Die organische Phase wird mit konzentrierter Natriumchloridlösung gewaschen, über Natriumsulfat getrocknet und in eine Suspension aus 2,4 g Lithiumcarbonat und 3,4 g Lithiumbromid in 120 ml Dimethylformamid filtriert. Das Gemisch wird unter Abdestillieren des Ethylacetats auf 150 °C erhitzt. Nach einer Stunde läßt man abkühlen, verdünnt das Reaktionsgemisch mit Wasser und extrahiert mit Ethylacetat. Die organische Phase wird mit Wasser und konzentrierter Natriumchloridlösung gewaschen, über Natriumsulfat getrocknet, abfiltriert und eingeengt. Der Rückstand wird an Kieselgel mit einem Gemisch aus n-Hexan und Ethylacetat chromatographiert. Es werden 880 mg 2b) erhalten.
Fp.: 150-152 °C [a]_{D}²⁰ = + 172,3° (CHCl₃; c = 0,510)
¹H-NMR (CDCl₃): d = 0,95 ppm (s, 3H, H-18); 2,19 (s, 3H, H-21); 5.82 (s breit, 1H, H-4); 5,92 und 6,04 (2d, J = 6 Hz, je 1H, H-17¹ und H-17²); 6,20-6,32 (m, 2H, H-6 und H-7)

### Beispiel 3

### 7β-Methyl-14,17-etheno-19-norpregn-4-en-3,20-dion

Eine Suspension aus 1,9 g Kupfer(I)jodid in 25 ml Diethylether wird bei 0 °C tropfenweise mit 8,5 ml einer 1,6-molaren Lösung von Methyllithium in Diethylether versetzt. Nach 30 Minuten Rühren bei 0 °C werden 40 ml Tetrahydrofuran zugesetzt und dann bei -40 °C 1,23 ml Bortrifluoridetherat und danach eine Lösung aus 340 mg der unter 2b) beschriebenen Verbindung in 15 ml Tetrahydrofuran zugetropft. Man läßt innerhalb von 4 Stunden auf Raumtemperatur erwärmen, rührt noch 72 Stunden und gießt das Reaktionsgemisch in 100 ml konzentrierte Ammoniumchloridlösung. Das Gemisch wird viermal mit Ethylacetat extrahiert, die vereinigten organischen Phasen mit Wasser gewaschen, über Natriumsulfat getrocknet, abfiltriert und eingeengt. Nach Chromatographie an Kieselgel mit einem Gemisch aus Ethylacetat und Hexan werden 46 mg 3) erhalten.
Fp.: 133-135 °C
¹H-NMR (CDCl₃): d = 0,94 ppm (s, 3H, H-18); 1,07 (d, J = 7,5 Hz, 3H, 7-CH₃); 2,20 (s, 3H. H-21); 5,83 (s breit, 1H, H-4); 6,05 (s, 2H, H-17¹ und H-17²)

### Beispiel 4

### 14,17-Etheno-19-norpregna-4,9-dien-3,20-dion

### a) 14,17-Etheno-19-norpregn-5(10)-en-3,20-dion

Zu einer Suspension von 3,0 g der unter 1d) beschriebenen Verbindung in 60 ml Aceton wird unter Rühren bei Raumtemperatur eine Lösung von 2,1 g Oxalsäuredihydrat in 30 ml Wasser zugetropft. Nach 2 Stunden wird mit 150 ml konzentrierter Natriumhydrogencarbonatlösung versetzt und dreimal mit Ethylacetat extrahiert. Die vereinigten organischen Phasen werden mit konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 1,51 g 4a) erhalten.
Fp.: 96-110 °C [a]_{D}²⁰ = + 231,6° (CHCl₃; c = 0,505)
¹H-NMR (CDCl₃): δ = 1,03 ppm (s, 3H, H-18); 2,20 (s, 3H, H-21); 2,72 und 2,82 (2d breit, J = 20 Hz, je 1H, H-4); 6,04 und 6,10 (2d, J = 6 Hz, je 1H, H-17¹ und H-17²)

### b) 14,17-Etheno-19-norpregna-4,9-dien-3,20-dion

Eine Lösung von 500 mg der unter 4a) beschriebenen Verbindung in 6,5 ml Pyridin wird unter Rühren mit 530 mg Pyridiniumbromid-perbromid versetzt, für eine Stunde bei Raumtemperatur und dann noch für 2 Stunden bei 50 °C gerührt. Nach dem Erkalten wird das Reaktionsgemisch in 20 ml 6-normale Salzsäure eingerührt und dreimal mit Ethylacetat extrahiert. Die vereinigten organischen Phasen werden mit Wasser und konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 0,31 g 4b) erhalten.
Fp.: 152-158 °C [a]_{D}²⁰ = -200° (CHCl₃, c = 0,496)
¹H-NMR (CDCl₃): d = 1,04 ppm (s, 3H, H-18); 2,20 (s, 3H, H-21); 5,72 (s breit, 3H, H-4); 6,03 (s, 2H, H-17¹ und H-17²)

### Beispiel 5

### 21-Hydroxy-14,17-etheno-19-norpregn-4-en-3,20-dion

### a) 3,3;20,20-Bis[2,2-dimethyl-1,3-propandiylbis(oxy)]-14,17-etheno-19-norpregn-5(10)-en

Zu einer Lösung von 3,2 g der unter 1e) beschriebenen Verbindung in 30 ml Toluol werden unter Rühren 2,08 g 2,2-Dimethylpropan-1,3-diol, 2,7 ml Trimethylorthoformiat und 190 mg p-Toluolsulfonsäure gegeben. Nach 2 Stunden wird mit 5 ml Triethylamin versetzt, mit Ethylacetat verdünnt, fünfmal mit Wasser und einmal mit konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 3,85 g 5a) als Schaum erhalten.
¹H-NMR (CDCl₃): d = 0,72, 0,88, 0,94, 1,07 und 1,19 ppm (5s, 15H, Ketal-CH₃ und H-18); 1,43 (s, 3H, H-21); 3,17-3,78 (m, 8H, Ketal-OCH₂); 5,88 und 5,95 (2d, J = 6 Hz, je 1H, H-17¹ und H-17²)

### b) 3,3-[2,2-Dimethyl-1,3-propandiylbis(oxy)]-14,17-etheno-19-norpregn-5(10)-en-20-on

Eine Lösung aus 3,85 g der unter 5a) beschriebenen Verbindung in 50 ml Dichlormethan wird mit 11 g Kieselgel (0,063 - 0,2 mm) und 1,1 ml konzentrierter wäßriger Oxalsäurelösung versetzt und für 30 Minuten intensiv gerührt. Man gibt 100 ml 1-normale Natronlauge und 100 ml Dichlormethan zu, rührt fünf Minuten, läßt absitzen, filtriert, wäscht den Rückstand mit Dichlormethan, wäscht die vereinigten organischen Phasen mit konzentrierter Natriumchloridlösung, trocknet über Natriumsulfat, filtriert und engt ein. Der Rückstand wird an Kieselgel mit einem Gemisch aus Ethylacetat und Hexan chromatographiert. Es werden 1,93 g 5b) als Schaum erhalten.
¹H-NMR (CDCl₃): d = 0,85 und 0,88 ppm (2s, 6H, Ketal-CH₃); 1,08 (s, 3H, H-18); 2,18 (s, 3H, H-21); 3,42-3,70 (m, 4H, Ketal-OCH₂); 5,98 und 6,07 (2d, J = 6 Hz, je 1H, H-17¹ und H-17²)

### c) 3,3-[2,2-Dimethyl-1,3-propandiylbis(oxy)]-21-jod-14,17-etheno-19-norpregn-5(10)-en-20-on

Zu einer Lösung von 1,9 ml N-Cyclohexylisopropylamin in 10 ml Tetrahydrofuran werden bei -40 °C 3,9 ml einer 1,6-molaren Lösung von n-Butyllithium in Hexan getropft. Nach 15 Minuten Rühren wird eine Lösung von 1,93 g der unter 5b) beschriebenen Substanz in 15 ml Tetrahydrofuran zugetroft. Nach 30 Minuten Rühren bei -30 °C wird die Lösung auf -50 °C gekühlt und dann via Teflonschlauch zu einer auf -50 °C gekühlten Lösung aus 1,37 g Jod in 10 ml Tetrahydrofuran gepumpt. Die Reaktionsmischung wird binnen 2 Stunden auf Raumtemperatur erwärmt, dann auf konzentrierte Ammoniumchloridlösung gegossen und mit Ethylacetat extrahiert. Die organische Phase wird mit konzentrierter Natriumthiosulfatlösung und konzentrierter Natriumhydrogencarbonatlösung gewaschen, über Natriumsulfat getrocknet und eingeengt. Es werden 2,6 g 5c) als hellgelbes Harz erhalten, welches ohne weitere Reinigung in der Folgestufe umgesetzt wird.
¹H-NMR (CDCl₃): d = 0,88 ppm (s, 6H, Ketal-CH₃); 1,08 (s, 3H, H-18); 3,42-3,70 (m, 4H, Ketal-OCH₂); 3,90 und 3,99 (2d, J = 12 Hz, je 1H, H-21); 6,07-6,18 (m, 2H, H-17¹ und H-17²)

### d) 21-(Acetyloxy)-3,3-[2,2-dimethyl-1,3-propandiylbis(oxy)]-14,17-etheno-19-norpregn-5(10)-en-20-on

Eine Lösung von 2,6 g der unter 5c) beschriebenen Substanz in 10 ml Dimethylformamid wird mit 4,9 g Kaliumacetat versetzt, 80 Minuten bei 80 °C gerührt, nach dem Erkalten auf Wasser gegossen und mit Ethylacetat extrahiert. Die organische Phase wird mit konzentrierter Natriumchloridlösung gewaschen, über Natriumsulfat getrocknet und eingeengt. Es werden 1,99 g 5d) als farbloses Harz erhalten, welches ohne weitere Reinigung in der Folgestufe umgesetzt wird.
¹H-NMR (CDCl₃): d = 0,88 ppm (s, 6H, Ketal-CH₃); 1,08 (s, 3H, H-18); 2,17 (s, 3H, Acetyloxy-CH₃); 3,42-3,72 (m, 4H, Ketal-OCH₂); 4,67 und 4,85 (2d, J = 15 Hz, je 1H, H-21); 5,99 und 6,12 (2d, J = 6 Hz, je 1H, H-17¹ und H-17²)

### e) 21-(Acetyloxy)-14,17-etheno-19-norpregn-5(10)-en-3,20-dion

Eine Lösung von 1,99 g der unter 5d) beschriebenen Substanz in 10 ml Tetrahydrofuran wird mit 100 ml 70%iger Essigsäure versetzt und für 60 Minuten bei Raumtemperatur und anschließend 60 Minuten bei 40 °C gerührt. Das Reaktionsgemisch wird auf Wasser gegossen, mit Natronlauge neutralisiert und mit Ethylacetat dreimal extrahiert. Die vereinigten organischen Phasen werden mit konzentrierter Natriumchloridlösung gewaschen, über Natriumsulfat getrocknet und eingeengt. Der Rückstand wird an Kieselgel mit einem Gemisch aus Ethylacetat und Hexan chromatographiert. Es werden 1,15 g 5e) erhalten.
Fp.: 126-128 °C [α]_{D}²⁰ = +199,6° (CHCl₃, c = 0,500)
¹H-NMR (CDCl₃): d = 0,90 ppm (s, 3H, H-18); 2,18 (s, 3H, Acetyloxy-CH₃); 4,67 und 4,84 (2d, J = 16 Hz, je 1H, H-21); 6,02 und 6,14 (2d, J = 6 Hz, je 1H, H-17¹ und H-17²)

### f) 21-(Acetyloxy)-14,17-etheno-19-norpregn-4-en-3,20-dion

Eine Lösung von 500 mg der unter 5e) beschriebenen Substanz in 25 ml Aceton wird mit 1 ml 4-normaler Salzsäure versetzt, für 30 Minuten bei Raumtemperatur gerührt und dann bis zur Trockene eingeengt. Es werden 500 mg 5f) als Schaum erhalten, welcher ohne weitere Reinigung in der Folgestufe umgesetzt wird.
¹H-NMR (CDCl₃): d = 0,93 ppm (s, 3H, H-18); 2,18 (s, 3H, Acetyloxy-CH₃); 4,68 und 4,83 (2d, J = 16 Hz, je 1H, H-21); 5,86 (s breit, 1H, H-4); 6,02 und 6,10 (2d, J = 6 Hz, je 1H, H-17¹ und H-17²)

### g) 21-Hydroxy-14,17-etheno-19-norpregn-4-en-3,20-dion

Eine Lösung von 500 mg der unter 5f) beschriebenen Substanz in 15 ml Methanol wird mit 1,8 ml 10%iger wäßriger Kaliumcarbonatlösung versetzt, für 30 Minuten bei Raumtemperatur gerührt und dann auf Wasser gegossen. Man säuert mit 1-normaler Salzsäure auf pH 5 an, extrahiert dreimal mit Ethylacetat, wäscht die vereinigten organischen Phasen mit konzentrierter Natriumchloridlösung, trocknet über Natriumsulfat, filtriert und engt ein. Der Rückstand wird an Kieselgel mit einem Gemisch aus Ethylacetat und Hexan chromatographiert. Es werden 282 mg 5g) erhalten.
Fp.: 160-163 °C [a]_{D}²⁰ = +162,3° (CHCl₃, c = 0,510)
¹H-NMR CDCl₃): d = 0,95 ppm (s, 3H, H-18); 3,32 (t, J = 5 Hz, 1H, OH); 4,23 und 4,42 (2dd, J = 16 Hz und 5 Hz, je 1H, H-21); 5,87 (s breit, 1H, H-4); 5,87 und 6,10 (2d, J = 6 Hz, je 1H, H-17¹und H-17²)

### Beispiel 6

### 21-Hydroxy-14,17-etheno-19-norpregna-4,9-dien-3,20-dion

### a) 21-(Acetyloxy)-14,17-etheno-19-norpregn-4,9-dien-3,20-dion

540 mg der unter 5e) beschriebenen Substanz werden nach der in Beispiel 4b) beschriebenen Methode umgesetzt. Man erhält 292 mg 6a).
Fp.: 182-184 °C [a]_{D}²⁰ = -106,6° (CHCl₃, c = 0,495)
¹H-NMR (CDCl₃): d = 1,04 ppm (s, 3H, H-18); 2,19 (s, 3H, Acetyloxy-CH₃); 4,69 und 4,83 (2d, J = 16 Hz, je 1H, H-21); 5,72 (s breit, 1H, H-4); 6,02 und 6,10 (2d, J = 6 Hz, je 1H, H-17¹ und H-17²)

### b) 21-Hydroxy-14,17-etheno-19-norpregna-4,9-dien-3,20-dion

270 mg der unter 6a) beschriebenen Substanz werden nach der in Beispiel 5g) beschriebenen Methode umgesetzt. Man erhält 159 mg 6b).
Fp.: 143-146 °C [a]_{D}²⁰ = -171,4° (CHCl₃, c = 0,505)
¹H-NMR CDCl₃); d = 1,04 ppm (s, 3H, H-18); 3,33 (s breit, 1H, OH); 4,24 und 4,43 (2d breit, J = 16 Hz, je 1H, H-21); 5,72 (s breit, 1H, H-4); 5,95 und 6,10 (2d, J = 6 Hz, je 1H, H-17¹ und H-17²)

### Beispiel 7

### 21-Methyl-14,17-etheno-19-norpregn-4-en-3,20-dion

### a) 3-Methoxy-21-methyl-14,17-etheno-19-norpregna-1,3,5(10)-trien-20-on

Zu einer Lösung aus 1,5 ml Diisopropylamin in 15 ml Tetrahydrofuran tropft man bei -20 °C 6,6 ml einer 1,6-molaren Lösung von n-Butyllithium in Hexan, rührt anschließend noch 30 Minuten bei 0 °C, tropft dann bei -30 °C eine Lösung aus 2,4 g der unter 1b) beschriebenen Substanz und 0,78 ml 1,3-Dimethylimidazolin-2-on in 46 ml Tetrahydrofuran zu und läßt noch 30 Minuten bei -30 °C rühren. Anschließend tropft man 0,66 ml Methyljodid zu und läßt auf 0 °C erwärmen. Man rührt die Reaktionsmischung in konzentrierte Ammoniumchloridlösung ein, verdünnt mit Wasser, extrahiert dreimal mit Ethylacetat, wäscht die vereinigten organischen Phasen mit konzentrierter Natriumchloridlösung, trocknet über Natriumsulfat, filtriert und engt ein. Der Rückstand wird aus Diisopropylether kristallisiert. Es werden 2,12 g 7a) erhalten.
Fp.: 94 °C [a]_{D}²⁰ = +170,8° (CHCl₃, c = 0,505)
¹H-NMR CDCl₃): d = 0,89 ppm (s, 3H, H-18); 1,08 (t, J = 7,5 Hz, 3H, H-22); 3,79 (s, 3H, 3-OCH₃); 6,05 und 6,12 (2d, J = 6 Hz, je 1H, H-17¹ und H-17²); 6,64 (d, J = 3 Hz, 1H, H-4); 6,72 (dd, J = 9, 3 Hz, 1H, H-2); 7,22 (d, J = 9 Hz, 1H, H-1)

### b) 21-Methyl-14,17-etheno-19-norpregn-4-en-20-ol-3-on

1,9 g der unter 7a) beschriebenen Substanz werden nach der in Beispiel 1d) beschriebenen Methode umgesetzt. Das Rohprodukt wird an Kieselgel mit einem Gemisch aus Ethylacetat und Hexan chromatographiert. Es werden 750 mg Zwischenprodukt erhalten, die nach der in Beispiel le) beschriebenen Methode umgesetzt werden. Nach Chromatographie an Kieselgel mit einem Gemisch aus Ethylacetat und Hexan erhält man 317 mg 7b).
¹H-NMR (CDCl₃); d = 0,89 (0,92) ppm (s, 3H, H-18); 1,05 (1,03) (t, J = 7,5 Hz, 3H, H-22); 3,70 (dd, J = 8 und 3 Hz, 1H, H-20); 5,83 (5,85) (s breit, 1H, H-4); 5,89 und 5,94 (5,96 und 6,02) (2d, J = 6 Hz, je 1H, H-17¹ und H-17²)
(Signale des 2. Diastereomeren in Klammern)

### c) 21-Methyl-14,17-etheno-19-norpregn-4-en-3,20-dion

Zu einer Suspension von 1,67 g Pyridiniumdichromat in 15 ml Dimethylformamid wird unter Rühren eine Lösung von 300 mg der unter 7b) beschriebenen Verbindung in 40 ml Dichlormethan gegeben. Die Mischung wird eine Stunde bei Raumtemperatur gerührt, dann mit 50 ml Ethylacetat versetzt, eine weitere Stunde gerührt und dann filtriert. Das Filtrat wird wird fünfmal mit Wasser und dann mit konzentrierter Natriumchloridlösung gewaschen, über Natriumsulfat getrocknet, abfiltriert und eingeengt. Der Rückstand wird mittels HPLC gereinigt. Es werden 100 mg 7c) erhalten.
Fp.: 140-149 °C [a]_{D}²⁰ = +147,0° (CHCl₃; c = 0,510)
¹H-NMR (CDCl₃); d = 0,90 ppm (s, 3H, H-18); 1,06 (t, J = 7,5 Hz, 3H, H-22); 5,86 (s breit, 1H, H-4); 6,02 (s, 2H, H-17¹ und H-17²)

### Beispiel 8

### 21-Methyl-14,17-etheno-19-norpregna-4,9-dien-3,20-dion

### a) 20,20-[1,2-Ethandiylbis(oxy)]-3-methoxy-31-methyl-14,17-etheno-19-norpregna-1,3,5(10)-trien

Zu einer Lösung von 21,3 g der unter 7a) beschriebenen Verbindung in 250 ml Toluol werden bei Raumtemperatur unter Rühren 62 ml Ethylenglykol, 52 ml Trimethylorthoformiat und 1,0 g p-Toluolsulfonsäure gegeben. Man erhitzt für 8 Stunden auf 60 °C. Nach dem Erkalten werden 15 ml Triethylamin und 250 ml Ethylacetat zugesetzt und das Gemisch dreimal mit konzentrierter Natriumhydrogencarbonatlösung gewaschen. Die organische Phase wird über Kaliumcarbonat getrocknet, abfiltriert und eingeengt. Es werden 27 g 8a) erhalten, welches ohne weitere Reinigung in der Folgestufe umgesetzt wird.
¹H-NMR (CDCl₃): d = 0,94 ppm (t, J = 7,5 Hz, 3H, H-22); 0,96 (s, 3H, H-18); 3,78 (s, 3H, 3-OCH₃); 3,95-4,18 (m, 4H, 20-OCH₂CH₂O-); 5,98 (s, 2H, H-17¹ und H-17²); 6,64 (d, J = 3 Hz, 1H, H-4); 6,72 (dd, J = 9, 3 Hz, 1H, H-2); 7,21 (d, J = 9 Hz, 1H, H-1)

### b) 20,20-[1,2-Ethandiylbis(oxy)]-3-methoxy-21-methyl-14,17-etheno-19-norpregna-2,5(10)-dien

27 g der unter 8a) beschriebenen Substanz werden nach der in Beispiel 1d) beschriebenen Methode umgesetzt. Man erhält 18,9 g 8b).
¹H-NMR (CDCl₃): d = 0,93 ppm (t, J = 7,5 Hz, 3H, H-22); 0,95 (s, 3H, H-18); 3,56 (s, 3H, 3-OCH₃); 3,93-4,10 (m, 4H, 20-OCH₂CH₂O-); 4,62-4,67 (m, 1H, H-2); 5,92 (s, 2H, H-17¹ und H-17²)

### c) 20,20-[1,2-Ethandiylbis(oxy)]-21-methyl-14,17-etheno-19-norpregn-5(10)-en-3-on

Eine Lösung aus 18,2 g der unter 8b) beschriebenen Substanz in 700 ml Tetrahydrofuran wird unter Rühren mit 250 ml konzentrierter Ammoniumchloridlösung und 18 ml konzentrierter Oxalsäurelösung versetzt und für 6 Stunden gerührt. Anschließend wird mit Wasser verdünnt und dreimal mit Ethylacetat extrahiert. Die vereinigten organischen Phasen werden mit konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 11,0 g 8c) als Schaum erhalten.
[a]_{D}²⁰ = + 169,6° (CHCl₃: c = 0.510)
¹H-NMR (CDCl₃): δ = 0,92 ppm (t, J = 7,5 Hz, 3H, H-22); 0,97 (s, 3H, H-18); 2,72 und 2,82 (2d breit, J = 20 Hz, je 1H, H-4); 3,95-4,12 (m, 4H, 20-OCH₂CH₂O-); 5,87-5,98 (m, 2H, H-17¹ und H-17²)

### d) 21-Methyl-14,17-etheno-19-norpregna-4,9-dien-3,20-dion

11 g der unter 8c) beschriebenen Substanz werden nach der in Beispiel 4b) beschriebenen Methode umgesetzt. Man erhält 3,75g 8d).
Fp.: 145-146 °C [a]_{D}²⁰ = -180,1° (CHCl₃, c = 0,510)
¹H-NMR (CDCl₃): d = 1,03 ppm (s, 3H, H-18); 1,08 (t, J = 7.5 Hz, 3H, H-22); 5,72 (s breit, 3H, H-4); 6,03 (s, 2H, H-17¹ und H-17²)

### Beispiel 9

### 21-Methyl-14,17-etheno-19-norpregna-4,9,11-trien-3.20-dion

### a) 3,3-[2,2-Dimethyl-1,3-propandiylbis(oxy)]-21-methyl-14,17-etheno-19-norpregna-5(10),9(11)-dien-20-on

Zu einer Lösung von 3,5 g der unter 8d) beschriebenen Verbindung in 30 ml Dichlormethan werden unter Rühren 2,87 g 2,2-Dimethylpropan-1,3-diol, 1,4 ml Trimethylorthoformiat und 100 mg p-Toluolsulfonsäure gegeben. Nach 3 Stunden wird mit Dichlormethan verdünnt, mit Wasser und mit konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 3,84 g 9a) als Schaum erhalten.
¹H-NMR (CDCl₃): d = 0,82 und 0,89 ppm (2s, 6H, Ketal-CH₃); 1,09 (s, 3H, H-18); 1,09 (t, J = 7,5 Hz, 3H, H-22); 3,42-3,52 (m, 2H, Ketal-OCH₂); 3,57-3,68 (m, 2H, Ketal-OCH₂); 5,45-5,53 (m, 1H, H-11); 6,03 und 6,12 (2d, J = 6 Hz, je 1H, H-17¹ und H-17²)

### b) 21-Methyl-14,17-etheno-19-norpregna-5(10),9(11)-dien-3,20-dion

500 mg der unter 9a) beschriebenen Verbindung werden mittels Ultraschall in 25 ml 70%iger Essigsäure und 5 ml Tetrahydrofuran gelöst und anschließend bei Raumtemperatur 4 Stunden gerührt. Anschließend wird unter Rühren mit konzentrierter Natriumhydrogencarbonatlösung neutralisiert. Man extrahiert dreimal mit Ethylacetat, wäscht die vereinigten organischen Phasen mit konzentrierter Natriumchloridlösung, trocknet über Natriumsulfat, filtriert ab und engt ein. Es werden 480 mg 9b) erhalten, welches ohne weitere Reinigung in der Folgestufe umgesetzt wird.
¹H-NMR (CDCl₃): δ = 0,87 ppm (s, 3H, H-18); 1,08 (t, J = 7,5 Hz, 3H, H-22); 2,91 (s breit, 2H, H-4); 5,53-5,60 (m, 1H, H-11); 6,07 und 6,13 (2d, J = 6 Hz, je 1H, H-17¹ und H-17²)

### c) 21-Methyl-14,17-etheno-19-norpregna-4,9,11-trien-3,20-dion

480 mg der unter 9b) beschriebenen Verbindung werden in 40 ml Dichlormethan gelöst und mit 600 mg 2,3-Dichlor-5.6-dicyan-p-benzochinön versetzt. Man rührt 4 Stunden bei Raumtemperatur, filtriert, wäscht das Filtrat mit konzentrierter Natriumhydrogencarbonatlösung, konzentrierter Natriumthiosulfatlösung und erneut mit konzentrierter Natriumhydrogencarbonatlösung, trocknet über Natriumsulfat, filtriert ab und engt ein. Der Rückstand wird an Kieselgel mit einem Gemisch aus Ethylacetat und Cyclohexan chromatographiert. Es werden 206 mg 9c) erhalten.
Fp.: 117-119 °C [a]_{D}²⁰ = -278,8° (CHCl₃, c = 0,500)
¹H-NMR (CDCl₃): d = 0,97 ppm (s, 3H, H-18); 1,11 (t, J = 7,5 Hz, 3H, H-22); 5,80 (s breit, 3H, H-4); 5,99 und 6,08 (2d, J = 6 Hz, je 1H, H-17¹ und H-17²); 6,04 (d, J = 12 Hz; 1H, H-11); 6,44 (d, J = 12 Hz; 1H, H-12)

### Beispiel 10

### 17¹-Methyl-14,17-etheno-19-norpregn-4-en-3,20-dion

### a) 3-Methoxy-16-methyl-19-norpregna-1,3,5(10),14,16-pentaen-20-on

Eine Suspension aus 15,2 g Kupfer(I)jodid in 50 ml Diethylether wird bei 0 °C tropfenweise mit 90 ml einer 1,6-molaren Lösung von Methyllithium in Diethylether versetzt. Nach 30 Minuten Rühren werden bei -70 °C tropfenweise 12 ml Triethylamin und danach 11 ml Trimethylchlorsilan zugesetzt. Anschließend wird eine Lösung aus 15 g der unter 1a) beschriebenen Verbindung in 220 ml Tetrahydrofuran zugetropft. Man läßt noch 2 Stunden bei -70 °C rühren, setzt dann 100 ml konzentrierte Ammoniumchloridlösung zu, läßt auf Raumtemperatur erwärmen, schüttelt mit 400 ml Ethylacetat, filtriert feste Bestandteile ab und extrahiert die wässrige Phase erneut mit Ethylacetat. Die vereinigten organischen Phasen werden viermal mit halbkonzentrierte Ammoniumchloridlösung gewaschen, über Natriumsulfat getrocknet, abfiltriert und eingeengt. Der Rückstand wird mittels Ultraschall in 500 ml Acetonitril gelöst. Zur Lösung gibt man 10,9 g Palladium(II)acetat und erhitzt für 20 Stunden auf 80 °C. Nach dem Erkalten gibt man 400 ml Ethylacetat zu, saugt über Celite ab und engt ein. Der Rückstand wird an Kieselgel mit einem Gemisch aus Ethylacetat und n-Hexan chromatographiert. Es werden 5,03 g 10a) erhalten.
Fp.: 166-167 °C [a]_{D}²⁰ = +459,2° (CHCl₃, c = 0,505)
¹H-NMR (CDCl₃); δ = 1,22 ppm (s, 3H, H-18); 2,37 und 2,40 (2s, 6H, 16-CH₃ und H-21); 3,80 (s, 3H, 3-OCH₃); 5,92 (d, J = 2 Hz, 1H, H-15); 6,68 (d, J = 3 Hz, 1H, H-4); 6,75 (dd, J = 9, 3 Hz, 1H, H-2); 7,25 (d, J = 9 Hz, 1H, H-1)

### b) 3-Methoxy-17¹-methyl-14,17-etheno-19-norpregna-1,3,5(10)-trien-20-on

5,0 g der unter 10a) beschriebenen Verbindung werden nach der in Beispiel 1b) beschriebenen Methode umgesetzt. Man erhält 3,38 g 10b) als Schaum.
¹H-NMR (CDCl₃); d = 0,85 ppm (s, 3H, H-18); 1,74 (s breit, 17¹-CH₃); 2,20 (s, 3H, H-21); 3,79 (s, 3H, 3-OCH₃); 5,67 (s breit, H-17²); 6,66 (d, J = 3 Hz, 1H, H-4); 6,73 (dd, J = 9, 3 Hz, 1H, H-2); 7,22 (d, J = 9 Hz, 1H, H-1)

### c) 17¹-Methyl-14,17-etheno-19-norpregn-4-en-3,20-dion

500 mg der unter 10b) beschriebenen Verbindung werden nach den in den Beispielen 1c), 1d) und 1e) beschriebenen Methoden umgesetzt. Man erhält 344 mg 10c).
Fp.: 137 °C [a]_{D}²⁰ = +109,6° (CHCl₃, c = 0,500)
¹H-NMR (CDCl₃): d = 0,86 ppm (s, 3H, H-18); 1,70 (s breit, 17¹-CH₃); 2,16 (s, 3H, H-21); 5,58 (s breit, 1H, H-17²); 5,84 (s breit, 1H, H-4)

### Beispiel 11

### 17¹-Methyl-14,17-etheno-19-norpregna-4,6-dien-3,20-dion

250 mg der unter 10c) beschriebenen Verbindung werden nach den in den Beispielen 2a) und 2b) beschriebenen Methoden umgesetzt. Man erhält 102 mg 11).
Fp.: 132-136 °C
¹H-NMR (CDCl₃): d = 0,89 ppm (s, 3H, H-18); 1,69 (s breit, 17¹-CH₃); 2,17 (s, 3H, H-21); 5,47 (s breit, 1H, H-17²) 5,80 (s breit, 1H, H-4); 6,17-6,30 (m, 2H, H-6 und H-7)

### Beispiel 12

### (17¹R)-17¹-Methyl-14,17-ethano-19-norpregn-4-en-3,20-dion

### a) 3-Methoxy-17¹-methyl-14,17-ethano-19-norpregna-1,3,5(10)-trien-20-on

2,75 g der unter 10b) beschriebenen Verbindung werden in einer Schüttelente in 125 ml Tetrahydrofuran gelöst. Man setzt 765 mg Palladium auf Aktivkohle (10%) zu, setzt die Apparatur unter Wasserstoff und schüttelt bis zum Ende der Wasserstoffaufnahme. Nach Filtration der Lösung über Celite wird im Vakuum eingeengt. Es werden 2,9 g 12a) als Schaum erhalten.
¹H-NMR (CDCl₃): d = 0,88 (0,92) ppm (s, 3H, H-18); 0,99 (1,10) (d, J = 7,5 Hz, 3H, 17¹-CH₃); 2.08 (2,11) (s, 3H, H-21); 3,78 (s, 3H, 3-OCH₃); 6,62 (d, J = 3 Hz, 1H, H-4); 6,73 (dd, J = 9, 3 Hz, 1H, H-2); 7,22 (d, J = 9 Hz, 1H, H-1)
(Signale des 2. Diastereomeren in Klammern)

### b) (17¹R)-17¹-Methyl-14,17-ethano-19-norpregn-5(10)-en-3,20-dion

2,9 g der unter 12a) beschriebenen Verbindung werden nach den in den Beispielen 1c), 1d) und 8c) beschriebenen Methoden umgesetzt. Man erhält 209 mg 12b), 310 mg der beiden C-17¹-Epimere von 20,20-[1,2-Ethandiylbis(oxy)]-17¹-methyl-14,17-ethano-19-norpregn-4-en-3-on im Gemisch mit (17¹S)-17¹-Methyl-14,17-ethano-19-norpregn-5(10)-en-3,20-dion sowie 1,36 g der beiden C-17¹-Epimere von 20,20-[1,2-Ethandiylbis(oxy)]-17¹-methyl-14,17-ethano-19-norpregn-5(10)-en-3-on.
¹H-NMR (CDCl₃): d = 0,90 ppm (s, 3H, H-18); 1,07 (d, J = 7,5 Hz, 3H, 17¹-CH₃); 2,06 (s, 3H, H-21)

### c) (17¹R)-17¹-Methyl-14,17-ethano-19-norpregn-4-en-3,20-dion

190 mg 12b) werden nach der in Beispiel 1e) beschriebenen Methode umgesetzt. Man erhält 105 mg 12c).
¹H-NMR (CDCl₃); d = 0,95 ppm (s, 3H, H-18); 1,05 (d, J = 7,5 Hz, 3H, 17¹-CH₃); 2,07 (s, 3H, H-21); 5,81 (s breit, 1H, H-4)

### Beispiel 13

### (17¹S)-17¹-Methyl-14,17-ethano-19-norpregn-4-en-3,20-dion

300 mg des unter 12b) beschriebenen Gemisches aus beiden C-17¹-Epimeren von 20,20-[1,2-Ethandylbis(oxy)]-17¹-methyl-14,17-ethano-19-norpregn-4-en-3-on und (17¹S)-17¹-Methyl-14,17-ethano-19-norpregn-5(10)-en-3,20-dion werden nach der in Beispiel 1e) beschriebenen Methode umgesetzt. Man erhält 77 mg 12c) und 122 mg 13).
¹H-NMR (CDCl₃): d = 0,68 ppm (dd, J = 6 Hz und 13 Hz, 1H, H-17²); 0,92 (s, 3H, H-18); 0,96 (d, J = 7,5 Hz, 3H, 17¹-CH₃); 2,08 (s, 3H, H-21); 5,82 (s breit, 1H, H-4)

### Beispiele 14 und 15

### 14: (17¹R)-17¹-Methyl-14,17-ethano-19-norpregna-4,9-dien-3,20-dion

### 15: (17¹S)-17¹-Methyl-14,17-ethano-19-norpregna-4,9-dien-3,20-dion

1,30 g des unter 12b) beschriebenen Gemisches aus beiden C-17¹-Epimeren von 20,20-[1,2-Ethandiylbis(oxy)]-17¹-methyl-14,17-ethano-19-norpregn-5(10)-en-3-on werden nach den in den Beispielen 4b) und le) beschriebenen Methoden umgesetzt. Man erhält 200 mg 14) und 120 mg 15).
**14):** ¹H-NMR (CDCl₃); d = 1,04 ppm (s, 3H, H-18); 1,06 (d, J = 7,5 Hz, 3H, 17¹-CH₃); 2,07 (s, 3H, H-21); 5,65 (s breit, 1H, H-4)
**15):** ¹H-NMR (CDCl₃): d = 0,76 ppm (dd, J = 5 Hz und 12 Hz, 1H, H-17²); 0,95 (d, J = 7,5 Hz, 3H, 17¹-CH₃); 1,01 (s, 3H, H-18); 2,09 (s, 3H, H-21); 5,66 (s breit, 1H, H-4)

### Beispiel 16

### 14,17-Ethano-19-norpregna-4,9-dien-3,20-dion

### a) 3-Methoxy-16α-phenylsulfonyl-14,17-etheno-19-norpregna-1,3,5(10)-trien-20-on

Eine Mischung aus 14,7 g der unter 1a) beschriebenen Substanz und 24,0 g Phenylvinylsulfon wird in 100 ml Benzol 10 Tage auf 155 °C erhitzt. Nach dem Erkalten wird das Reaktionsgemisch eingeengt und der Rückstand an Kieselgel zunächst mit Dichlormethan und dann mit einem Gemisch aus Ethylacetat und Hexan chromatographiert. Es werden 14,9 g 16a) erhalten.
Fp.: 178-179 °C
¹H-NMR (CDCl₃); δ = 0,84 ppm (s, 3H, H-18); 2,30 (s, 3H, H-21); 3,77 (s, 3H, 3-OCH₃); 4,58 (dd, J = 8, 4 Hz, 1H, H-16); 6,48 und 6,50 (2d, J = 5 Hz, je 1H, H-17¹ und H-17²); 6,64 (d, J = 3 Hz, 1H, H-4); 6,72 (dd, J = 9, 3 Hz, 1H, H-2); 7,18 (d, J = 9 Hz, 1H, H-1); 7,52-7,87 (m, 5H, SO₂C₆H₅)

### b) 3-Methoxy-14,17-ethano-19-norpregna-1,3,5(10)-trien-20-ol

120 g wasserfeuchtes Raney-Nickel werden mehrfach mit Ethanol gewaschen und schließlich in 900 ml Ethanol suspendiert. Zu dieser Suspension werden 6,95 g der unter 16a) beschriebenen Substanz zugegeben und für 16 Stunden am Rückfluß erhitzt. Nach dem Erkalten wird vom Raney-Nickel abdekantiert, mehrmals mit Ethanol nachgewaschen und eingeengt. Der Rückstand wird an Kieselgel mit einem Gemisch aus Ethylacetat und Cyclohexan chromatographiert. Es werden 1,40 g 3-Methoxy-14,17-ethano-19-norpregna-1,3,5(10)-trien-20-on mit einem Fp. von 140-142 °C und 2,70 g 16b) erhalten.
Fp.: 90-100 °C
¹H-NMR (CDCl₃): δ = 0,88 und 0,92 ppm (2s, 3H, H-18); 1,12 und 1,18 (2d, J = 6 Hz, 3H, H-21); 3,78 (s, 3H, 3-OCH₃); 3,95 (q, J = 6 Hz, 1H, H-20); 6,63 (d, J = 3 Hz, 1H, H-4); 6,72 (dd, J = 9, 3 Hz, 1H, H-2); 7,21 (d, J = 9 Hz, 1H, H-1)

### c) 3-Methoxy-14,17-ethano-19-norpregna-2,5(10)-dien-20-ol

5,50 g der unter 16b) beschriebenen Verbindung werden nach der in Beispielen 1d) beschriebenen Methode umgesetzt. Man erhält 5,50 g 16c).
¹H-NMR (CDCl₃): δ = 0,87 und 0,90 ppm (2s, 3H, H-18); 1,10 und 1,16 (2d, J = 6 Hz, 3H, H-21); 3,55 (s, 3H, 3-OCH₃); 3,87-3,98 (m, 1H, H-20); 4,65 (m, 1H, H-2)

### d) 14,17-Ethano-19-norpregn-5(10)-en-20-ol-3-on

1,70 g der unter 16c) beschriebenen Verbindung werden nach der in Beispiel 4a) beschriebenen Methode umgesetzt. Man erhält 0,80 g 16d).
Fp.: 103-117 °C
¹H-NMR (CDCl₃): δ = 0,87 und 0,90 ppm (2s, 3H, H-18); 1,11 und 1,16 (2d, J = 6 Hz, 3H, H-21); 2,69 und 2,78 (2d, J = 20 Hz, je 1H, H-4); 3,85-3,98 (m, 1H, H-20)

### e) 14,17-Ethano-19-norpregna-4,9-dien-20-ol-3-on

Zu einer Lösung von 0,16 ml Brom in 10 ml Pyridin wird unter Eiskühlung und Rühren eine Lösung von 0,80 g der unter 16d) beschriebenen Verbindung in 10 ml Pyridin zugetropft. Nach 3 Stunden wird das Reaktionsgemisch in 2-normale Salzsäure gegossen und auf einen pH-Wert zwischen 4 und 5 eingestellt. Es wird mit Ethylacetat extrahiert. Die organische Phase wird mit Wasser und konzentrierter Natriumchloridlösung gewaschen, über Natriumsulfat getrocknet, abfiltriert und eingeengt. Der Rückstand wird an Kieselgel mit einem Gemisch aus Ethylacetat und Hexan chromatographiert. Es werden 0,22 g 16e) erhalten.
¹H-NMR (CDCl₃): δ = 1,01 und 1,07 ppm (2s, 3H, H-18); 1,12 und 1,15 (2d, J = 6 Hz, 3H, H-21); 3,88-4,00 (m, 1H, H-20); 5,67 (s breit, 1H, H-4)

### f) 14,17-Ethano-19-norpregna-4,9-dien-3,20-dion

Zu einer Lösung von 220 mg der unter 16e) beschriebenen Verbindung in 20 ml Dichlormethan werden unter Eiskühlung 360 mg Pyridiniumchlorochromat zugegeben. Die Mischung wird 2 Stunden bei Raumtemperatur gerührt und dann filtriert. Das Filtrat wird eingeengt und an Kieselgel mit einem Gemisch aus Ethylacetat und Hexan chromatographiert. Es werden 130 mg 16f) als Schaum erhalten.
[a]_{D}²⁰ = -255,3° (CHCl₃, c = 0,600)
1H-NMR (CDCl₃): d = 1,04 ppm (s, 3H, H-18); 2,12 (s, 3H, H-21); 5,67 (s breit, 3H, H-4)

### Beispiel 17

### 14,17-Ethano-19-norpregna-4,6-dien-3,20-dion

### a) 14,17-Ethano-19-norpregn-4-en-3,20-dion

5,50 g der unter 16c) beschriebenen Verbindung werden nach den in den Beispielen le) und 16f) angegebenen Methoden umgesetzt. Man erhält 2,80 g 17a).
Fp.: 140-145 °C [a]_{D}²⁰ = +67,6° (CHCl₃; c = 0,550)
1H-NMR (CDCl₃): d = 0,94 ppm (s, 3H, H-18); 2,10 (s, 3H, H-21); 5,83 (s breit, 1H, H-4)

### b) 14,17-Ethano-19-norpregna-4,6-dien-3,20-dion

326 mg der unter 17a) beschriebenen Verbindung werden nach den in den Beispielen 2a) und 2b) angegebenen Methoden umgesetzt. Man erhält 160 mg 17c).
Fp.: 126-132 °C [a]_{D}²⁰ = + 31,8° (CHCl₃; c = 0,575)
¹H-NMR (CDCl₃): d = 0,96 ppm (s, 3H, H-18); 2,11 (s, 3H, H-21); 5,78 (s breit, 1H, H-4); 6,14-6,23 (m, 2H, H-6 und H-7)

### Beispiel 18

### 21-Methyl-14,17-ethano-19-norpregn-4-en-3,20-dion

### a) 3-Methoxy-21-methyl-14,17-ethano-19-norpregna-1,3,5(10)-trien-20-on

133 g der unter 1b) beschriebenen Verbindung werden in einer Schüttelente in 2 l Ethylacetat gelöst. Man setzt 13 g Palladium auf Aktivkohle (10%) zu, setzt die Apparatur unter Wasserstoff und schüttelt bis zum Ende der Wasserstoffaufnahme. Nach Filtration der Lösung über Celite wird eingeengt. Nach Kristallisation aus Ethylacetat erhält man 129 g 18a).
Fp.: 146-147 °C [a]_{D}²⁰ = + 66,7° (CHCl₃; c = 0,490)
¹H-NMR (CDCl₃): d = 0,90 ppm (s, 3H, H-18); 2,23 (s, 3H, H-21); 3,78 (s, 3H, 3-OCH₃); 6,63 (d, J = 3 Hz, 1H, H-4); 6,73 (dd, J = 9, 3 Hz, 1H, H-2); 7,22 (d, J = 9 Hz, 1H, H-1)

### b) 3-Methoxy-21-methyl-14,17-ethano-19-norpregna-1,3,5(10)-trien-20-on

5,00 g der unter 18a) beschriebenen Verbindung werden nach der in Beispiel 7a) angegebenen Methode umgesetzt. Man erhält 4,4 g 18b) als Schaum.
[a]_{D}²⁰ = + 71,3° (CHCl₃; c = 0,545)
¹H-NMR (CDCl₃): d = 0,89 ppm (s, 3H, H-18); 1,03 (t, J = 7 Hz, 3H, H-22); 2,40-2,50 (m, 2H, H-21); 3,78 (s, 3H, 3-OCH₃); 6,63 (d, J = 3 Hz, 1H, H-4); 6,72 (dd, J = 9, 3 Hz, 1H, H-2); 7,22 (d, J = 9 Hz, 1H, H-1)

### c) 3-Methoxy-21-methyl-14,17-ethano-19-norpregna-2,5(10)-dien-20-ol

2,70 g der unter 18b) beschriebenen Verbindung werden nach der in Beispiel 1d) angegebenen Methode umgesetzt. Es werden 1,75 g 18c) erhalten.
Fp.: 137-143 °C
¹H-NMR (CDCl₃): d = 0,86 ppm (s, 3H, H-18); 0,98 (t, J = 7 Hz, 3H, H-22); 3,55 (s, 3H, 3-OCH₃); 4,66 (s breit, 1H, H-2)

### d) 21-Methyl-14,17-ethano-19-norpregn-4-en-20-ol-3-on

356 mg der unter 18c) beschriebenen Verbindung werden nach der in Beispiel le) angegebenen Methode umgesetzt. Es werden 300 mg 18d) erhalten.
¹H-NMR (CDCl₃); d = 0,91 ppm (s, 3H, H-18); 0,98 (t, J = 7 Hz, 3H, H-22); 3,55-3,63 (m, 1H, H-20); 5,81 (s breit, 1H, H-4)

### e) 21-Methyl-14,17-ethano-19-norpregn-4-en-3,20-dion

300 mg der unter 18d) beschriebenen Verbindung werden nach der in Beispiel 14f) angegebenen Methode umgesetzt. Es werden 200 mg 18e) erhalten.
Fp.: 123-128 °C [a]_{D}²⁰ = +63,8° (CHCl₃; c = 0,525)
¹H-NMR (CDCl₃); d = 0,93 ppm (s, 3H, H-18); 1,00 (t, J = 7 Hz, 3H, H-22); 5,82 (s breit, 1H, H-4)

### Beispiel 19

### 21-Methyl-14,17-ethano-19-norpregna-4,9-dien-3,20-dion

770 mg der unter 18c) beschriebenen Verbindung werden nach den in den Beispielen 4a), 4b) und 16f) angegebenen Methoden umgesetzt. Es werden 170 mg 19) erhalten.
Fp.: 130 °C [a]_{D}²⁰ = -251,2° (CHCl₃; c = 0,470)
¹H-NMR (CDCl₃): d = 1,02 ppm (t, J = 7 Hz, 3H, H-22); 1,05 (s, 3H, H-18); 5,68 (s breit, 1H, H-4)

### Beispiel 20

### 21-Methyl-14,17-ethano-19-norpregna-4,6-dien-3, 20-dion

370 mg der unter 18d) beschriebenen Verbindung werden nach den in den Beispielen 2a), 2b) und 16f) angegebenen Methoden umgesetzt. Es werden 120 mg 20) erhalten.
Fp.: 155-158 °C [a]_{D}²⁰ = +20,0° (CHCl₃; c = 0,490)
¹H-NMR (CDCl₃):d = 0,96 ppm (s, 3H, H-18); 1,02 (t, J = 7 Hz, 3H, H-22); 5,78 (s breit, 1H, H-4); 6,15-6.23 (m, 2H, H-6 und H-7)

### Beispiel 21

### 21,21-Dimethyl-14,17-ethano-19-norpregna-4,9-dien-3,20-dion

### a) 3,3-[2,2-Dimethyl-1,3-propandiylbis(oxy)]-21-methyl-14,17-ethano-19-norpregna-5(10),9(11)-dien-20-on

18,3 g der unter 18c) beschriebenen Verbindung werden nach den in den Beispielen 4a), 4b), 9a) und 7c) angegebenen Methoden umgesetzt. Es werden 1,0 g 21a) erhalten.
¹H-NMR (CDCl₃): d = 0,85 und 0,90 ppm (2s, 6H, Ketal-CH₃); 1,03 (t, J = 7 Hz, 3H, H-22); 1,09 (s, 3H, H-18); 3,42-3,52 (m, 2H, Ketal-OCH₂); 3,57-3,68 (m, 2H, Ketal-OCH₂); 5,50-5,55 (m, 1H, H-11);

### b) 21,21-Dimethyl-14,17-ethano-19-norpregna-4,9-dien-3,20-dion

210 mg der unter 21a) beschriebenen Verbindung werden nach den in den Beispielen 7a) und 1e) angegebenen Methoden umgesetzt. Es werden 108 mg 21b) erhalten.
¹H-NMR (CDCl₃): d = 1,01 ppm (d, J = 6 Hz, 6H, H-22, H-22'); 1,05 (s, 3H, H-18); 5,68 (s breit, 1H, H-4)

### Beispiel 22

### 6-Methyl-14,17-ethano-19-norpregna-4,6-dien-3,20-dion

### a) 6-Methylen-14,17-ethano-19-norpregn-4-en-3,20-dion

6,10 g der unter 17a) beschriebenen Verbindung werden nach der im Beispiel 2a) beschriebenen Methode zum entsprechenden Dienolether umgesetzt, welcher als Rohprodukt in 60 ml Dimethylformamid aufgenommen wird und bei 0 °C mit einer Lösung von 5,2 ml Phosphoroxichlorid in 30 ml Dimethylformamid versetzt wird. Nach einer Stunde wird das Reaktionsgemisch in konzentrierte Natriumhydrogencarbonatlösung getropft und mit Ethylacetat extrahiert. Die organische Phase wird mit Wasser und konzentrierter Natriumchloridlösung gewaschen, über Natriumsulfat getrocknet, abfiltriert und eingeengt. Es werden 5,27 g der 6-Formylverbindung als Rohprodukt erhalten, welche in 14 ml Ethanol und 28 ml Dimethylformamid gelöst werden und portionsweise mit 0,66 g Natriumborhydrid versetzt werden. Nach einer Stunde werden 7,5 ml zweinormale Schwefelsäure zugetropft. Nach 15 Minuten wird das Reaktionsgemisch mit 120 ml Wasser verdünnt, mit konzentrierter Natriumhydrogencarbonatlösung neutralisiert und mit Ethylacetat extrahiert. Die organische Phase wird mit Wasser und konzentrierter Natriumchloridlösung gewaschen, über Natriumsulfat getrocknet, abfiltriert und eingeengt. Es werden 4,31 g 22a) als Rohprodukt erhalten.
¹H-NMR (CDCl₃): d = 0,93 ppm (s, 3H, H-18); 2,12 (s, 3H, H-21); 4,94 und 5,18 (2s breit, je 1H, 6-=CH₂), 6,11 (s breit, 1H, H-4)

### b) 6-Methyl-14,17-ethano-19-norpregna-4,6-dien-3,20-dion

1,05 g Palladium auf Kohle (5%) werden in 50 ml Methanol 30 Minuten am Rückfluß erhitzt und anschließend mit einer Lösung von 2,15 g der unter 22a) beschriebenen Verbindung in 90 ml Methanol versetzt und 90 Minuten am Rückfluß erhitzt. Der Katalysator wird abfiltiert und nach dem Einengen wird der Rückstand an Kieselgel mit einem Gemisch aus Ethylacetat und Hexan chromatographiert. Es werden 88 mg 22b) erhalten.
¹H-NMR (CDCl₃): d = 0,95 ppm (s, 3H, H-18); 1,83 (s breit, 1H, 6-CH₃) 2,12 (s, 3H, H-21); 5,93 und 5,99 (2s breit, 2H, H-4 und H-7)

### Beispiel 23

### 6α-Methyl-14,17-ethano-19-norpregn-4-en-3,20-dion

Eine Lösung von 2,15 g der unter 22a) beschriebenen Verbindung in 30 ml Ethanol wird mit 3ml Cyclohexan und 0,25 g Palladium auf Kohle (10%) versetzt und 75 Minuten am Rückfluß erhitzt. Der Katalysator wird abfiltiert und nach dem Einengen wird der Rückstand an Kieselgel mit einem Gemisch aus Ethylacetat und Hexan chromatographiert. Es werden 140 mg 23) erhalten.
¹H-NMR (CDCl₃): d = 0,95 ppm (s, 3H, H-18); 1,12 (d, J = 7Hz, 3H, 6-CH₃) 2,12 (s, 3H, H-21); 5,86 (s breit, 1H, H-4)

### Beispiel 24

### 21-Hydroxy-14,17-ethano-19-norpregn-4-en-3,20-dion

4,9 g der unter 17a) beschriebenen Verbindung werden nach den in den Beispielen 9a), 5c), 5d), 1e) und 5g) angegebenen Methoden umgesetzt. Es werden 253 mg 24) erhalten.
[a]_{D}²⁰ = +65,9° (CHCl₃; c = 0,525)
¹H-NMR (CDCl₃): d = 0,95 ppm (s, 3H, H-18); 3,35 (t, J = 5 Hz, 1H, 21-OH), 4,24 und 4,27 (2d, J = 5 Hz, je 1H, H-21); 5,72 (s breit, 1H, H-4)

### Beispiel 25

### 21-Hydroxy-14,17-ethano-19-norpregna-4,9-dien-3,20-dion

805 mg der unter 16f) beschriebenen Verbindung werden nach den in den Beispielen 9a), 5c), 5d), 1e) und 5g) angegebenen Methoden umgesetzt. Es werden 110 mg 25) erhalten.
[a]_{D}²⁰ = -232,8° (CHCl₃; c = 0,500)
¹H-NMR (CDCl₃): d = 1,04 ppm (s, 3H, H-18); 3,32 (t, J = 5 Hz, 1H, 21-OH), 4,23 und 4,27 (2d, J = 5 Hz, je 1H, H-21); 5,68 (s breit, 1H, H-4)

### Beispiele 26 und 27

### 26: (21R)-21-Hydroxy-21-methyl-14,17-ethano-19-norpregna-4,9-dien-3,20-dion

### 27: (21S)-21-Hydroxy-21-methvl-14,17-ethano-19-norpregna-4,9-dien-3,20-dion

2,00 g der unter 21a) beschriebenen Verbindung werden nach den in den Beispielen 5c), 5d), 1e) und 5g) angegebenen Methoden umgesetzt. Es werden 640 mg des 21-Epimerengemischs erhalten, welches durch Chromatographie an Kieselgel mit einem Gemisch aus Ethylacetat und Hexan in 210 mg 26) und 230 mg 27) getrennt wird.
**26:** [a]_{D}20 = -1,6° (CHCl₃; c = 0,495)
   ¹H-NMR (CDCl₃): d = 1,03 ppm (s, 3H, H-18); 1,32 (d, J = 6 Hz, 3H, H-22), 3,60 (d, J = 6 Hz, 1H, 21-OH), 4,37-4,46 (m, 1H, H-21); 5,68 (s breit, 1H, H-4)
**27:** [a]_{D}²⁰ = -1,0° (CHCl₃; c = 0,475)
   ¹H-NMR (CDCl₃): d = 1,07 ppm (s, 3H, H-18); 1,29 (d, J = 6 Hz, 3H, H-22), 3,40 (d, J = 7 Hz, 1H, 21-OH), 4,33-4,44 (m, 1H, H-21); 5,68 (s breit, 1H, H-4)

### Beispiel 28

### 16α-Methyl-14,17-ethano-19-norpregn-4-en-3,20-dion

### a) 3-Methoxy-20-oxo-14,17-etheno-19-norpregna-1,3,5(10)-trien-16α-carbonsäuremethylester

19,4 g der in Beispiel la) beschriebenen Verbindung, 37 ml frisch destillierter Acrylsäuremethylester und 200 mg Hydrochinon werden im geschlossenen Rohr 7 Tage bei 120°C belassen. Nach Abkühlung und Abdestillieren aller flüchtigen Bestandteile unter vermindertem Druck wird der Rückstand an Kieselgel mit einem Gemisch aus Ethylacetat und Hexan chromatographiert. Es werden 21,0 g 28a) erhalten.
Fp.: 145-146°C [a]_{D}²⁰ = +216,4° (CHCl₃; c = 0,505)
¹H-NMR (CDCl₃): d = 0,96 ppm (s, 3H, H-18); 2,29 (s, 3H, H-21); 3,60 (s, 3H, CO₂CH₃); 3,78 (s, 3H, 3-OCH₃); 3,84 (dd, J = 9,5 und 4,5Hz, H-16); 6,15 und 6,27 (2d, J = 6Hz, je 1H, H-17¹ und H-17²): 6,64 (d, J = 3Hz, 1H, H-4); 6,72 (dd, J = 9 und 3Hz, 1H, H-2); 7,19 (d, J = 9Hz, 1H, H-1)

### b) 20,20-[1,2-Ethandiylbis(oxy)]-3-methoxy-14,17-etheno-19-norpregna-1,3,5(10)-trien-16α-carbonsäuremethylester

20.8 g der unter 28a) beschriebenen Verbindung werden nach der in Beispiel 1c) angegebenen Methode umgesetzt. Es werden 17,0 g 28b) erhalten.
Fp.: 128-130°C [a]_{D}²⁰ = +141,2° (CHCl₃; c = 0,505)
¹H-NMR (CDCl₃); d = 1,03 ppm (s, 3H, H-18); 1,32 (s, 3H, H-21); 3,38 (dd, J = 9,5 und 4,5Hz, 1H, H-16); 3,60 (s, 3H, CO₂CH₃); 3,78 (s, 3H, 3-OCH₃); 3,82-4,18 (m, 4H, 20-OCH₂CH₂O-); 6,00 und 6,23 (2d, J = 6Hz, je 1H, H-17¹ und H-17²); 6,63 (d, J = 3Hz, 1H, H-4); 6,71 (dd, J = 9 und 3Hz, 1H, H-2); 7,20 (d, J = 9Hz, 1H, H-1)

### c) 20,20-[1,2-Ethandiylbis(oxy)]-3-methoxy-14,17-etheno-19-norpregna-1,3,5(10)-trien-16α-methanol

Eine Lösung von 8,2 g der unter 28b) beschriebenen Verbindung in 150 ml Tetrahydrofuran wird in eine auf 0°C gekühlte Suspension von 2,84 g Lithiumaluminiumhydrid in 100 ml Tetrahydrofuran getropft. Nach 2 Stunden Rühren bei Raumtemperatur wird langsam mit Sml Wasser versetzt. Nach weiteren 20 Minuten wird über Celite abfiltriert, mit Dichlormethan nachgewaschen, über Natriumsulfat getrocknet und eingeengt. Es werden 7,1 g 28c) erhalten. Für analytische Zwecke wird eine Probe aus Pentan kristallisiert.
Fp.: 162-164°C [a]_{D}²⁰ = +104,2° (CHCl₃; c = 0,520)
¹H-NMR (CDCl₃); d = 1,05 ppm (s, 3H, H-18); 1,48 (s, 3H, H-21); 3,18-3,44 (m, 2H, 16-CH₂OH); 3,77 (s, 3H, 3-OCH₃); 4,01-4,12 (m, 4H, 20-OCH₂CH₂O-); 5.95 und 6,04 (2d, J = 6Hz, je 1H, H-17¹ und H-17²); 6,62 (d, J = 3Hz, 1H, H-4); 6,72 (dd, J = 9 und 3Hz, 1H, H-2); 7,21 (d, J = 9Hz, 1H, H-1)

### d) 20,20-[1,2-Ethandiylbis(oxy)]-3-methoxy-14,17-ethano-19-norpregna-1,3,5(10)-trien-16α-methanol

7,8 g der unter 28c) beschriebenen Verbindung werden nach der in Beispiel 16a) angegebenen Methode umgesetzt. Es werden 7,4 g 28d) erhalten.
Fp.: 190-193°C [a]_{D}²⁰ = +5,5° (CHCl₃; c = 0,505)
¹H-NMR (CDCl₃): d = 1,05 ppm (s, 3H, H-18); 1,43 (s, 3H, H-21); 3,54 (m, 1H, 16-CH₂OH); 3,78 (s, 3H, 3-OCH₃); 3,69-4,10 (m, 5H, 20-OCH₂CH₂O- und 16-CH₂OH); 6,62 (d, J = 3Hz, 1H, H-4); 6,70 (dd, J = 9 und 3Hz, 1H, H-2); 7,21 (d, J = 9Hz, 1H, H-1)

### e) 16α-(Brommethyl)-20,20-[1,2-ethandiylbis(oxy)]-3-methoxy-14,17-ethano-19-norpregna-1,3,5(10)-trien

6,8 g der unter 28d) beschriebenen Verbindung, 7,2 g Tetrabrommethan und 5,7 g Triphenylphosphin werden in 250 ml Dichlormethan 16 Stunden bei Raumtemperatur gerührt. Nach Einengen wird an Kieselgel mit einem Gemisch aus Ethylacetat und Hexan chromatographiert. Es werden 2,2g 28e) erhalten.
Fp.: 176-177°C [a]_{D}²⁰ = -21,7° (CHCl₃; c = 0,505)
¹H-NMR (CDCl₃); d = 1.02 ppm (s, 3H, H-18); 1,30 (s, 3H, H-21); 3,34 (dd, J = 10 und 12Hz, 16-CH₂Br); 3,78 (s, 3H, 3-OCH₃); 3,82-4,06 (m, 5H, 20-OCH₂CH₂O-und 16-CH₂Br); 6,63 (d, J = 3Hz, 1H, H-4); 6,71 (dd, J = 9 und 3Hz, 1H, H-2); 7,20 (d, J = 9Hz, 1H, H-1)

### f) 20,20-[1,2-Ethandiylbis(oxy)]-3-methoxy-16α-methyl-14,17-ethano-19-norpregna-2,5(10)-dien

1,78 g der unter 28e) beschriebenen Verbindung werden nach der in Beispiel ld)angegebenen Methode umgesetzt. Es werden 1,1 g 28f) erhalten.
Fp.: 174-178°C [a]_{D}²⁰ = +41,4° (CHCl₃; c = 0,50)
¹H-NMR (CDCl₃): d = 0,99 ppm (s, 3H, H-18); 1,06 (d, J = 7Hz, 16-CH₃); 1,25 (s, 3H, H-21); 3,56 (s, 3H, 3-OCH₃); 3,78-4,01 (m, 4H, 20-OCH₂CH₂O-); 4,64 (m, 1H, H-2)

### g) 16α-Methyl-14,17-ethano-19-norpregn-4-en-3,20-dion

1,05 g der unter 28f) beschriebenen Verbindung werden nach der in Beispiel 1e) angegebenen Methode umgesetzt. Es werden 0,7 g 28g) erhalten.
Fp.: 172-173°C [a]_{D}²⁰ = +52,7° (CHCl₃; c = 0,485)
¹H-NMR (CDCl₃); d = 0,96 ppm (s, 3H, H-18); 0,96 (d, J = 7Hz, 16-CH₃); 2,09 (s, 3H, H-21); 5,81 (t, J = 1Hz, H-4))

### Beispiel 29

### 16α-Ethyl-14,17-ethano-19-norpregn-4-en-3,20-dion

### a) 20,20-[1,2-Ethandiylbis(oxy)]-3-methoxy-14,17-ethano-19-norpregna-1,3,5(10)-trien-16α-carbaldehyd

2,7 g der unter 28d) beschriebenen Verbindung werden nach der in Beispiel 7c) angegebenen Methode umgesetzt. Es werden 2,4 g 29a) erhalten.
¹H-NMR (CDCl₃): d = 1,02 ppm (s, 3H, H-18); 1,34 (s, 3H, H-21); 3,78 (s, 3H, 3-OCH₃); 3,82-4,16 (m, 4H, 20-OCH₂CH₂O-); 6,61 (d, J = 3Hz, 1H, H-4); 6,72 (dd, J = 9 und 3Hz, 1H, H-2); 7,20 (d, J = 9Hz, 1H, H-1); 9,88 (d, J = 2Hz, CHO)

### b) 20,20-[1,2-Ethandiylbis(oxy)]-16α-ethenyl-3-methoxy-14,17-ethano-19-norpregna-1,3,5(10)-trien

10,7 g Methyltriphenylphosphoniumbromid werden in 70 ml Tetrahydrofuran suspendiert und bei 0°C tropfenweise mit insgesamt 18 ml einer 1,6 molaren Lösung von n-Butyllithium in Hexan versetzt. Nach 20 Minuten Rühren bei 0°C und 1 Stunde Rühren bei Raumtemperatur werden 2,6 g der unter 29a) beschriebenen Verbindung in 40 ml Tetrahydrofuran zugetropft. Nach 2 Stunden wird von festen Bestandteilen abfiltriert und eingeengt. Der Rückstand wird zwischen Wasser und Ethylacetat verteilt, die organische Phase mit konzentrierter wäßriger Natriumchloridlösung gewaschen, über Natriumsulfat getrocknet, filtriert und eingeengt. Nach Chromatographie an Kieselgel mit einem Gemisch aus Ethylacetat und Hexan werden 1,6 g 29b) erhalten.
[a]_{D}²⁰ = +13,7° (CHCl₃; c = 0,510)
¹H-NMR (CDCl₃): d = 1,03 ppm (s, 3H, H-18); 1,38 (s, 3H, H-21); 3,78 (s, 3H, 3-OCH₃); 3,84-4,03 (m, 4H, 20-OCH₂CH₂O-); 4,96-5,08 (m, 2H, Vinyl-CH₂); 6,02-6,17 (m, 1H, Vinyl-CH); 6,62 (d, J = 3Hz, 1H, H-4); 6,71 (dd, J = 9 und 3Hz, 1H, H-2); 7,21 (d, J = 9Hz, 1H, H-1)

### c) 20,20-[1,2-Ethandiylbis(oxy)]-16α-ethyl-3-methoxy-14,17-ethano-19-norpregna-1,3,5(10)-trien

1,0 g der unter 29b) beschriebenen Verbindung werden nach der in Beispiel 16a) angegebenen Methode umgesetzt. Es werden 1,0 g 29c) erhalten.
¹H-NMR (CDCl₃); d = 0,91 ppm (t, J = 7Hz, 3H, 16-Ethyl-CH₃); 1,00 (s, 3H, H-18); 1,30 (s, 3H, H-21); 3,77 (s, 3H, 3-OCH₃); 3,70-4,02 (m, 4H, 20-OCH₂CH₂O-); 6,61 (d, J = 3Hz, 1H, H-4); 6,71 (dd, J = 9 und 3Hz, 1H, H-2); 7,21 (d, J = 9Hz, 1H, H-1)

### d) 20,20-[1,2-Ethandiylbis(oxy)]-16α-ethyl-3-methoxy-14,17-ethano-19-norpregna-2,5(10)-dien

1,0 g der unter 29c) beschriebenen Verbindung werden nach der in Beispiel 1d) angegebenen Methode umgesetzt. Es werden 1,07 g 29d) erhalten, welches als Rohprodukt weiter umgesetzt wird.
¹H-NMR (CDCl₃); d = 3,54 ppm (s, 3H, 3-OCH₃); 3,75-4,01 (m, 4H, 20-OCH₂CH₂O-); 4,63 (m, 1H, H-2)

### e) 16α-Ethyl-14,17-ethano-19-norpregn-4-en-3,20-dion

0,97 g der unter 29d) beschriebenen Verbindung werden nach der in Beispiel 1e) angegebenen Methode umgesetzt. Nach Chromatographie an Kieselgel mit einem Gemisch aus Ethylacetat und Hexan werden 0,56 g 29e) erhalten, welches aus Diisopropylether kristallisiert wird.
Fp.: 145-147°C [a]_{D}²⁰ = +42,9° (CHCl₃; c = 0,515)
¹H-NMR (CDCl₃): d = 0,96 ppm (t, J = 7Hz, 3H, 16-Ethyl-CH₃); 0,96 (s, 3H, H-18); 2,10 (s, 3H, H-21); 5,81 (t, J = 1Hz, 1H, H-4)

### Beispiel 30

### 16α-Ethenyl-14,17-ethano-19-norpregn-4-en-3,20-dion

### a) 20,20-[1,2-Ethandiylbis(oxy)]-16α-ethenyl-3-methoxy-14,17-ethano-19-norpregna-2,5(10)-dien

0,5 g der unter 29b) beschriebenen Verbindung werden nach der in Beispiel 1d) beschriebenen Methode umgesetzt. Es werden 0,5 g 30a) erhalten, welches ohne Reinigung weiterverarbeitet wird.
¹H-NMR (CDCl₃): d = 1,02 ppm (s, 3H, H-18); 1,23 (s, 3H, H-21); 3,56 (s, 3H, 3-OCH₃); 3,82-4,02 (m, 4H, 20-OCH₂CH₂O-); 4,64 (m, 1H, H-2); 4,93-5,07 (m, 2H, Vinyl-CH₂); 6,08 (m, 1H, Vinyl-CH)

### b) 16α-Ethenyl-14,17-ethano-19-norpregn-4-en-3,20-dion

0,49 g 30a) werden nach der in Beispiel le) beschriebenen Methode umgesetzt. Nach Chromatographie an Kieselgel mit einem Gemisch aus Ethylacetat und Hexan 0,27 g 30b) erhalten.
Fp.: 171°C [a]_{D}²⁰ = +26,7° (CHCl₃; c = 0,515)
¹H-NMR (CDCl₃); d = 1,00 ppm (s, 3H, H-18); 2,08 (s, 3H, H-21); 5,03-5,13 (m, 2H, Vinyl-CH₂); 5,72-5,87 (m, 2H, Vinyl-CH und H-4)

### Beispiel 31

### 16-Methylen-14,17-ethano-19-norpregn-4-en-3,20-dion

### a) 20,20-[1,2-Ethandiylbis(oxy)]-3-methoxy-16-methylen-14,17-ethano-19-norpregna-1,3,5(10)-trien

27,86 g der unter 29a) beschriebenen Verbindung werden in 268 ml Tetrahydrofuran gelöst und bei 0 °C mit 26,6 g Kaliumhexamethyldisilazid versetzt. Nach 1 Stunde werden 17,8 ml Nonaflylfluorid (1,1,2,2,3,3,4,4,4-Nonafluorbutansulfonylfluorid) zugetropft. Nach 3 Stunden rühren bei Raumtemperatur wird zwischen Wasser und Ethylacetat verteilt und die organische Phase mit konzentrierter Natriumhydrogencarbonatlösung und Kochsalzlösung gewaschen. Nach Trocknung der organischen Phase über Natriumsulfat wird filtriert, eingeengt und der Rückstand in 546 ml Dimethylformamid aufgenommen. Nach Zugabe von 111,5 ml Triethylamin, 2,0 g Bis-(triphenylphosphin)palladium(II)-chlorid und 19,5 ml Ameisensäure wird 7 Stunden auf 80 °C erwärmt und anschließend über Nacht bei Raumtemperatur belassen. Nach verteilen zwischen Ethylacetat und Wasser wird die organische Phase mit konzentrierter Kochsalzlösung gewaschen, über Natriumsulfat getrocknet, filtriert und eingeengt. Der Rückstand wird an Kieselgel mit einem Gemisch aus Ethylacetat und Hexan chromatographiert. Es werden 5,33 g 31a) erhalten.
[a]_{D}²⁰ = +21,1° (CHCl₃; c = 0,530)
¹H-NMR (CDCl₃); d = 0,99 ppm (s, 3H, H-18)); 1,44 (s, 3H, H-21); 3,78 (s, 3H, 3-OCH₃); 3,87-4,07 (m, 4H, 20-OCH₂CH₂O-); 4,90 ppm (s breit, 1H, 16-Methylen); 5,15 (s breit, 1H, 16-Methylen), 6,63 (d, J = 3Hz, 1H, H-4); 6,72 (dd, J = 9 und 3Hz, 1H, H-2); 7,23 (d, J = 9Hz, 1H, H-1)

### b) 16-Methylen-14,17-ethano-19-norpregn-4-en-3,20-dion

510 mg der unter 31a) beschriebenen Verbindung werden nach den in den Beispielen 1d) und 1e) beschriebenen Methoden umgesetzt. Es werden 440 mg 31b) erhalten, die mit Diisopropylether digeriert werden.
¹H-NMR (CDCl₃); d = 1,09 ppm (s, 3H, H-18); 2,22 (s, 3H, H-21); 4,83 (s breit, 1H, 16-Methylen); 4,90 (s breit, 1H, 16-Methylen); 5,82 (s breit, 1H, H-4)

### Beispiel 32

### 16-Methylen-14,17-ethano-19-norpregna-4,9-dien-3,20-dion

### a) 20,20-[1,2-Ethandiylbis(oxy)]-16-methylen-14,17-ethano-19-norpregn-5(10)-en-3-on

6,8 g der unter 31a) beschriebenen Verbindung werden nach den in den Beispielen 1d) und 8c) beschriebenen Methoden umgesetzt. Nach Chromatographie an Kieselgel mit einem Gemisch aus Ethylacetat und Hexan werden 4,9 g 32a) erhalten. Außerdem werden 600 mg 20,20-[1,2-Ethandiylbis(oxy)]-16-methylen-14,17-ethano-19-norpregn-4-en-3-on erhalten.
¹H-NMR (CDCl₃) für Verbindung 32a): d = 0,98 ppm (s, 3H, H-18); 1,41 (s, 3H, H-21); 2,68 und 2,78 (2d breit, J = 20 Hz, je 1H, H-4); 3,84-4,05 (m, 4H, 20-OCH₂CH₂O-); 4,89 (s breit, 1H, 16-Methylen); 5,12 (s breit, 1H, 16-Methylen)
¹H-NMR (CDCl₃) für 20,20-[1,2-Ethandiylbis(oxy)]-16-methylen-14,17-ethano-19-norpregn-4-en-3-on: d = 1,00 ppm (s, 3H, H-18); 1,42 (s, 3H, H-21); 3,84-4,04 (m, 4H, 20-OCH₂CH₂O-); 4,88 (s breit, 1H, 16-Methylen); 5,13 (s breit, 1H, 16-Methylen); 5,82 (s breit, 1H, H-4)

### b) 16-Methylen-14,17-ethano-19-norpregna-4,9-dien-3,20-dion

4,9 g der in Beispiel 32a) beschriebenen Verbindung werden nach den in den Beispielen 4b) und 1e) beschriebenen Methoden umgesetzt. Nach Chromatographie an Kieselgel mit einem Gemisch aus Ethylacetat und Hexan und digerieren des Produktes mir Diisopropylether werden 1,52 g 32b) erhalten.
Fp.: 141°C (Zersetzung) [a]_{D}²⁰ = -358,8° (CHCl₃; c = 0,515)
¹H-NMR (CDCl₃): d = 1.19 ppm (s, 3H, H-18); 2,22 (s, 3H, H-21); 4,84 (s breit, 1H, 16-Methylen); 4,90 (s breit, 1H, 16-Methylen); 5,68 (s breit, 1H, H-4)

### Beispiel 33

### 16-Methylen-14,17-ethano-19-norpregna-4,6-dien-3,20-dion

600 mg 20,20-[1,2-Ethandiylbis(oxy)]-16-methylen-14,17-ethano-19-norpregn-4-en-3-on aus Beispiel 32a) werden nach den in den Beispielen 2a), 2b) und 1e) beschriebenen Methoden umgesetzt. Nach Chromatographie an Kieselgel mit einem Gemisch aus Ethylacetat und Hexan und Kristallisation des Produktes aus Diisopropylether werden 70 mg 33) erhalten.
¹H-NMR (CDCl₃); d = 1,10 ppm (s, 3H, H-18); 2,21 (s, 3H, H-21); 4,86 (s breit, 1H, 16-Methylen); 4,92 (s breit, 1H, 16-Methylen); 5,78 (s breit, 1H, H-4); 6,10-6,26 (m, 2H, H-6 und H-7)

### Beispiel 34

### 16α-Methyl-14,17-ethano-19-norpregna-4,6-dien-3,20-dion

### a) 20,20-[1,2-Ethandiylbis(oxy)]-3-methoxy-16α-[[(methylsulfonyl)oxy]methyl]-14,17-ethano-19-norpregna-1,3,5(10)-trien

11,7 g der unter 28b) beschriebenen Verbindung, gelöst in einem Gemisch aus 15 ml Pyridin und 110 ml Dichlormethan, werden bei 0 °C langsam mit 4,7 ml Methansulfonsäurechlorid versetzt. Nach 24 Stunden bei Raumtemperatur wird mit konzentrierter, eiskalter Natriumhydrogencarbonatlösung versetzt. Die organische Phase wird dreimal mit konzentrierter, eiskalter Natriumhydrogencarbonatlösung gewaschen, über Natriumsulfat getrocknet und filtriert. Unter vermindertem Druck werden alle flüchtigen Bestandteile entfernt. Es werden 14,3 g 34a) erhalten, die ohne Reinigung weiter umgesetzt werden.
¹H-NMR (CDCl₃): d = 1,11 ppm (s, 3H, H-18); 1,31 (s, 3H, H-21); 3,02 (s, 3H, SO₂CH₃); 3,68 (s, 3H, 3-OCH₃); 3,82-4,06 (m, 5H, 20-OCH₂CH₂O-); 4,12 (dd, J = 9 und 10 Hz, 1H, 16-CH₂); 4,80 (dd, J = 4 und 10 Hz, 1H, 16-CH₂); 6,62 (d, J = 3Hz, 1H, H-4); 6,70 (dd, J = 9 und 3Hz, 1H, H-2); 7,20 (d, J = 9Hz, 1H, H-1)

### b) 20,20-[1,2-Ethandiylbis(oxy)]-3-methoxy-16α-methyl-14,17-ethano-19-norpregna-1,3,5(10)-trien

14,3 g der unter 34a) beschriebenen Verbindung werden in 20 ml Tetrahydrofuran suspendiert und mit 150 ml einer 1 molaren Lösung von Lithiumtriethylborhydrid in Tetrahydrofuran versetzt. Nach 5,5 Stunden Erhitzen unter Argon wird 15 Stunden bei Raumtemperatur belassen und zwischen Ethylacetat und konzentrierter Ammoniumchloridlösung verteilt. Die organische Phase wird mit konzentrierter Natriumhydrogencarbonatlösung gewaschen, über Natriumsulfat getrocknet, filtriert und eingeengt. Nach Chromatographie an Kieselgel mit einem Gemisch aus Ethylacetat und Hexan werden 6,0 g 34b) erhalten.
Fp.: 132-134°C
¹H-NMR (CDCl₃): d = 1,01 ppm (s, 3H, H-18); 1,19 (d, J = 7,5 Hz, 16-CH₃); 1,30 (s, 3H, H-21); 3,78 (s, 3H, 3-OCH₃); 3,83-4,04 (m, 5H, 20-OCH₂CH₂O-); 6,62 (d, J = 3Hz, 1H, H-4); 6,71 (dd, J = 9 und 3Hz, 1H, H-2); 7,22 (d, J = 9Hz, 1H, H-1)

### c) 3-Methoxy-16α-methyl-14,17-ethano-19-norpregna-1,3,5(10)-trien-20-on

5,96 g der unter 34b) beschriebenen Verbindung werden nach der in Beispiel 1e) beschriebenen Methode umgesetzt. Es werden 5,58 g 34c) als Rohprodukt erhalten.
Fp.: 115-116°C [a]_{D}²⁰ = +51,3° (CHCl₃; c = 0,530)
¹H-NMR (CDCl₃): d = 0,93 ppm (s, 3H, H-18); 0,99 (d, J = 7,5 Hz, 16-CH₃); 2,11 (s, 3H, H-21); 3,78 (s, 3H, 3-OCH₃); 6,62 (d, J = 3Hz, 1H, H-4); 6,71 (dd, J = 9 und 3Hz, 1H, H-2); 7,21 (d, J = 9Hz, 1H, H-1)

### d) 3-Methoxy-16α-methyl-14,17-ethano-19-norpregna-1,3,5(10)-trien-20ξ-ol

5,53 g der unter 34c) beschriebenen Verbindung werden in einem Gemisch aus 90 ml Methanol und 130 ml Dichlormethan gelöst und portionsweise mit 2,37 g Natriumborhydrid versetzt. Nach 2 Stunden bei Raumtemperatur wird mit Wasser versetzt, mit 2-normaler Salzsäure angesäuert und die Wasserphase zweimal mit Dichlormethan extrahiert. Nach Waschen der organischen Phase mit Wasser, konzentrierter Natriumhydrogencarbonatlösung und konzentrierter Kochsalzlösung wird über Natriumsulfat getrocknet, filtriert und eingeengt. Nach Chromatographie an Kieselgel mit einem Gemisch aus Ethylacetat und Hexan werden 4,28 g 34d) erhalten.
¹H-NMR (CDCl₃); d = 0,98 ppm (s, 3H, H-18); 1,04 (1,13) (d, J = 7,5 Hz, 16-CH₃); 1,24 (1,22) (d, J = 6,5 Hz, 3H, H-21); 3,78 (s, 3H, 3-OCH₃); 3,88-3,98 (m, 1H, H-20); 6,62 (d, J = 3Hz, 1H, H-4); 6,71 (dd, J = 9 und 3Hz, 1H, H-2); 7,21 (d, J = 9Hz, 1H, H-1)
(Signale des 2. Diastereomeren in Klammern)

### e) 3-Methoxy-16α-methyl-14,17-ethano-19-norpregna-2,5(10)-dien-20ξ-ol

4,26 g der unter 34d) beschriebenen Verbindung werden nach der in Beispiel 1d) beschriebenen Methode umgesetzt. Es werden 4,45 g 34e) als Rohprodukt erhalten.
¹H-NMR (CDCl₃); d = 0,94 ppm (s, 3H, H-18); 1,02 (d, J = 7,5 Hz, 16-CH₃); 1,20 (d, J = 6,5 Hz, 3H, H-21); 3,53 (s, 3H, 3-OCH₃); 3,84-3-96 (m, 1H, H-20); 4,64 (s breit, 1H, H-2)
(NMR-Daten nur für das Hauptdiastereomer)

### f) 16α-Methyl-14,17-ethano-19-norpregna-4,6-dien-3,20-dion

2 g der unter 34e) beschriebenen Verbindung werden nach den in den Beispielen 1e), 2a), 2b) und 7c) beschriebenen Methoden umgesetzt. Es werden 446 mg 34f) erhalten.
Fp.: 165°C [a]_{D}²⁰ = +16,4° (CHCl₃; c = 0,525)
¹H-NMR (CDCl₃): d = 0,98 ppm (s, 3H, H-18); 1,00 (d, J = 7,5 Hz, 3H, 16-CH₃); 2,11 (s, 3H, H-21); 5,78 (s breit, 1H, H-4); 6,08-6,22 (m, 2H, H-6 und H-7)

### Beispiel 35

### 16α-Methyl-14,17-ethano-19-norpregna-4,9-dien-3.20-dion

2,5 g der unter 34e) beschriebenen Verbindung werden nach den in den Beispielen 4a), 4b) und 7c) beschriebenen Methoden umgesetzt. Es werden 410 mg 35) erhalten.
Fp.: 125-126°C [a]_{D}²⁰ = -300,7° (CHCl₃; c = 0,530)
¹H-NMR (CDCl₃); d = 0,98 (d, J = 7,5 Hz, 3H, 16-CH₃); 1,07 ppm (s, 3H, H-18); 2,11 (s, 3H, H-21); 5,68 (s breit, 1H, H-4)

### Beispiel 36

### 16α,21-Dimethyl-14,17-ethano-19-norpregna-4,9-dien-3,20-dion

3,2 g der unter 34e) beschriebenen Verbindung werden nach den in den Beispielen 4a), 4b), 9a), 7c), 7a) und le) beschriebenen Methoden umgesetzt. Es werden 308 mg 36) erhalten.
[a]_{D}²⁰ = -274,3° (CHCl₃; c = 0,535)
¹H-NMR (CDCl₃); d = 0,99 (d, J = 7,5 Hz, 3H, 16-CH₃); 1,04 (t, J = 7 Hz, 3H, H-22); 1,08 ppm (s, 3H, H-18); 5,69 (s breit, 1H, H-4)

### Beispiel 37

### 21-Hydroxy-16α-methyl-14,17-ethano-19-norpregn-4-en-3,20-dion

1,25 g der unter 34e) beschriebenen Verbindung werden nach den in den Beispielen 1e), 9a), 7c), 5c,) 5d), 1e) und 5g) beschriebenen Methoden umgesetzt. Es werden 73 mg 37) erhalten.
[a]_{D}²⁰ = +56,4° (CHCl₃; c = 0,250)
¹H-NMR (CDCl₃): d = 0,97 (d, J = 7,5 Hz, 3H, 16-CH₃); 0,98 ppm (s, 3H, H-18); 4,22 (s breit, 2H, H-21); 5,82 (s breit, 1H, H-4)

### Beispiel 38

### 17²-Methyl-14,17-etheno-19-norpregn-4-en-3,20-dion

### a) 17α-Ethinyl-3-methoxy-15-methylestra-1,3,5(10),15-tetraen-17β-ol

Bei 0 °C wird für 30 Minuten Acetylen in 500 ml Tetrahydrofuran eingeleitet. Anschließend werden 230 ml einer 1,6 molaren Lösung von n-Butyllithium in Hexan zugetropft. Nach weiteren 30 Minuten wird eine Lösung von 12,1 g 3-Methoxy-15-methyl-estra-1,3,5(10),15-tetraen-17-on (siehe DE 4326240 A1) in 250 ml Tetrahydrofuran zugetropft. Nach 30 Minuten wird zwischen halbgesättigter Kochsalzlösung und Ethylacetat verteilt, die organische Phase mit halbgesättigter und gesättigter Kochsalzlösung gewaschen, über Natriumsulfat getrocknet, filtriert und eingeengt. Dabei setzt Kristallisation ein. Insgesamt werden 12,12 g 38a) erhalten
[a]_{D}²⁰ = -191,4° (CHCl₃; c = 0,500)
¹H-NMR (CDCl₃): d = 0,95 ppm (s, 3H, H-18); 1,90 (s breit, 3H, 15-CH₃); 2,66 (s. 1H, 17-Ethinyl); 3,79 (s, 3H, 3-OCH₃); 5,40 (s breit, 1H, H-16); 6,64 (d, J = 3Hz, 1H, H-4); 6,73 (dd, J = 9 und 3Hz, 1H, H-2); 7,22 (d, J = 9Hz, 1H, H-1)

### b) 3-Methoxy-17²-methyl-14,17-etheno-19-norpregna-1,3,5(10)-trien-20-on

12,10g der unter 38a) beschriebenen Verbindung werden nach den in den Beispielen 1a) und 1b) beschriebenen Methoden umgesetzt. Es werden 8,95g 38b) erhalten.
Fp.: 123,5-125°C [a]_{D}²⁰ = -207,5° (CHCl₃; c = 0,520)
¹H-NMR (CDCl₃): d = 0,86 ppm (s, 3H, H-18); 1,88 (s breit, 3H, 17²-CH₃); 2,21 (s, 3H, H-21); 3,79 (s, 3H, 3-OCH₃); 5,66 (s breit, 1H, H-17¹); 6,63 (d, J = 3Hz, 1H, H-4); 6,73 (dd, J = 9 und 3Hz, 1H, H-2); 7,22 (d, J = 9Hz, 1H, H-1)

### c) 20,20-[1,2-Ethandiylbis(oxy)]-3-methoxy-172-methyl-14,17-etheno-19-norpregna-2,5(10)-dien

1,50 g der unter 38b) beschriebenen Verbindung werden nach den in den Beispielen 1c) und 1d) beschriebenen Methoden umgesetzt. Es werden 1,65g rohes 38c) erhalten.
¹H-NMR (CDCl₃): d = 0,92 ppm (s, 3H, H-18); 1,32 (s, 3H, H-21); 1,80 (s breit, 3H, 17²-CH₃); 3,56 (s, 3H, 3-OCH₃); 3,83-4,02 (m, 4H, 20-OCH₂CH₂O-); 4,65 (s breit, 1H, H-2); 5,53 (s breit, 1H, H-17¹)

### d) 17²-Methyl-14,17-etheno-19-norpregn-4-en-3,20-dion

270 mg der unter 38c) beschriebenen Verbindung werden nach der in Beispiel 1e) beschriebenen Methoden umgesetzt. Es werden nach HPLC 126 mg 38d) erhalten.
¹H-NMR (CDCl₃); d = 0,90 ppm (s, 3H, H-18); 1,82 (s breit, 3H, 17²-CH₃); 2,18 (s, 3H, H-21); 5,63 (s breit, 1H, H-17¹); 5,85 (s breit, 1H, H-4)

### Beispiel 39

### 17²-Methyl-14,17-etheno-19-norpregna-4,9-dien-3,20-dion

1,41 g der unter 38c) beschriebenen Verbindung werden nach den in den Beispielen 4a) und 4b) beschriebenen Methoden umgesetzt. Es werden nach HPLC 178 mg 39) erhalten.
[a]_{D}²⁰ = -306,2° (CHCl₃; c = 0,510)
¹H-NMR (CDCl₃): d = 0,98 ppm (s, 3H, H-18); 1,73 (s breit, 3H, 17²-CH₃); 2,18 (s, 3H, H-21); 5,67 und 5,73 (s breit, je 1H, H-4 und H-17¹)

### Beispiel 40

### (17²R)-17²-Methyl-14,17-ethano-19-norpregn-4-en-3,20-dion

### a) (17²R)-3-Methoxy-17²-methyl-14,17-ethano-19-norpregna-1,3,1(10)-trien-20-on

7,95 g der unter 38b) beschriebenen Verbindung werden nach der in Beispiel 12a) beschriebenen Methode umgesetzt. Es werden 6,97 g 40a) erhalten.
Fp.: 107,5-109,5°C
¹H-NMR (CDCl₃): d = 0,94 ppm (s, 3H, H-18); 1,07 (d, J = 7,5 Hz, 3H, 17²-CH₃); 2,11 (s, 3H, H-21); 3,78 (s, 3H, 3-OCH₃); 6,61 (d, J = 3Hz, 1H, H-4); 6,72 (dd, J = 9 und 3Hz, 1H, H-2); 7,23 (d, J = 9Hz, 1H, H-1)

### b) (17²R)-20,20-[1,2-Ethandiylbis(oxy)]-3-methoxy-17²-methyl-14,17-ethano-19-norpregna-2,5(10)-dien

3,5g der unter 40a) beschriebenen Verbindung werden nach den in den Beispielen 1c) und 1d) beschriebenen Methoden umgesetzt. Es werden 4,0 g rohes 40b) erhalten, welches ohne Reinigung weiter umgesetzt wird.
¹H-NMR (CDCl₃); d = 0,99 ppm (s, 3H, H-18); 0,99 (d, J = 7,5 Hz, 3H, 17²-CH ₃); 2,11 (s, 3H, H-21); 3,55 (s, 3H, 3-OCH₃); 3,83-4,00 (m, 4H, 20-OCH2CH2O-); 4,64 (s breit, 1H, H-2)

### c) (17²R)-17²-Methyl-14,17-ethano-19-norpregn-4-en-3,20-dion

0,27g der unter 40b) beschriebenen Verbindung werden nach der in Beispiel 1e) beschriebenen Methode umgesetzt. Es werden 0,14g 40c) erhalten.
¹H-NMR (CDCl₃):d = 0,95 ppm (s, 3H, H-18); 1,05 (d, J = 7,5 Hz, 3H, 17²-CH₃); 2,05 (s, 3H, H-21); 5,79 (s breit, 1H, H-4)

### Beispiel 41

### (17²R)-17²-Methyl-14,17-ethano-19-norpregna-4,6-dien-3,20-dion

1,1 g der unter 40b) beschriebenen Verbindung werden nach den in den Beispielen 1e), 2a) und 2b) beschriebenen Methoden umgesetzt. Es werden 0,21 g 41) erhalten.
[a]_{D}²⁰ = +117,3° (CHCl₃; c = 0,450)
¹H-NMR (CDCl₃); d = 0,98 ppm (d, J = 7,5 Hz, 3H, 17²-CH₃); 0,99 (s, 3H, H-18); 2,08 (s, 3H, H-21); 5,68 (s breit, 1H, H-4); 6,11-6,27 (m, 2H, H-6 und H-7)

### Beispiel 42

### (17²R)-17²-Methyl-14,17-ethano-19-norpregna-4,9-dien-3,20-dion

1,4 g der unter 40b) beschriebenen Verbindung werden nach den in den Beispielen 4a) und 4b) beschriebenen Methoden umgesetzt. Es werden 0,56 g 42) erhalten.
Fp.: 118-120°C [a]_{D}²⁰ = -270,5° (CHCl₃; c = 0,495)
¹H-NMR (CDCl₃); d = 1,06 ppm (s, 3H, H-18); 1,09 (d, J = 7,5 Hz, 3H, 17²-CH₃); 2,09 (s, 3H, H-21); 5,66 (s breit, 1H, H-4)

### Beispiel 43

### (17²R)-17²,21-Dimethyl-14,17-ethano-19-norpregna-4,9-dien-3,20-dion

### a) (17²R)-17²,21-Dimethyl-20,20-[1,2-ethandiylbis(oxy)]-14,17-ethano-19-norpregn-5(10)-en-3-on

3,65 g der unter 40a) beschriebenen Verbindung werden nach den in den Beispielen 7a), 1c), 1d) und 8c) beschriebenen Methoden umgesetzt. Es werden 2,33 g 43a) erhalten und daneben 0,63 g (17²*R*)-17²,21-Dimethyl-20,20-[1,2-ethandiylbis(oxy)]-14,17-ethano-19-norpregn-4-en-3-on.
¹H-NMR (CDCl₃): d = 0,86 ppm (t, J = 7,7 Hz, 3H, H-22); 0,99 (s, 3H, H-18); 1,00 (d, J = 7,5 Hz, 3H, 17²-CH₃); 2,67 und 2,78 (d, J = 20 Hz, je 1H, H-4); 3,90-4,08 (m, 4H, 20-OCH2CH2O-)

### b) (17²R)-17²,21-Dimethyl-14,17-ethano-19-norpregna-4,9-dien-3,20-dion

2,33 g der unter 43a) beschriebenen Verbindung werden nach den in den Beispielen 4b) und 1e) beschriebenen Methoden umgesetzt. Es werden 0,8 g 43b) erhalten und daneben 0,48 g (172R)-17²,21-Dimethyl-14,17-ethano-19-norpregna-5(10),9(11)-dien-3,20-dion.
[a ]_{D}²⁰ für Verbindung 43b) = -285,4° (CHCl₃; c = 0,515)
¹H-NMR (CDCl₃) für Verbindung 43b): d = 1,00 ppm (t, J = 7,5 Hz, 3H, H-22); 1,05 (s, 3H, H-18); 1,08 (d, J = 7,5 Hz, 3H, 17²-CH₃); 5,67 (s breit, 1H, H-4)
¹H-NMR (CDCl₃) für (172R)-172,21-Dimethyl-14,17-ethano-19-norpregna-5(10),9(11)-dien-3,20-dion: d = 0,86 ppm (s, 3H, H-18); 1,00 (t, J = 7,5 Hz, 3H, H-22); 1,04 (d, J = 7,5 Hz, 3H, 17²-CH₃); 2,88 (s breit, 2H, H-4); 5,59-5,68 (m, 1H, H-11)

### Beispiel 44

### (17²R)-17²,21-Dimethyl-14,17-ethano-19-norpregna-4,9,11-trien-3,20-dion

0,45 g der in Beispiel 43b) beschriebenen Verbindung (17²*R*)-17²,21-Dimethyl-14,17-ethano-19-norpregna-5(10),9(11)-dien-3,20-dion werden nach der in Beispiel 9c) beschriebenen Methode umgesetzt. Es werden 0,16 g 44) erhalten.
[a ]_{D}²⁰ = -48,1° (CHCl₃; c = 0,455)
¹H-NMR (CDCl₃); d = 1,00 ppm (s, 3H, H-18); 1,02 (d, J = 7,5 Hz, 3H, 17²-CH₃); 1,03 (t, J = 7,5 Hz, 3H, H-22); 5,76 (s breit, 3H, H-4); 6,44 (d, J = 12 Hz; 1H, H-11); 6,48 (d, J = 12 Hz; 1H, H-12)

### Beispiel 45

### (17-²R)-17²,21-Dimethyl-14,17-ethano-19-norpregna-4,6-dien-3,20-dion

0,62 g der in Beispiel 43a) beschriebenen Verbindung (17²*R*)-17²,21-Dimethyl-20,20-[1,2-ethandiylbis(oxy)]-14,17-ethano-19-norpregn-4-en-3-on werden nach den in den Beispielen 2a), 2b) und 1e) beschriebenen Methoden umgesetzt. Es werden 0,13 g 45) erhalten.
¹H-NMR (CDCl₃) für Verbindung 43b): d = 0,93-1,02 ppm (m, 9H, 17²-CH₃ H-18 und H-22); 5,76 (s breit, 1H, H-4); 6,12-6,24 (m, 2H, H-6 und H-7)

### Beispiel 46

### 14,17-Ethano-19-norpregna-4,15-dien-3,20-dion

### a) 15β,16β-Dihydro-3-methoxy[1,3]dioxolo[4',5':15,16]-14,17-etheno-19-norpregna-1,3,5(10)-trien-2',20-dion

56 g der in Beispiel 1a) beschriebenen Verbindung werden mit 90,5 ml Vinylencarbonat und 50 mg Hydrochinon versetzt und unter Argon für 18 Stunden bei einer Badtemperatur von 170 °C gehalten. Nach Entfernung aller flüchtigen Bestandteile im Hochvakuum wird der Rückstand an Kieselgel mit einem Gemisch aus Ethylacetat und Hexan chromatographiert. Es werden nach Kristallisation aus einem Gemisch von Diisopropylether und Aceton 56,04 g 46a) erhalten.
Fp.: 217-217,5°C [a]_{D}²⁰ = -219,8° (CHCl₃; c = 0,495)
¹H-NMR (CDCl₃); d = 0,94 ppm (s, 3H, H-18); 2,30 (s, 3H, H-21); 3,79 (s, 3H, 3-OCH₃); 4,99 und 5,76 (2d, J = 8 Hz, H-15 und H-16); 6,31 und 6,40 (2d, J = 6 Hz, je 1H, H-17¹ und H-17²): 6.66 (d, J = 3 Hz, 1H, H-4); 6,72 (dd. J = 9, 3 Hz, 1H, H-2); 7,18 (d, J = 9 Hz, 1H, H-1)

### b) 15β,16β-Dihydro-3-methoxy[1,3]dioxolo[4',5':15,16]-14,17-ethano-19-norpregna-1,3,5(10)-trien-2',20-dion

56 g der in Beispiel 46a) beschriebenen Verbindung werden nach der in Beispiel 12a) beschriebenen Methode umgesetzt. Es werden 56 g 46b) erhalten.
Fp.: 223-224°C [a]_{D}²⁰ = -111.2° (CHCl₃; c = 0,515)
¹H-NMR (CDCl₃): d = 0,94 ppm (s, 3H, H-18); 2,20 (s, 3H, H-21); 3,78 (s, 3H, 3-OCH₃); 4,68 und 5,48 (2dd, J = 1,5 und 9 Hz, je 1H, H-17¹ und H-17²); 6,64 (d, J = 3 Hz, 1H, H-4); 6,73 (dd. J = 9, 3 Hz, 1H, H-2); 7,19 (d, J = 9 Hz, 1H, H-1)

### c) 15α,16α-Dihydroxy-3-methoxy-14,17-ethano-19-norpregna-1,3,5(10)-trien-20-on

50 g der in Beispiel 46b) beschriebenen Verbindung werden mit 26 g Kaliumcarbonat in einem Gemisch aus 250 ml Methanol, 500 ml Tetrahydrofuran und 150 ml Wasser 6 Stunden zum Sieden erhitzt. Nach weitgehender Entfernung der Lösemittel wird auf 2 Liter Eiswasser gegossen, abgesaugt und der Filterkuchen mit 1 Liter Wasser gewaschen. Es werden 45,80 g 46c) erhalten.
¹H-NMR (CDCl₃); d = 0,91 ppm (s, 3H, H-18); 2,18 (s, 3H, H-21); 3,78 (s, 3H, 3-OCH₃); 3,84-3,93 und 4,62-4,71 (2m, je 1H, H-17¹ und H-17²); 6,63 (d, J = 3 Hz, 1H, H-4); 6,72 (dd, J = 9, 3 Hz, 1H, H-2); 7,21 (d, J = 9 Hz, 1H, H-1)

### d) 3-Methoxy-14,17-ethano-19-norpregna-1,3,5(10),15-tetraen-20-on

45,7 g der in Beispiel 46c) beschriebenen Verbindung werden in 1,5 Liter Dichlormethan gelöst und bei 0°C mit 150 ml Trimethylorthoformiat sowie 6 g Pyridiniumparatoluolsulfonat versetzt. Nach 6 Stunden bei Raumtemperatur wird der Ansatz über eine Kieselgelsäule filtriert und eingeengt. Der Eindampfrückstand wird in 1 Liter Acetanhydrid aufgenommen und 5 Stunden zum Sieden erhitzt. Nach einengen wird der Rückstand zwischen konzentrierter Natriumhydrogencarbonatlösung und Dichlormethan verteilt. Nach dem Waschen der organischen Phase mit konzentrierter Kochsalzlösung, trocknen über Natriumsulfat, filtrieren und einengen wird an Kieselgel mit einem Gemisch aus Ethylacetat und Hexan chromatographiert. Es werden 6,75 g 46d) erhalten. Alle polareren Fraktionen der Chromatographie werden vereinigt und eingeengt. Der Rückstand wird mit 20 g Kaliumcarbonat in 800 ml Methanol 3 Stunden zum Sieden erhitzt und auf 2 Liter Eiswasser gegossen, abgesaugt und der Filterkuchen mit 0,5 Liter Wasser gewaschen. Es werden 27,5 g der in Beispiel 46c) beschriebenen Verbindung erhalten, woraus nach der in Beispiel 46d) beschriebenen Methode weitere 4,50 g 46 d) erhalten werden.
[a]_{D}²⁰ = +0,5° (CHCl₃; c = 0,505)
¹H-NMR (CDCl₃); d = 0,91 ppm (s, 3H, H-18); 2,23 (s, 3H, H-21); 3,79 (s, 3H, 3-OCH₃); 6,22 und 6,13 (2d, J = 6 Hz, je 1H, H-15 und H-16); 6,66 (d, J = 3 Hz, 1H, H-4); 6,73 (dd, J = 9, 3 Hz, 1H, H-2); 7,21 (d, J = 9 Hz, 1H, H-1)

### e) 20,20-[1,2-Ethandiylbis(oxy)]-3-methoxy-14,17-ethano-19-norpregna-2,5(10),15-trien

4,5 g der in Beispiel 46d) beschriebenen Verbindung werden nach den in den Beispielen 1c) und 1d) beschriebenen Methoden umgesetzt. Es werden 5,33 g rohes 46e) erhalten.
¹H-NMR (CDCl₃); d = 0,98 ppm (s, 3H, H-18); 1,32 (s, 3H, H-21); 3,56 (s, 3H, 3-OCH₃); 3,93-4,07 (m, 4H, 20-OCH₂CH₂O-); 4,65 (s breit, 1H, H-2); 5,94 und 6,03 (2d, J = 6 Hz, je 1H, H-15 und H-16)

### f) 14,17-Ethano-19-norpregna-4,15-dien-3,20-dion

2,0 g der in Beispiel 46e) beschriebenen Verbindung werden nach der in Beispiel le) beschriebenen Methode umgesetzt. Es werden 1,32 g rohes 46f) erhalten.
Fp.: 131-133°C [a]_{D}²⁰ = +32,0° (CHCl₃; c = 0,505)
¹H-NMR (CDCl₃); d = 0,93 ppm (s, 3H, H-18); 2,20 (s, 3H, H-21); 5.85 (s breit, 1H, H-4); 6,05 und 6,19 (2d, J = 6 Hz, je 1H, H-15 und H-16)

### Beispiel 47

### 14,17-Ethano-19-norpregna-4,6,15-trien-3.20-dion

1,2 g der in Beispiel 46f) beschriebenen Verbindung werden nach den in den Beispielen 2a) und 2b) beschriebenen Methoden umgesetzt. Es werden 0,54 g 47) erhalten.
Fp.: 138-140°C
[a]_{D}²⁰ = -28,7° (CHCl₃; c = 0,480)
¹H-NMR (CDCl₃); d = 0,94 ppm (s, 3H, H-18); 2,21 (s, 3H, H-21); 5,79 (s breit, 1H, H-4); 6,14 bis 6,34 (m, 4H, H-6 und H-7 und H-15 und H-16)

### Beispiel 48

### 14,17-Ethano-19-norpregna-4,9,15-trien-3,20-dion

3,33 g der in Beispiel 46e) beschriebenen Verbindung werden nach den in den Beispielen 8c) und 4b) beschriebenen Methoden umgesetzt. Es werden 1,08 g 48) erhalten.
[a ]_{D}²⁰ = -272,4° (CHCl₃; c = 0,475)
¹H-NMR (CDCl₃); d = 1,01 ppm (s, 3H, H-18); 2,21 (s, 3H, H-21); 5,70 (s breit, 1H, H-4); 6,06 und 6,23 (2d, J = 6 Hz, 2H, H-15 und H-16)

### Beispiel 49

### 21-Hydroxy-14,17-ethano-19-norpregna-4,4,15-trien-3,20-dion

### a) 3,3-[1,2-Ethandiylbis(oxy)]-14,17-ethano-19-norpregna-5(10),9(11),15-trien-20-on

1,0 g der in Beispiel 48) beschriebenen Verbindung werden nach der in Beispiel 1c) beschriebenen Methode umgesetzt. Es werden 0,29 g 49a) erhalten, sowie 0,7 g des entsprechenden 3,20-Bisketals.
¹H-NMR (CDCl₃); d = 0,82 ppm (s, 3H, H-18); 2,22 (s, 3H, H-21); 4,00 (s, 4H, 3-OCH₂CH₂O-); 5,51 (s breit, 1H, H-11); 6,04 und 6,22 (2d, J = 6 Hz, 2H, H-15 und H-16)

### b) 21-Hydroxy-14,17-ethano-19-norpregna-4,9,15-trien-3,20-dion

0,28 g der in Beispiel 49a) beschriebenen Verbindung werden nach den in den Beispielen 5c), 5d), 1e) und 5g) beschriebenen Methoden umgesetzt. Es werden 11 mg 49b) erhalten.
¹H-NMR (CDCl₃); d = 1,00 ppm (s, 3H, H-18); 4,33 und 4,42 (2d, J = 20 Hz, 2H, H-21); 5,70 (s breit, 1H, H-4); 6,12 und 6,15 (2d, J = 6 Hz, 2H, H-15 und H-16)

### Beispiel 50

### 21-Methyl-14,17-ethano-19-norpregna-4,15-dien-3,20-dion

### a) 20,20-[1,2-Ethandiylbis(oxy)]-21-methyl-14,17-ethano-19-norpregna-4,15-dien-3-on

6,6 g der in Beispiel 46d) beschriebenen Verbindung werden nach den in den Beispielen 7a), 8a), 1d) und 8c) beschriebenen Methoden umgesetzt. Es werden 0,605 g 50a) erhalten, sowie 3,70 g 20,20-[1,2-Ethandiylbis(oxy)]-21-methyl-14,17-ethano-19-norpregna-5(10),15-dien-3-on.
¹H-NMR (CDCl₃) für Verbindung 50a): d = 0,93 ppm (t, J = 7 Hz, 3H, H-22): 0,99 (s, 3H, H-18); 3,98-4,15 (m, 4H, 20-OCH₂CH₂O-); 5,83 (s breit, 1H, H-4); 5,96 (s, 2H, H-15 und H-16)
¹H-NMR (CDCl₃) für 20,20-[1,2-Ethandiylbis(oxy)]-21-methyl-14,17-ethano-19-norpregna-5(10),15-dien-3-on: d = 0,94 ppm (t, J = 7 Hz, 3H, H-22); 0,96 (s, 3H, H-18); 2,70 und 2,80 (2d, J = 20 Hz, 2H, H-4); 3,96-4,14 (m, 4H, 20-OCH₂CH₂O-); 5,94 und 6,01 (2d, J = 6 Hz, 2H, H-15 und H-16)

### b) 21-Methyl-14,17-ethano-19-norpregna-4,15-dien-3,20-dion

142 mg der in Beispiel 50a) beschriebenen Verbindung werden nach der in Beispiel le) beschriebenen Methode umgesetzt. Es werden 127 g 50b) erhalten.
Fp.: 140-141°C [a]_{D}²⁰ = +25,4° (CHCl₃; c = 0,495)
¹H-NMR (CDCl₃); d = 0,90 ppm (s, 3H, H-18); 1,07 (t, J = 7 Hz, 3H, H-22); 5,85 (s breit, 1H, H-4); 6,05 und 6,20 (2d, J = 6 Hz, 2H, H-15 und H-16)

### Beispiel 51

### 21-Methyl-14,17-ethano-19-norpregna-4,6,15-trien-3,20-dion

460 mg der in Beispiel 50a) beschriebenen Verbindung werden nach den in den Beispielen 2a), 2b) und le) beschriebenen Methoden umgesetzt. Es werden 210 mg 51) erhalten.
Fp.: 153,5-154-5°C [a]_{D}²⁰ = -37,9° (CHCl₃; c = 0,490)
¹H-NMR (CDCl₃); d = 0,93 ppm (s, 3H, H-18); 1,08 (t, J = 7 Hz, 3H, H-22); 5,80 (s breit, 1H, H-4); 6,16 bis 6,36 (m, 4H, H-6 und H-7 und H-15 und H-16)

### Beispiel 52

### 21-Methyl-14,17-ethano-19-norpregna-4,9,15-irien-3,20-dion

3,7 g 20,20-[1,2-Ethandiylbis(oxy)]-21-methyl-14,17-ethano-19-norpregna-5(10),15-dien-3-on aus Beispiel 50a) werden nach den in den Beispielen 4b) und le) beschriebenen Methoden umgesetzt. Es werden 2,1 g 52) erhalten.
¹H-NMR (CDCl₃): d = 1,00 ppm (s, 3H, H-18); 1,08 (t, J = 7 Hz, 3H, H-22); 5,70 (s breit, 1H, H-4); 6,06 und 6,24 (2d, J = 6 Hz, 2H, H-15 und H-16)

### Beispiele 53 und 54

### 53: (21R)-21-Hydroxy-21-methyl-14,17-ethano-19-norpregna-4,9,15-trien-3,20-dion

### 54: (21S)-21-Hydroxy-21-methyl-14,17-ethano-19-norpregna-4,9,15-trien-3,20-dion

1,72 g der in Beispiel 52) beschriebenen Verbindung werden nach den in den Beispielen 9a), 5c), 5d), le) und 5g) beschriebenen Methoden umgesetzt. Es werden 198 mg 53) und 250 mg 54) erhalten.
**53:** [a]_{D}²⁰ = -290,0° (CHCl₃; c = 0,520)
   ¹H-NMR (CDCl₃); d = 0,95 ppm (s, 3H, H-18); 1,38 (d, J = 7 Hz, 3H, H-22); 4,43 bis 4,56 (m, 1H, H-21); 5,68 (s breit, 1H, H-4); 6,15 und 6,22 (2d, J = 6 Hz, 2H, H-15 und H-16)
**54:** [a]_{D}²⁰ = -218,4° (CHCl₃; c = 0,515)
   ¹H-NMR (CDCl₃); d = 0,98 ppm (s, 3H, H-18); 1,38 (d, J = 7 Hz, 3H, H-22); 4,41 bis 4,52 (m, 1H, H-21); 5,71 (s breit, 1H, H-4); 6,10 und 6,18 (2d, J = 6 Hz, 2H, H-15 und H-16)

### Beispiel 55

### 16-Methyl-14,17-ethano-19-norpregna-4,15-dien-3.20-dion

### a) 3-Methoxy-20-oxo-14,17-etheno-19-norpregna-1,3,5(10),15-tetraen-16-carbonsäuremethylester

20 g der in Beispiel la) beschriebenen Verbindung, 20 ml Propiolsäuremethylester und 50 mg Hydrochinon werden in einem geschlossenen Rohr unter Argon 34 Stunden bei 110°C Badtemperatur gehalten. Nach Abkühlung, Entfernung flüchtiger Bestandteile und Chromatographie des Rückstandes an Kieselgel mit einem Gemisch aus Ethylacetat und Hexan werden 12,66 g 55a) erhalten.
Fp.: 149-149,5°C [a]_{D}²⁰ = -8,3° (CHCl₃; c = 0,505)
¹H-NMR (CDCl₃); d = 1,30 ppm (s, 3H, H-18); 2,28 (s, 3H, H-21); 3,73 und 3,78 (2s, je 3H, 3-OCH₃ und CO₂CH₃): 6,65 (d, J = 3Hz, 1H, H-4); 6,73 (dd, J = 9 und 3Hz, 1H, H-2); 6,76 und 7,02 (2d, J = 6Hz, je 1H, H-17¹ und H-17²); 7,20 (d, J = 9Hz, 1H, H-1); 7,58 (s, 1H, H-15)

### b) 20,20-[1,2-Ethandiylbis(oxy)]-3-methoxy-14,17-etheno-19-norpregna-1,3,5(10),15-tetraen-16-carbonsäuremethylester

10,83 g der in Beispiel 55a) beschriebenen Verbindung werden nach der in Beispiel 1c) beschriebenen Methoden umgesetzt. Es werden 11,46 g 55b) erhalten.
Fp.: 147-147,5°C [a]_{D}²⁰ = -14.7° (CHCl₃; c = 0,530)
¹H-NMR (CDCl₃); d = 1,28 ppm (s, 3H, H-18); 1,55 (s, 3H, H-21); 3,73 und 3,78 (2s, je 3H, 3-OCH₃ und CO₂CH₃); 3,95 bis 4,11 (m, 4H, 20 -OCH₂CH₂O-); 6,64 (d, J = 3Hz, 1H, H-4); 6,72 (dd, J = 9 und 3Hz, 1H, H-2); 6,67 und 6,80 (2d, J = 6Hz, je 1H, H-17¹ und H-17²); 7,21 (d, J = 9Hz, 1H, H-1); 7,50 (s, 1H, H-15)

### c) 20,20-[1,2-Ethandiylbis(oxy)]-3-methoxy-14,17-ethano-19-norpregna-1,3,5(10),15-tetraen-16-carbonsäuremethylester

2,50 g der in Beispiel 55b) beschriebenen Verbindung werden an 100 mg Palladium auf Kohle (10%) in einem Gemisch aus je 250 ml Methanol und Ethylacetat bei Normaldruck hydriert, bis 1 Equivalent Wasserstoff aufgenommen ist. Nach Abfiltern vom Katalysator, einengen und Chromatographie an Kieselgel mit einem Gemisch aus Ethylacetat und Hexan werden 1,99 g 55c) erhalten.
¹H-NMR (CDCl₃); d = 0,97 ppm (s, 3H, H-18); 1,54 (s, 3H, H-21); 3,73 und 3,78 (2s, je 3H, 3-OCH₃ und CO₂CH₃); 3,90 bis 4,06 (m, 4H, 20 -OCH₂CH₂O-); 6,64 (d, J = 3Hz, 1H, H-4); 6,72 (dd, J = 9 und 3Hz, 1H, H-2); 6,92 (s, 1H, H-15); 7,21 (d, J = 9Hz, 1H, H-1)

### d) 20,20-[1,2-Ethandiylbis(oxy)]-3-methoxy-14,17-ethano-19-norpregna-1,3,1(10),15-tetraen-16-methanol

2,52 g der in Beispiel 55c) beschriebenen Verbindung, gelöst in 40 ml Tetrahydrofuran werden mit 157 mg Zinkchlorid versetzt. Bei -78 °C werden 24 ml einer 1,2 molaren Lösung von Diisobutylaluminiumhydrid in Toluol zugetropft. Anschließend wird 3,5 Stunden bei dieser Temperatur belassen, mit Wasser versetzt, aufgetaut und mit Ethylacetat extrahiert, die organische Phase mit konzentrierter Kochsalzlösung gewaschen, über Natriumsulfat getrocknet, filtriert und eingeengt. Nach Chromatographie an Kieselgel mit einem Gemischaus Ethylacetat und Hexan werden 1,46 g 55d) erhalten.
¹H-NMR (CDCl₃); d = 0,98 ppm (s, 3H, H-18); 1,39 (s, 3H, H-21); 3,24 bis 3,32 (m, 1H, 16-CH₂); 3,78 (s, 3H, 3-OCH₃); 3,94 bis 4,13 (m, 4H, 20 -OCH₂CH₂O-); 4,18 bis 4,26 (m, 1H, 16-CH₂); 6,00 (s breit, 1H, H-15); 6,64 (d, J = 3Hz, 1H, H-4); 6,71 (dd, J = 9 und 3Hz, 1H, H-2); 7,20 (d, J = 9Hz, 1H, H-1)

### e) 16-[(Acetyloxy)methyl]-20,20-[1,2-ethandiylbis(oxy)]-3-methoxy-14,17-ethano-19-norpregna-1,3,5(10),15-tetraen

1,475 g der in Beispiel 55d) beschriebenen Verbindung in 60 ml Pyridin werden bei 0 °C tropfenweise mit 1,3 ml Acetylchlorid versetzt. Nach 1,5 Stunden bei Raumtemperatur wird auf eiskalte konzentrierte Natriumhydrogencarbonatlösung gegossen und mit Ethylacetat extrahiert. Die organische Phase wird nacheinander mit konzentrierter Natriumhydrogencarbonatlösung und Kochsalzlösung gewaschen, über Natriumsulfat getrocknet, filtriert und eingeengt. Es werden 1,85 g rohes 55e) erhalten.
¹H-NMR (CDCl₃): d = 0,97 ppm (s, 3H, H-18); 1,33 (s, 3H, H-21); 2,09 (s, 3H, Acetat); 3,77 (s, 3H, 3-OCH₃); 3,92 bis 4,12 (m, 4H, 20 -OCH₂CH₂O-); 4,74 und 4,82 (2d, J = 1,5 und 20 Hz, je 1H, 16-CH₂); 5,95 (s breit, 1H, H-15); 6,64 (d, J = 3Hz, 1H, H-4); 6,72 (dd, J = 9 und 3Hz, 1H, H-2); 7,20 (d, J = 9Hz, 1H, H-1)

### f) 16-Methyl-14,17-ethano-19-norpregna-4,15-dien-3,20-dion

1,73 g rohes 55e) werden nach den in den Beispielen 1d) und 1e)beschriebenen Methoden umgesetzt. Es werden 34 mg 55f) und 92 mg der in Beispiel 31b) beschriebenen Verbindung erhalten.
¹H-NMR (CDCl₃): d = 0,96 ppm (s, 3H, H-18); 1,77 (s breit, 3H, 16-CH₃); 2,17 (s, 3H, H-21); 5,62 (s breit, 1H, H-15); 5,84 (s breit, 1H, H-4)

### Beispiel 56

### 15β,16α-Dimethyl-14,17-etheno-19-norpregn-4-en-3,20-dion

### a) 20,20-[1,2-Ethandiylbis(oxy)]-3-methoxy-15β-methyl-14,17-etheno-19-norpregna-1,3,5(10)-trien-16α-carbonsäuremethylester

Zu 30,47 g Kupfer(I)-jodid in 420 ml Diethylether werden bei 0 °C 200 ml einer 1,6 molaren Lösung von Methyllithium in Diethylether getropft. Nach 30 Minuten bei dieser Temperatur wird mit 500 ml Tetrahydrofuran verdünnt. Nach Kühlung auf-50 °C werden 7,0 g der in Beispiel 55b) beschriebenen Verbindung in 200 ml Tetrahydrofuran zugetropft. Nach Erwärmung auf 0 °C wird 4 Stunden bei dieser Temperatur belassen. Nach Zusatz von konzentrierter Ammoniumchloridlösung bei -20 °C wird zwischen Wasser und Ethylacetat verteilt, die organische Phase nacheinander mit Ammoniaklösung, Wasser und konzentrierter Kochsalzlösung gewaschen, über Natriumsulfat getrocknet, filtriert, eingeengt und der Rückstand an Kieselgel mit einem Gemisch aus Ethylacetat und Hexan chromatographiert. Es werden 5,47 g 56a) erhalten.
¹H-NMR (CDCl₃); d = 1,18 ppm (d, J = 7 Hz, 3H, 15-CH₃); 1,20 (s, 3H, H-18); 1,30 (s, 3H, H-21); 3,14 (d, J = 5 Hz, 1H, H-16); 3,62 (s, 3H, CO₂CH₃); 3,78 (s, 3H, 3-OCH₃); 3,79 bis 4,13 (m, 4H, 20 -OCH₂CH₂O-); 5,98 und 6,30 (2d, J = 6Hz, je 1H, H-17¹ und H-17²); 6,64 (d, J = 3Hz, 1H, H-4); 6,72 (dd, J = 9 und 3Hz, 1H. H-2); 7,20 (d, J = 9Hz, 1H, H-1)

### b) 15β,16α-Dimethyl-14,17-etheno-19-norpregn-4-en-3,20-dion

2,0 g der in Beispiel 56a) beschriebenen Verbindung werden nach den in den Beispielen 28c), 34a), 34b), 1d) und le) beschriebenen Methoden umgesetzt. Es werden 303 mg 56b) erhalten.
[a]_{D}²⁰ = +99,8° (CHCl₃; c = 0,510)
¹H-NMR (CDCl₃); d = 0,88 und 1,05 ppm (2d, J = 7 Hz, je 3H, 15-CH₃ und 16-CH₃); 1,04 (s, 3H, H-18); 2,14 (s, 3H, H-21); 5,85 (s breit, 1H, H-4); 6,01 und 6,20 (2d, J = 6Hz, je 1H, H-17¹ und H-17²)

### Beispiel 57

### 15β,16α-Dimethyl-14,17-ethano-19-norpregn-4-en-3,20-dion

1,48 g der in Beispiel 56a) beschriebenen Verbindung werden nach den in den Beispielen 28c), 34a), 34b), 55c), 1d) und 1e) beschriebenen Methoden umgesetzt. Es werden 223 mg 57) erhalten.
Fp.: 212-214°C [a]_{D}²⁰ = +21,1° (CHCl₃; c = 0,505)
¹H-NMR (CDCl₃): d = 0,97 und 1,01 ppm (2d, J = 7 Hz, je 3H, 15-CH₃ und 16-CH₃); 1,08 (s, 3H, H-18); 2,09 (s, 3H, H-21); 5,82 (s breit, 1H, H-4)

### Beispiel 58

### 2',5',15β,16β-Tetrahydrofuro[3',4';15,16]-14,17-etheno-19-norpregn-4-en-3.20-dion

### a) 15β,16β-Dihydro-3-methoxy[2H,5H]furo[3',4':15,16]-14,17-etheno-19-norpregna-1,3,5(10)-trien-2',5',20-trion

10,0 g der in Beispiel la) beschriebenen Verbindung und 10,0 g Maleinsäureanhydrid werden 18 Stunden unter Argon bei 95 °C gerührt. Nach Entfernung des überschüssigen Maleinsäureanhydrids im Hochvakuum wird der Rückstand aus Diisopropylether kristallisiert. Es werden 9,8 g 58a) erhalten.
Fp.: 186-187°C (Zersetzung) [a]_{D}²⁰ = +197,0° (CHCl₃; c = 0,500)
¹H-NMR (CDCl₃): d = 1,00 ppm (s, 3H, H-18); 2,35 (s, 3H, H-21); 3,57 und 4,47 (2d, J = 8 Hz, je 1H, H-15 und H-16); 3,79 (s, 3H, 3-OCH₃); 6,41 und 6,49 (2d, J = 6Hz, je 1H, H-17¹ und H-17²); 6,66 (d, J = 3Hz, 1H, H-4); 6,73 (dd, J = 9 und 3Hz, 1H, H-2); 7,18 (d, J = 9Hz, 1H, H-1)

### b) 20,20-[1,2-Ethandiylbis(oxy)]-14,17-etheno-19-norpregna-1,3,5(10)-trien-15α,16α-dimethanol

5,45 g der in Beispiel 58a) beschriebenen Verbindung werden nach den in den Beispielen 34d), 7c), 1c) und 28c) angegebenen Methoden umgesetzt. Es werden 4,13 g rohes 58b) erhalten.
¹H-NMR (CDCl₃); d = 1,01 ppm (s, 3H, H-18); 1,42 (s, 3H, H-21); 3,48 bis 3,58 und 3,60 bis 3,69 (2m, je 1H, CH₂OH); 3,78 (s, 3H, 3-OCH₃); 3,93 bis 4,10 (m, 6H, CH₂OH und 20-OCH₂CH₂O-); 5,96 und 6,04 (2d, J = 6Hz, je 1H, H-17¹ und H-17²); 6,63 (d, J = 3Hz, 1H, H-4); 6,72 (dd, J = 9 und 3Hz, 1H, H-2); 7,19 (d, J = 9Hz, 1H, H-1)

### c) 20,20-[1,2-Ethandiylbis(oxy)]-3-methoxy-2',5',15β,16β-tetrahydrofuro[3',4':15,16]-14,17-etheno-19-norpregna-1,3,5(10)-trien

4,1 g der in Beispiel 58b) beschriebenen Verbindung werden in einem Gemisch aus 70 ml Dichlormethan und 14 ml Pyridin auf 0 °C gekühlt und tropfenweise mit insgesamt 3,34 ml Methansulfonsäurechlorid versetzt. Nach 3 Stunden rühren bei Raumtemperatur wird mit konzentrierter Natriumhydrogencarbonatlösung versetzt. Nach 20 Minuten wird zwischen Wasser und Ethylacetat verteilt, die organische Phase mit konzentrierter Natriumhydrogencarbonatlösung und Kochsalzlösung gewaschen, über Natriumsulfat getrocknet, filtriert, eingeengt und an Kieselgel mit einem Gemisch aus Ethylacetat und Hexan chromatographiert. Es werden 0,81 g 58c) erhalten.
Fp.: 148-150°C [a]_{D}²⁰ = +135,0° (CHCl₃; c = 0,480)
¹H-NMR (CDCl₃); d = 1,19 ppm (s, 3H, H-18); 1,30 (s, 3H, H-21); 3,34 bis 3,83 (m, 4H, 15-CH₂ und 16-CH₂); 3,79 (s, 3H, 3-OCH₃); 3,85 bis 4,08 (m, 4H, 20-OCH₂CH₂O-); 6,12 und 6,18 (2d, J = 6Hz, je 1H, H-17¹ und H-17²); 6,63 (d, J = 3Hz, 1H, H-4); 6,72 (dd, J = 9 und 3Hz, 1H, H-2); 7,21 (d, J = 9Hz, 1H, H-1)

### d) 2',5',15β,16β-Tetrahydrofuro[3',4':15,16]-14,17-etheno-19-norpregn-4-en-3,20-dion

0,41 g der in Beispiel 58c) beschriebenen Verbindung werden nach den in den Beispielen 1d) und le) angegebenen Methoden umgesetzt. Es werden 0,23 g 58d) erhalten.
Fp.: 163,5-165°C [a]_{D}²⁰ = +149,8° (CHCl₃; c = 0,485)
¹H-NMR (CDCl₃): d = 1,08 ppm (s, 3H, H-18); 2,17 (s, 3H, H-21); 3,33 bis 3,46 und 3,60 bis 3,76 (2m, je 2H, 15-CH₂ und 16-CH₂); 5,88 (s breit, 1H, H-4); 6,21 und 6,27 (2d, J = 6Hz, je 1H, H-17¹ und H-17²)

### Beispiel 59

### 2',5',15β,16β-Tetrahydrofuro[3',4':15,16]-14,17-ethano-19-norpregn-4-en-3,20-dion

0,4 g der in Beispiel 58c) beschriebenen Verbindung werden nach den in den Beispielen 55c), 1d) und le) angegebenen Methoden umgesetzt. Es werden 0,234 g 59) erhalten.
Fp.: 187-189°C [a]_{D}²⁰ = +72,8° (CHCl₃; c = 0,520)

### Beispiel 60

### 14,17-Ethano-18a-homo-19-norpregna-4,15-dien-3,20-dion

### a) 3-Methoxy-14,17-ethano-18a-homo-19-norpregna-1,3,5(10),15-tetraen-20-on

34,0 g 3-Methoxy-15-methyl-18a-homoestra-1,3,5(10),15-tetraen-17-on (siehe DE 3710728 A1) werden nach den in den Beispielen 38a), 1a), 58a), und 18a) beschriebenen Methoden und anschließend mit zweinormaler Natronlauge in Tetrahydrofuran umgesetzt. 2,0 g der entstehenden Dicarbonsäure werden als Rohprodukt in 20 ml Pyridin gelöst, mit 2,2 g Bleitetraacetat versetzt und für 10 Stunden auf 70 °C erhitzt. Anschließend wird das Reaktionsgemisch in viernormale Salzsäure eingetragen. Der Niederschlag wird abfiltriert und an Kieselgel mit einem Gemisch aus n-Hexan und Ethylacetat chromatographiert. Es werden 90 mg 60a) erhalten.
¹H-NMR (CDCl₃); d = 0,62 ppm (t, J = 7 Hz, 3H, H-18a); 2,25 (s, 3H, H-21); 3,78 (s, 3H, 3-OCH₃); 6,10 und 6,26 (2d, J = 6 Hz, je 1H, H-15 und H-16); 6,65 (d, J = 3 Hz, 1H, H-4); 6,72 (dd, J = 9, 3 Hz, 1H, H-2); 7,19 (d, J = 9 Hz, 1H, H-1)

### b) 14,17-Ethano-18a-homo-19-norpregna-4,15-dien-3,20-dion

115 mg der unter 60a) beschriebenen Verbindung werden nach den in den Beispielen 1c), 1d) und 1e) beschriebenen Methoden umgesetzt. Es werden 18 mg 60b) erhalten.
¹H-NMR (CDCl₃): d = 0,63 ppm (t, J = 8 Hz, 3H, H-18a); 2,23 (s, 3H, H-21); 5,86 (s breit, 1H, H-4), 6,03 und 6,24 (2d, J = 5Hz, je 1H, H-15 und H-16)

## Patentansprüche

1. 14,17-C₂-überbrückte Steroide der allgemeinen Formel (**I**), 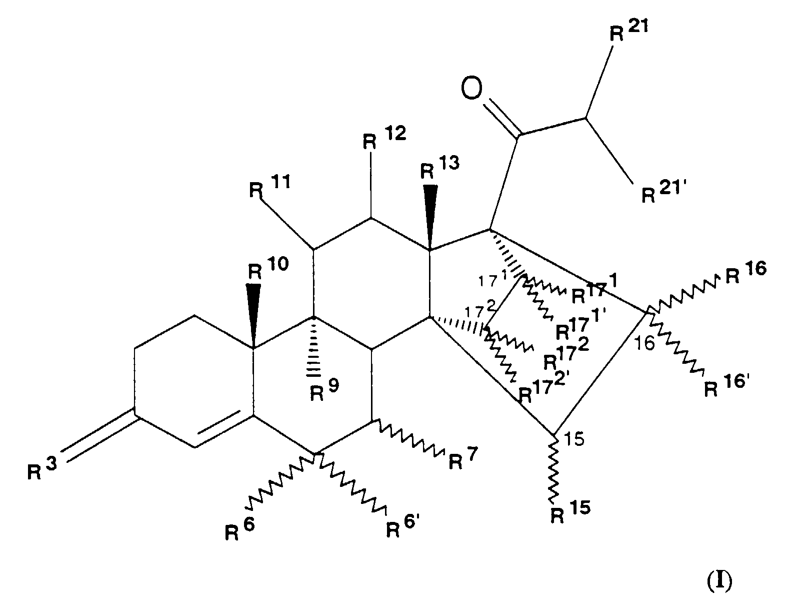 worin
R³ für ein Sauerstoffatom, die Hydroxyiminogruppe oder zwei Wasserstoffatome,
R⁶ für ein Wasserstoff-, Fluor-, Chlor- oder Bromatom oder für einen α- oder β -ständigen C₁-C₄-Alkylrest, wobei dann R^{6'} und R⁷ Wasserstoffatome darstellen, oder aber
R⁶ für ein Wasserstoff-, Fluor-, Chlor- oder Bromatom oder für einen C₁-C₄-Alkylrest, wobei dann R^{6'} und R⁷ eine gemeinsame zusätzliche Bindung darstellen,
R⁷ für einen α- oder β-ständigen C₁-C₄-Alkylrest, wobei dann R⁶ und R^{6'} Wasserstoffatome darstellen, oder aber
R⁶ und R⁷ gemeinsam für eine α- oder β-ständige Methylengruppe und R^{6'} für ein Wasserstoffatom oder
R⁶ und R^{6'} gemeinsam für eine Ethylen- oder Methylengruppe und R⁷ für ein Wasserstoffatom,
R⁹ und R¹⁰ jeweils für ein Wasserstoffatom oder eine gemeinsame Bindung,
R¹¹ und R¹² jeweils für ein Wasserstoffatom oder eine gemeinsame Bindung,
R¹³ für eine Methyl- oder Ethylgruppe,
R¹⁵ für ein Wasserstoffatom oder einen C₁-C₃-Alkylrest,
R¹⁶ und R^{16'} unabhängig voneinander für ein Wasserstoffatom, einen C₁-C₃-Alkylrest
oder einen C₂-C₄-Alkenylrest oder gemeinsam für eine C₁-C₃-Alkylidengruppe
R¹⁵ und R¹⁶ für eine gemeinsame Bindung sowie R^{16'} für ein Wasserstoffatom oder
einen C₁-C₃-Alkylrest oder
R¹⁵ und R¹⁶ gemeinsam für einen Ring der Teilformel 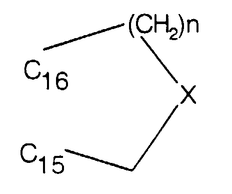 worin n = 1 und 2 und X eine Methylengruppe oder ein Sauerstoffatom bedeuten sowie
R^{16'} für ein Wasserstoffatom,
R^{17¹} für ein Wasserstoffatom oder einen C₁-C₃-Alkylrest,
R^{17²} für ein Wasserstoffatom, einen C₁-C₃-Alkylrest oder einen C₂-C₄-Alkenylrest
R^{17^{1'}} und R^{17^{2'}} jeweils für ein Wasserstoffatom oder für eine gemeinsame Bindung,
R²¹ für ein Wasserstoffatom oder einen C₁-C₃-Alkylrest,
R^{21'} für ein Wasserstoffatom, einen C₁-C₃-Alkylrest oder eine Hydroxygruppe stehen,
ausgenommen der Verbindung 14,17-Ethano-19-norpregn-4-en-3,20-dion.

2. Verbindungen der allgemeinen Formel I nach Anspruch 1, dadurch gekennzeichnet, daß R³ für ein Sauerstoffatom oder zwei Wasserstoffatome steht.

3. Verbindungen der allgemeinen Formel I nach Anspruch 1, dadurch gekennzeichnet, daß R⁶ für ein Wasserstoffatom oder für einen α- oder β-ständigen C₁-C₄-Alkylrest steht, wenn R^{6'} und R⁷ Wasserstoffatome darstellen.

4. Verbindungen der allgemeinen Formel I nach Anspruch 1, dadurch gekennzeichnet, daß R⁶ für ein Wasserstoff-, Chlor- oder Bromatom oder für einen C₁-C₄-Alkylrest steht, wenn R^{6'} und R⁷ eine gemeinsame zusätzliche Bindung darstellen.

5. Verbindungen der allgemeinen Formel I nach Anspruch 1, dadurch gekennzeichnet, daß R¹⁶ und R^{16'} jeweils für ein Wasserstoffatom, jeweils für eine Methylgruppe oder der eine dieser beiden Substituenten für eine C₁-C₄-Alkyl- oder eine Vinylgruppe und der andere dieser beiden Substituenten für ein Wasserstoffatom oder beide Substituenten gemeinsam für eine C₁-C₃-Alkylidengruppe stehen.

6. Verbindungen der allgemeinen Formel I nach Anspruch 1, dadurch gekennzeichnet, daß R^{17¹} für ein Wasserstoffatom oder einen C₁-C₃-Alkylrest und R^{17²} für ein Wasserstoffatom, einen C₁-C₃-Alkylrest oder einen C₂-C₄-Alkenylrest stehen und daß R^{17^{1'}} und R^{17^{2'}} jeweils für ein Wasserstoffatom oder gemeinsam für eine zusätzliche Bindung stehen.

7. Verbindungen der allgemeinen Formel I nach Anspruch 1, dadurch gekennzeichnet, daß R²¹ für ein Wasserstoffatom oder einen C₁-C₃-Alkylrest sowie R^{21'} für ein Wasserstoffatom oder eine Hydroxygruppe stehen.

8. Verbindungen der allgemeinen Formel I nach Anspruch 1, dadurch gekennzeichnet, daß der Alkylrest R⁶, R⁷, R¹⁵, R¹⁶, R^{16'}, R^{17¹}, R^{17²}, R²¹ und/oder R^{21'} ein Methyl- oder Ethylrest ist.

9. Verbindungen der allgemeinen Formel I nach Anspruch 1, dadurch gekennzeichnet, daß der C₂-C₄-Alkenylrestes für R¹⁶, R^{16'} und/oder R^{17²} ein Vinylrest ist.

10. Verbindungen der allgemeinen Formel I nach Anspruch 1, nämlich:
14,17-Ethano-19-norpregna-4,9-dien-3,20-dion;
14,17-Ethano-19-norpregna-4,6-dien-3,20-dion;
14,17-Ethano-19-norpregna-4,15-dien-3,20-dion
14,17-Ethano-19-norpregna-4,6,15-trien-3,20-dion
14,17-Ethano-19-norpregna-4,9,15-trien-3,20-dion
21-Methyl-14,17-ethano-19-norpregn-4-en-3,20-dion;
21-Methyl-14,17-ethano-19-norpregna-4,9-dien-3,20-dion;
21-Methyl-14,17-ethano-19-norpregna-4,6-dien-3,20-dion;
21-Methyl-14,17-ethano-19-norpregna-4,15-dien-3,20-dion
21-Methyl-14,17-ethano-19-norpregna-4,9,15-trien-3,20-dion
14,17-Etheno-19-norpregn-4-en-3,20-dion;
14,17-Etheno-19-norpregna-4,6-dien-3,20-dion;
14,17-Etheno-19-norpregna-4,9-dien-3,20-dion;
21-Methyl-14,17-etheno-19-norpregn-4-en-3,20-dion
21-Methyl-14,17-etheno-19-norpregna-4,6-dien-3,20-dion
21-Methyl-14,17-etheno-19-norpregna-4,9-dien-3,20-dion;
21-Methyl-14,17-etheno-19-norpregna-4,9,11-trien-3,20-dion
21-Hydroxy-14,17-etheno-19-norpregn-4-en-3,20-dion
21-Hydroxy-14,17-etheno-19-norpregna-4,9-dien-3,20-dion
17¹-Methyl-14,17-etheno-19-norpregn-4-en-3,20-dion
17¹-Methyl-14,17-etheno-19-norpregna-4,6-dien-3,20-dion
17²-Methyl-14,17-etheno-19-norpregn-4-en-3,20-dion
17²-Methyl-14,17-etheno-19-norpregna-4,9-dien-3,20-dion
15β,16α-Dimethyl-14,17-etheno-19-norpregn-4-en-3,20-dion
6-Methyl-14,17-ethano-19-norpregna-4,6-dien-3,20-dion;
6-Chlor -14,17-ethano-19-norpregna-4,6-dien-3,20-dion;
6α-Methyl-14,17-ethano-19-norpregn-4-en-3,20-dion;
6,21-Dimethyl-14,17-ethano-19-norpregna-4,6-dien-3,20-dion;
15β,16α-Dimethyl-14,17-ethano-19-norpregn-4-en-3,20-dion
6-Chlor-21-methyl-14,17-ethano-19-norpregna-4,6-dien-3,20-dion;
16α-Methyl-14,17-ethano-19-norpregn-4-en-3,20-dion;
16α-Methyl-14,17-ethano-19-norpregna-4,6-dien-3,20-dion;
16α-Methyl-14,17-ethano-19-norpregna-4,9-dien-3,20-dion;
16α,21-Dimethyl-14,17-ethano-19-norpregna-4,9-dien-3,20-dion
21-Hydroxy-16α-methyl-14,17-ethano-19-norpregn-4-en-3,20-dion
16α-Ethyl-14,17-ethano-19-norpregn-4-en-3,20-dion;
16α-Ethenyl-14,17-ethano-19-norpregn-4-en-3,20-dion;
16-Methyl-14,17-ethano-19-norpregna-4,15-dien-3,20-dion
(17¹*R*)-17¹-Methyl-14,17-ethano-19-norpregn-4-en-3,20-dion
(17¹*S*)-17¹-Methyl-14,17-ethano-19-norpregn-4-en-3,20-dion
(17¹*R*)-17¹-Methyl-14,17-ethano-19-norpregna-4,9-dien-3,20-dion
(17¹*S*)-17¹-Methyl-14,17-ethano-19-norpregna-4,9-dien-3,20-dion
(17²*R*)-172-Methyl-14,17-ethano-19-norpregn-4-en-3,20-dion
(17²*R*)-172-Methyl-14,17-ethano-19-norpregna-4,6-dien-3,20-dion
(17²*R*)-172-Methyl-14,17-ethano-19-norpregna-4,9-dien-3,20-dion
(17²*R*)-172,21-Dimethyl-14,17-ethano-19-norpregna-4,6-dien-3,20-dion
(17²*R*)-172,21-Dimethyl-14,17-ethano-19-norpregna-4,9-dien-3,20-dion
(17²*R*)-172,21-Dimethyl-14,17-ethano-19-norpregna-4,9,11-trien-3,20-dion
16-Methylen-14,17-ethano-19-norpregn-4-en-3,20-dion
16-Methylen-14,17-ethano-19-norpregna-4,6-dien-3,20-dion
16-Methylen-14,17-eihano-19-norpregna-4,9-dien-3,20-dion
21-Hydroxy-14,17-ethano-19-norpregn-4-en-3,20-dion;
21-Hydroxy-14,17-ethano-19-norpregna-4,6-dien-3,20-dion;
21-Hydroxy-14,17-ethano-19-norpregna-4,9-dien-3,20-dion;
21-Hydroxy-14,17-ethano-19-norpregna-4,9,15-trien-3,20-dion
(21*R*)-21-Hydroxy-21-methyl-14,17-ethano-19-norpregn-4-en-3,20-dion;
(21*S*)-21-Hydroxy-21-methyl-14,17-ethano-19-norpregn-4-en-3,20-dion;
(21*R*)-21-Hydroxy-21-methyl-14,17-ethano-19-norpregna-4,9-dien-3,20-dion;
(21*S*)-21-Hydroxy-21-methyl-14,17-ethano-19-norpregna-4,9-dien-3 ,20-dion;
(21*R*)-21-Hydroxy-21-methyl-14,17-ethano-19-norpregna-4,6-dien- 3,20-dion;
(21*S*)-21-Hydroxy-21-methyl-14,17-ethano-19-norpregna-4,6-dien- 3,20-dion;
(21*R*)-21-Hydroxy-21-methyl-14,17-ethano-19-norpregna-4,9,15-trien-3,20-dion
(211*S*)-21-Hydroxy-21-methyl-14,17-ethano-19-norpregna-4,9,15-trien-3,20-dion
14,17-Ethano-18a-homo-19-norpregn-4-en-3,20-dion
14,17-Ethano-18a-homo-19-norpregna-4,6-dien-3,20-dion
14,17-Ethano-18a-homo-19-norpregna-4,9-dien-3,20-dion
14,17-Ethano-18a-homo-19-norpregna-4,15-dien-3,20-dion
21-Methyl-14,17-ethano-18a-homo-19-norpregn-4-en-3,20-dion
21-Methyl-14,17-ethano-18a-homo-19-norpregna-4,6-dien-3,20-dion
21-Methyl-14,17-ethano-18a-homo-19-norpregna-4,9-dien-3,20-dion
(21*R*)-21-Hydroxy-21-methyl-14,17-ethano-18a-homo-19-norpregn-4-en-3,20-dion
(21*S*)-21-Hydroxy-21-methyl-14,17-ethano-18a-homo-19-norpregn-4-en-3,20-dion
(21*R*)-21-Hydroxy-21-methyl-14,17-ethano-18a-homo-19-norpregna-4,9-en-3,20-dion
(21*S*)-21-Hydroxy-21-methyl-14,17-ethano-18a-homo-19-norpregna-4,9-en-3,20-dion
(21R)-21-Hydroxy-21-methyl-14,17-ethano-18a-homo-19-norpregna-4,6-en-3,20-dion
(21*S*)-21-Hydroxy-21-methyl-14,17-ethano-18a-homo-19-norpregna-4,6-en-3,20-dion

11. Pharmazeutische Präparate, enthaltend mindestens eine Verbindung der allgemeinen Formel I gemäß Anspruch 1 sowie einen pharmazeutisch verträglichen Träger.

12. Verwendung der Verbindungen der allgemeinen Formel I gemäß Anspruch 1 zur Herstellung von Arzneimitteln.

13. Zwischenprodukte der allgemeinen Formel II 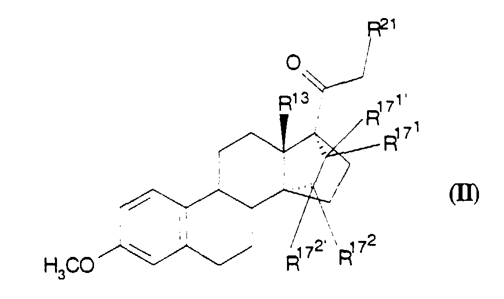 worin
R¹³ = -C₂H₅; R²¹ = Wasserstoff, C₁-C₃-Alkyl oder
R¹³ = -CH₃; R²¹ = C₁-C₃-Alkyl und
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung bedeuten.

14. Zwischenprodukte der allgemeinen Formel III 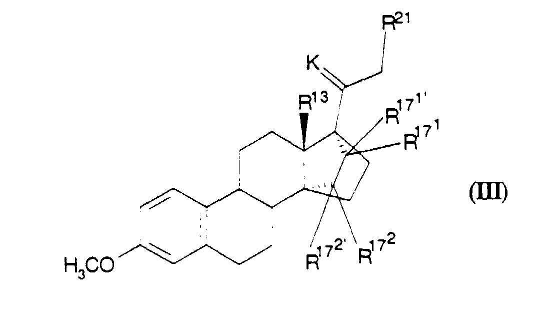 worin
R¹³ = -CH₃, -C₂H₅,
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = eine Ketalschutzgruppe,
R²¹ = Wasserstoff, C₁-C₃-Alkyl
bedeuten.

15. Zwischenprodukte der allgemeinen Formel IV 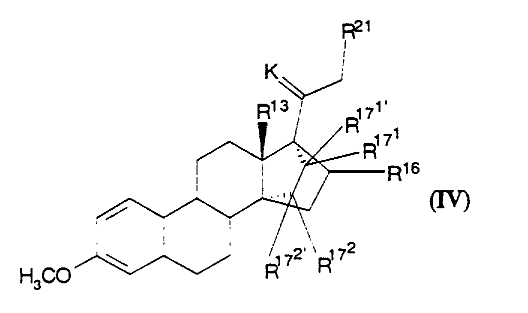 worin
R¹³ = CH₃, -C₂H₅,
R¹⁶ = -COOAlkyl, wobei Alkyl ein C₁-C₄-Alkylrest ist, oder -CH₂OH, oder CHO, oder Methylen,
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff, C₁-C₃-Alkyl
bedeuten.

16. Zwischenprodukte der allgemeinen Formel V 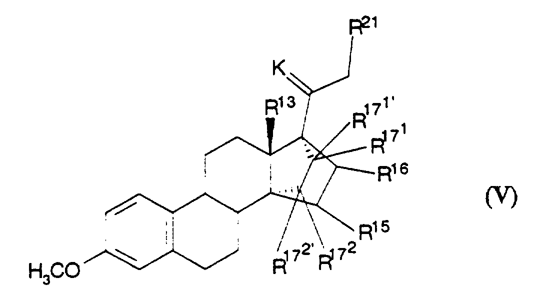 worin
R¹³ = -CH₃, -C₂H₅,
R¹⁵ und R¹⁶ = gemeinsam einen Ring der Teilformeln 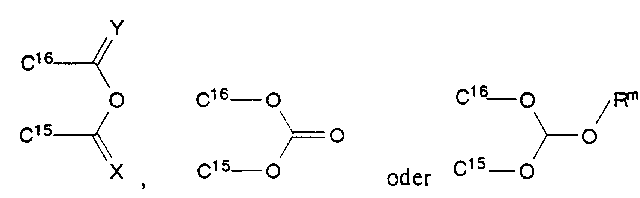 bedeuten,
worin
X und Y = unabhängig voneinander jeweils ein Sauerstoffatom oder zwei Wasserstoffatome und
R^{m} = C₁-C₃-Alkyl
bedeuten, oder
R¹⁵ und R¹⁶ = jedes für sich eine -OH Gruppe oder
R¹⁵ und R¹⁶ = gemeinsam eine Bindung und
R^{17¹} und R^{17²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff oder C₁-C₃-Alkyl
bedeuten.

17. Zwischenprodukte der allgemeinen Formel VI 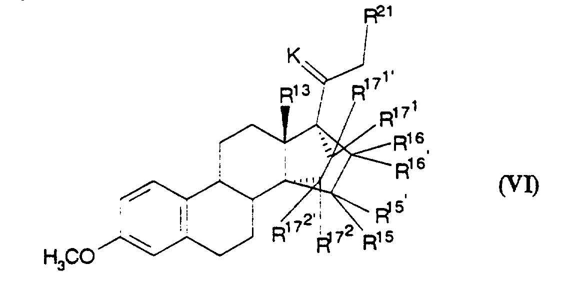 worin
R¹³ = -CH₃, -C₂H₅,
R¹⁵ und R¹⁶ = jeweils Wasserstoff oder gemeinsam eine Bindung,
R^{15'} = Wasserstoff oder C₁-C₃-Alkyl,
R^{16'} = -COOAlkyl, wobei Alkyl ein C₁-C₄-Alkylrest ist, oder CH₂OH, oder CHO, oder einen C₁-C₃-Alkylrest,
R^{17¹} und R1^{7²} = unabhängig voneinander Wasserstoff oder C₁-C₃-Alkyl,
R^{17^{1'}} und R^{17^{2'}} = jeweils Wasserstoff oder gemeinsam eine Bindung,
K = ein Sauerstoffatom oder eine Ketalschutzgruppe,
R²¹ = Wasserstoff oder C₁ - C₃ -Alkyl
bedeuten.

18. Zwischenprodukte der allgemeinen Formel III nach Anspruch 14, worin K für eine 1,2-Ethandiylbis(oxy)- oder 2,2-Dimethyl-1,3-propandiylbis(oxy)gruppe steht.

19. Zwischenprodukte der allgemeinen Formel IV nach Anspruch 15, worin K, wenn es sich um eine Ketalschutzgruppe handelt, für eine 1,2-Ethandiylbis(oxy)- oder 2,2-Dimethyl-1,3-propandiylbis(oxy)gruppe steht.

20. Zwischenprodukte der allgemeinen Formel V nach Anspruch 16, worin K, wenn es sich um eine Ketalschutzgruppe handelt, für eine 1,2-Ethandiylbis(oxy)- oder
2,2-Dimethyl-1,3-propandiylbis(oxy)gruppe steht.

21. Zwischenprodukte der allgemeinen Formel VI nach Anspruch 17, worin K, wenn es sich um eine Ketalschutzgruppe handelt, für eine 1,2-Ethandiylbis(oxy)- oder
2,2-Dimethyl-1,3-propandiylbis(oxy)gruppe steht.

## Claims

1. 14,17-C₂-Bridged steroids of general formula (I), 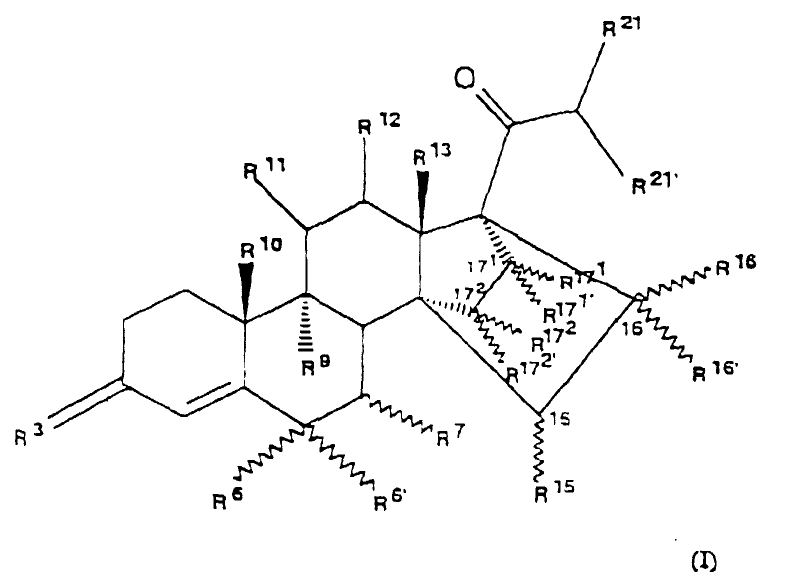 in which
R³ stands for an oxygen atom, the hydroxyimino group or two hydrogen atoms,
R⁶ stands for a hydrogen, fluorine, chlorine or bromine atom or R⁶ stands for a C₁-C₄ alkyl radical in α- or β-position, whereby then R^{6'} and R⁷ represent hydrogen atoms, or else
R⁶ stands for a hydrogen, fluorine, chlorine or bromine atom or R⁶ stands for a C₁-C₄ alkyl radical, whereby then R^{6'} and R⁷ represent a common additional bond,
R⁷ stands for a C₁-C₄ alkyl radical in α- or β-position, whereby then R⁶ and R^{6'} represent hydrogen atoms, or else
R⁶ and R⁷ together stand for a methylene group in α- or β- position and R^{6'} stands for a hydrogen atom or
R⁶ and R^{6'} together stand for an ethylene or methylene group and R⁷ stands for a hydrogen atom,
R⁹ and R¹⁰ each stand for a hydrogen atom or a common bond,
R¹¹ and R¹² each stand for a hydrogen atom or a common bond,
R¹³ stands for a methyl or ethyl group,
R¹⁵ stands for a hydrogen atom or a C₁-C₃ alkyl radical,
R¹⁶ and R^{16'}, independently of one another, stand for a hydrogen atom, a C₁-C₃ alkyl radical or a C₂-C₄ alkenyl radical or together for a C₁-C₃ alkylidene group,
R¹⁵ and R¹⁶ stand for a common bond and R^{16'} stands for a hydrogen atom or a C₁-C₃ alkyl radical or
R¹⁵ and R¹⁶ together stand for a ring of partial formula 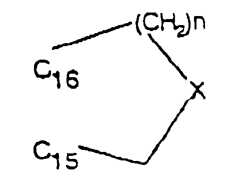 in which n = 1 and 2 and X means a methylene group or an oxygen atom, and R^{16'} stands for a hydrogen atom,
R¹⁷¹ stands for a hydrogen atom or a C₁-C₃ alkyl radical,
R¹⁷² stands for a hydrogen atom, a C₁-C₃ alkyl radical or a C₂-C₄ alkenyl radical,
R¹⁷¹ and R¹⁷²' each stand for a hydrogen atom or for a common bond,
R²¹ stands for a hydrogen atom or a C₁-C₃ alkyl radical,
R^{21'} stands for a hydrogen atom, a C₁-C₃ alkyl radical or a hydroxy group, except for the compound 14,17-ethano-19-norpregn-4-ene-3,20-dione.

2. Compounds of general formula I according to claim 1, characterized in that R³ stands for an oxygen atom or two hydrogen atoms.

3. Compounds of general formula I according to claim 1, wherein R⁶ stands for a hydrogen atom or R° stands for a C₁-C₄ alkyl radical in α- or β-position, if R^{6'} and R⁷ represent hydrogen atoms.

4. Compounds of general formula I according to claim 1, wherein R⁶ stands for a hydrogen, chlorine or bromine atom or R⁶ stands for a C₁-C₄ alkyl radical, if R^{6'} and R⁷ represent a common additional bond.

5. Compounds of general formula I according to claim 1, wherein R¹⁶ and R^{16'} each stand for a hydrogen atom, each for a methyl group or one of these two substituents stands for a C₁-C₄ alkyl group or a vinyl group and the other of these two substituents stands for a hydrogen atom or both substituents together stand for a C₁-C₃ alkylidene group.

6. Compounds of general formula I according to claim 1, wherein R¹⁷¹ stands for a hydrogen atom or a C₁-C₃ alkyl radical and R¹⁷² stands for a hydrogen atom, a C₁-C₃ alkyl radical or a C₂-alkenyl radical and wherein R^{171'} and R^{172'} each stand for a hydrogen atom or together for an additional bond.

7. Compounds of general formula I according to claim 1, wherein R²¹ stands for a hydrogen atom or a C₁-C₃ alkyl radical and R^{21'} stands for a hydrogen atom or a hydroxy group.

8. Compounds of general formula I according to claim 1, wherein alkyl radical R⁶, R⁷, R¹⁵, R¹⁶, R^{16'}, R¹⁷¹, R¹⁷², R²¹ and/or R^{21'} is a methyl or ethyl radical.

9. Compounds of general formula I according to claim 1, wherein the C₂-C₄ alkenyl radical for R¹⁶, R^{16'} and/or R¹⁷² is a vinyl radical.

10. Compounds of general formula I according to claim 1, namely:
14,17-Ethano-19-norpregna-4,9-diene-3,20-dione;
14,17-ethano-19-norpregna-4,6-diene-3,20-dione;
14,17-ethano-19-norpregna-4,15-diene-3,20-dione
14,17-ethano-19-norpregna-4,6,15-triene-3,20-dione
14,17-ethano-19-norpregna-4,9,15-triene-3,20-dione
21-methyl-14,17-ethano-19-norpregn-4-ene-3,20-dione;
21-methyl-14,17-ethano-19-norpregna-4,9-diene-3,20-dione;
21-methyl-14,17-ethano-19-norpregna-4,6-diene-3,20-dione;
21-methyl-14,17-ethano-19-norpregna-4,15-diene-3,20-drone
21-methyl-14,17-ethano-19-norpregna-4,9,15-triene-3,20-dione
14,17-etheno-19-norpregn-4-ene-3,20-dione;
14,17-etheno-19-norpregna-4,6-diene-3,20-dione;
14,17-etheno-19-norpregna-4,9-diene-3,20-dione;
21-methyl-14,17-etheno-19-norpregn-4-ene-3,20-dione
21-methyl-14,17-etheno-19-norpregna-4,6-diene-3,20-dione
21-methyl-14,17-etheno-19-norpregna-4,9-diene-3,20-dione;
21-methyl-14,17-etheno-19-norpregna-4,9,11-triene-3,20-dione
21-hydroxy-14,17-etheno-19-norpregn-4-ene-3,20-dione
21-hydroxy-14,17-etheno-19-norpregna-4,9-diene-3,20-dione
17¹-methyl-14,17-etheno-19-norpregn-4-ene-3,20-dione
17¹-mechyl-14,17-etheno-19-norpregna-4,6-diene-3,20-dione
17²-methyl-14,17-etheno-19-norpregn-4-ene-3,20-dione
17²-methyl-14,17-etheno-19-norpregna-4,9-diene-3,20-dione
15β,16α-dimethyl-14,17-etheno-19-norpregn-4-ene-3,20-dione
6-methyl-14,17-ethano-19-norpregna-4,6-diene-3,20-dione;
6-chloro-14,17-ethano-19-norpregna-4,6-diene-3,20-dione;
6α-methyl-14,17-ethano-19-norpregn-4-ene-3,20-dione;
6,21-dimethyl-14,17-ethano-19-norpregna-4,6-diene-3,20-dione;
15β,16α-dimethyl-14,17-ethano-19-norpregn-4-ene-3,20-dione
6-chloro-21-methyl-14,17-ethano-19-norpregna-4,6-diene-3,20-dione;
16α-methyl-14,17-ethano-19-norpregn-4-ene-3,20-dione;
16α-methyl-14,17-ethano-19-norpregna-4,6-diene-3,20-dione;
16α-methyl-14,17-ethano-19-norpregna-4,9-diene-3,20-dione;
16α,21-dimethyl-14,17-ethano-19-norpregna-4,9-diene-3,20-dione
21-hydroxy-16α-methyl-14,17-ethano-19-norpregn-4-ene-3,20-dione
16α-ethyl-14,17-ethano-19-norpregn-4-ene-3,20-dione;
16α-ethenyl-14,17-ethano-19-norpregn-4-ene-3,20-dione;
16-methyl-14,17-ethano-19-norpregna-4,15-diene-3,20-dione
(17¹R)-17¹-methyl-14,17-ethano-19-norpregn-4-ene-3,20-dione
(17¹S)-17¹-methyl-14,17-ethano-19-norpregn-4-ene-3,20-dione
(17¹R)-17¹-methyl-14,17-ethano-19-norpregna-4,9-diene-3,20-dione
(17¹S)-17¹-methyl-14,17-ethano-19-norpregna-4,9-diene-3,20-dione
(17²R)-17²-methyl-14,17-ethano-19-norpregn-4-ene-3,20-dione
(17²R)-17²-methyl-14,17-ethano-19-norpregna-4,6-diene-3,20-dione
(17²R)-17²-methyl-14,17-ethano-19-norpregna-4,9-diene-3,20-dione
(17²R)-17²,21-dimethyl-14,17-ethano-19-norpregna-4,6-diene-3,20-dione
(17²R)-17²,21-dimethyl-14,17-ethano-19-norpregna-4,9-diene-3,20-dione
(17²R)-17²,21-dimethyl-14,17-ethano-19-norpregna-4,9,11-triene-3,20-dione
16-methylene-14,17-ethano-19-norpregn-4-ene-3,20-dione
16-methylene-14,17-ethano-19-norpregna-4,6-diene-3,20-dione
16-methylene-14,17-ethano-19-norpregna-4,9-diene-3,20-dione
21-hydroxy-14,17-ethano-19-norpregn-4-ene-3,20-dione;
21-hydroxy-14,17-ethano-19-norpregna-4,6-diene-3,20-dione;
21-hydroxy-14,17-ethano-19-norpregna-4,9-diene-3,20-dione;
21-hydroxy-14,17-ethano-19-norpregna-4,9,15-triene-3,20-dione
(21R)-21-hydroxy-21-methyl-14,17-ethano-19-norpregn-4-ene-3,20-dione;
(21S)-21-hydroxy-21-methyl-14,17-ethano-19-norpregn-4-ene-3,20-dione;
(21R)-21-hydroxy-21-methyl-14,17-ethano-19-norpregna-4,9-diene-3,20-dione;
(21S)-21-hydroxy-21-methyl-14,17-ethano-19-norpregna-4,9-diene-3,20-dione;
(21R)-21-hydroxy-21-methyl-14,17-ethano-19-norpregna-4,6-diene-3,20-dione;
(21S)-21-hydroxy-21-methyl-14,17-ethano-19-norpregna-4,6-diene-3,20-dione;
(21R) -21-hydroxy-21-methyl-14,17-ethano-19-norpregna-4,9,15-triene-3,20-dione
(21S)-21-hydroxy-21-methyl-14,17-ethano-19-norpregna-4,9,15-triene-3,20-dione
14,17-ethano-18a-homo-19-norpregn-4-ene-3,20-dione
14,17-ethano-18a-homo-19-norpregna-4,6-diene-3,20-dione
14,17-ethano-18a-homo-19-norpregna-4,9-diene-3,20-dione
14,17-ethano-18a-homo-19-norpregna-4,15-diene-3,20-dione
21-methyl-14,17-ethano-18a-homo-19-norpregn-4-ene-3,20-dione
21-methyl-14,17-ethano-18a-home-19-norpregna-4,6-diene-3,20-dione
21-methyl-14,17-ethano-18a-home-19-norpregna-4,9-diene-3,20-dione
(21R)-21-hydroxy-21-methyl-14,17-ethano-18a-homo-19-norpregn-4-ene-3,20-dione
(21S)-21-hydroxy-21-methyl-14,17-ethano-18a-home-19-norpregn-4-ene-3,20-dione
(21R)-21-hydroxy-21-methyl-14,17-ethano-18a-home-19-norpregna-4,9-ene-3,20-dione
(21S)-21-hydroxy-21-methyl-14,17-ethano-18a-home-19-norpregna-4,9-ene-3,20-dione
(21R)-21-hydroxy-21-methyl-14,17-ethano-18a-homo-19-norpregna-4,6-ene-3,20-dione
(21S)-21-hydroxy-21-methyl-14,17-ethano-18a-homo-19-norpregna-4,6-ene-3,20-dione.

11. Pharmaceutical preparations that contain at least one compound of general formula I according to claim 1 as well as a pharmaceutically compatible vehicle.

12. Use of the compounds of general formula I according to claim 1 for the production of pharmaceutical agents.

13. Intermediate products of general formula II 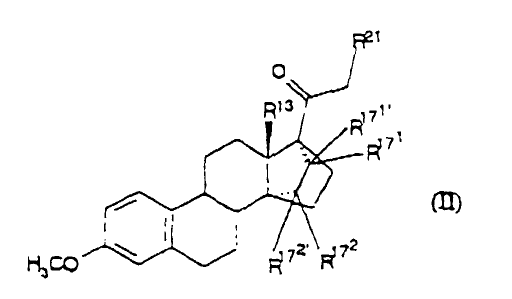 in which
R¹³ = -C₂H₅; R²¹ = hydrogen, C₁-C₃ alkyl or
R¹³ = -CH₃; R²¹ = C₁-C₃ alkyl and
R¹⁷¹ and R¹⁷² = independently of one another, hydrogen or C₁-C₃ alkyl,
R^{171'} and R^{172'} = in each case hydrogen or together a bond.

14. Intermediate products of general formula III 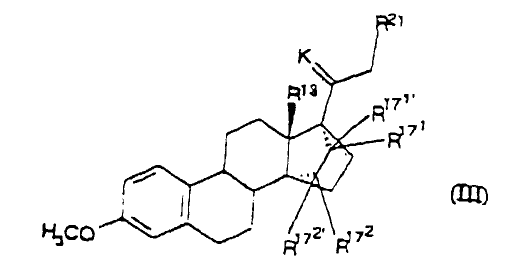 in which
R¹³ = -CH₃, -C₂H₅,
R¹⁷¹ and R¹⁷² = independently of one another, hydrogen or C₁-C₃ alkyl,
R^{171'} and R^{172'} = in each case hydrogen or together a bond,
K = a ketal protective group,
R²¹ = hydrogen, C₁-C₃ alkyl.

15. Intermediate products of general formula IV 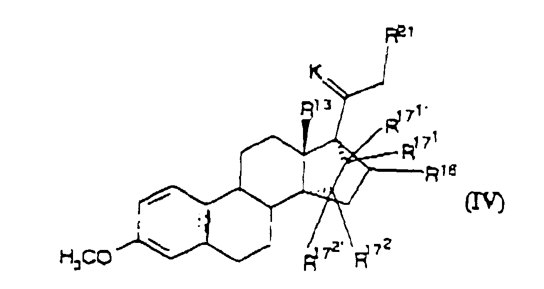 in which
R¹³ = CH₃, -C₂H₅,
R¹⁶ = -COOalkyl, whereby alkyl is a C₁-C₄ alkyl radical, or -CH₂OH or CHO, or methylene,
R¹⁷¹ and R¹⁷² = independently of one another, hydrogen or C₁-C₃ alkyl,
R^{171'} and R^{172'} = in each case hydrogen or together a bond,
K = an oxygen atom or a ketal protective group,
R²¹ = hydrogen, C₁-C₃ alkyl.

16. Intermediate products of general formula V 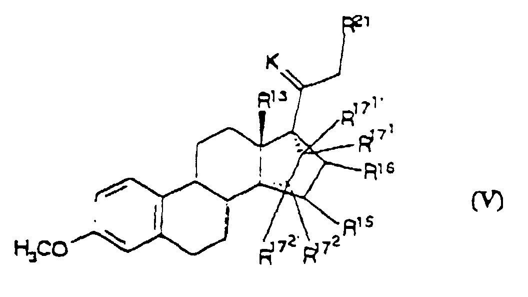 in which
R¹³ = -CH₃, -C₂H₅,
R¹⁵ and R¹⁶ = together a ring of partial formulas 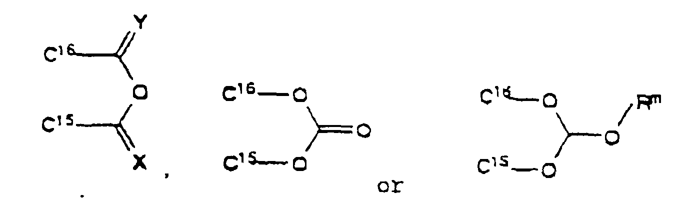 in which
X and Y = independently of one another, in each case an oxygen atom or two hydrogen atoms and
R^{m} = C₁-C₃ alkyl
or
R¹⁵ and R¹⁶ = each per se for an -OH group or
R¹⁵ and R¹⁶ = together a bond
and
R¹⁷¹ and R¹⁷² = independently of one another, hydrogen or C₁-C₃ alkyl,
R^{171'} and R^{172'} = in each case hydrogen or together a bond,
K = an oxygen atom or a ketal protective group,
R²¹ = hydrogen or C₁-C₃ alkyl.

17. Intermediate products of general formula VI 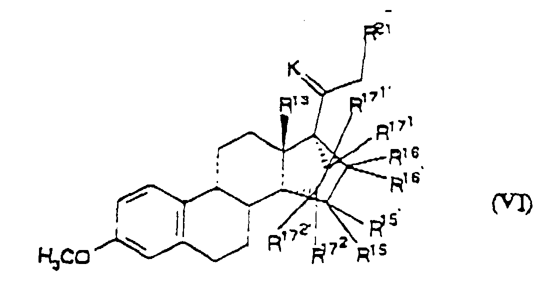 in which
R¹³ = -CH₃, -C₂H₅,
R¹⁵ and R¹⁶ = in each case hydrogen or together a bond,
R^{15'} = hydrogen or C₁-C₃ alkyl,
R^{16'} = -COOalkyl, whereby alkyl is a C₁-C₄ alkyl radical, or CH₂OH or CHO or a C₁-C₃ alkyl radical,
R¹⁷¹ and R¹⁷² = independently of one another, hydrogen or C₁-C₃ alkyl,
R^{171'} and R^{172'} = in each case hydrogen or together a bond,
K = an oxygen atom, or a ketal protective group,
R²¹ = hydrogen or C₁-C₃ alkyl.

18. Intermediate products of general formula III according to claim 14, in which K stands for a 1,2-ethanediylbis(oxy) group or 2,2-dimethyl-1,3-propanediylbis(oxy) group.

19. Intermediate products of general formula IV according to claim 15, in which K, if this is a ketal protective group, stands for a 1,2-ethanediylbis(oxy) group or 2,2-dimethyl-1,3-propanediylbis(oxy) group.

20. Intermediate products of general formula V according to claim 16, in which K, if this is a ketal protective group, stands for a 1,2-ethanediylbis(oxy) group or 2,2-dimethyl-1,3-propanediylbis(oxy) group.

21. Intermediate products of general formula VI according to claim 17, in which K, if this is a ketal protective group, stands for a 1,2-ethanediylbis(oxy) group or 2,2-dimethyl-1,3-propanediylbis(oxy) group.

## Revendications

1. Stéroïdes à pontage 14,17-C₂ de formule générale (I), 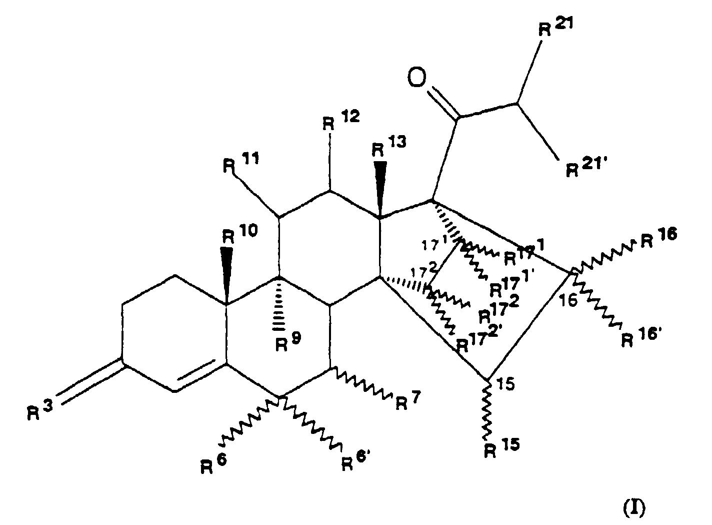 dans laquelle
R³ désigne un atome d'oxygène, le groupe hydroxyimino ou deux atomes d'hydrogène,
R⁶ désigne un atome d'hydrogène, de fluor, de chlore ou de brome ou un radical alkyle en C₁-C₄ en position α ou β, R^{6'} et R⁷ représentant alors des atomes d'hydrogène, ou
R⁶ désigne un atome d'hydrogène, de fluor, de chlore ou de brome ou un radical alkyle en C₁-C₄, R^{6'} et R⁷ représentant alors une liaison commune supplémentaire,
R⁷ désigne un radical alkyle en C₁-C₄ en position α ou β, R⁶ et R^{6'} représentant alors des atomes d'hydrogène, ou
R⁶ et R⁷ désignent conjointement un groupe méthylène en position α ou β et R^{6'} désigne un atome d'hydrogène, ou R⁶ et R^{6'} désignent conjointement un groupe éthylène ou méthylène et R⁷ désigne un atome d'hydrogène,
R⁹ et R¹⁰ désignent chacun un atome d'hydrogène ou une liaison commune,
R¹¹ et R¹² désignent chacun un atome d'hydrogène ou une liaison commune,
R¹³ désigne un groupe méthyle ou éthyle,
R¹⁵ désigne un atome d'hydrogène ou un radical alkyle en C₁-C₃,
R¹⁶ et R^{16'} désignent, indépendamment l'un de l'autre, un atome d'hydrogène, un radical alkyle en C₁-C₃ ou un radical alcényle en C₂-C₄, ou conjointement un groupe alkylidène en C1-C3,
R¹⁵ et R¹⁶ désignent une liaison commune et R^{16'} désigne un atome d'hydrogène, ou un radical en C₁-C₃, ou
R¹⁵ et R¹⁶ désignent conjointement un noyau de formule partielle 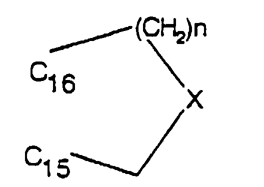 dans laquelle n = 1 et 2, et X représente un groupe méthylène ou un atome d'hydrogène, et
R^{16'} désigne un atome d'hydrogène,
R^{17¹} désigne un atome d'hydrogène ou un radical alkyle en C₁-C₃,
R^{17²} désigne un atome d'hydrogène, un radical alkyle en C₁-C₃, ou un radical alcényle en C₂-C₄,
R^{17^{1'}} et R^{17^{2'}} désignent chacun un atome d'hydrogène ou une liaison commune,
R²¹ désigne un atome d'hydrogène ou un radical alkyle en C₁-C₃,
R^{21'} désigne un atome d'hydrogène, un radical alkyle en C₁-C₃, ou un groupe hydroxy,
à l'exception du composé 14,17-éthano-19-norprégn-4-ène-3,20-dione.

2. Composés de formule générale I selon la revendication 1, caractérisés en ce que R³ désigne un atome d'oxygène ou deux atomes d'hydrogène.

3. Composés de formule générale I selon la revendication 1, caractérisés en ce que R⁶ désigne un atome d'hydrogène ou un radical alkyle en C₁-C₄ en position α ou β, si R^{6'} et R⁷ représentent des atomes d'hydrogène.

4. Composés de formule générale I selon la revendication 1, caractérisés en ce que R⁶ désigne un atome d'hydrogène, de chlore ou de brome ou un radical alkyle en C₁-C_{4,} si R^{6'} et R⁷ représentent une liaison commune supplémentaire.

5. Composés de formule générale I selon la revendication 1, caractérisés en ce que R¹⁶ et R^{16'} désignent chacun un atome d'hydrogène, chacun un groupe méthyle, ou l'un de ces deux substituants désigne un groupe alkyle en C₁-C₄ ou un groupe vinyle, et l'autre de ces deux substituants désigne un atome d'hydrogène, ou les deux substituants désignent conjointement un groupe alkylidène en C₁-C₃.

6. Composés de formule générale I selon la revendication 1, caractérisés en ce que R^{17¹} désigne un atome d'hydrogène ou un radical alkyle en C₁-C₃ et R^{17²} désigne un atome d'hydrogène, un radical alkyle en C₁-C₃ ou un radical alcényle en C₂-C₄, et en ce que R^{17^{1'}} et R^{17^{2'}} désignent chacun un atome d'hydrogène ou conjointement une liaison supplémentaire.

7. Composés de formule générale I selon la revendication 1, caractérisés en ce que R²¹ désigne un atome d'hydrogène ou un radical alkyle en C₁-C₃ et R^{21'} désigne un atome d'hydrogène ou un groupe hydroxy.

8. Composés de formule générale I selon la revendication 1, caractérisés en ce que les radicaux alkyle R⁶, R⁷, R¹⁵, R¹⁶, R^{16'}, R^{17¹}, R^{17²}, R²¹ et/ou R^{21'} sont un radical méthyle ou éthyle.

9. Composés de formule générale I selon la revendication 1, caractérisés en ce que le radical alcényle en C₂-C₄ pour R¹⁶, R^{16'} et/ou R^{17²} est un radical vinyle.

10. Composés de formule générale I selon la revendication 1, à savoir :
la 14,17-éthano-19-norprégna-4,9-diène-3,20-dione ;
la 14,17-éthano-19-norprégna-4,6-diène-3,20-dione ;
la 14,17-éthano-19-norprégna-4,15-diène-3,20-dione ;
la 14,17-éthano-19-norprégna-4,6,15-diène-3,20-dione ;
la 14,17-éthano-19-norprégna-4,9,15-diène-3,20-dione ;
la 21-méthyl-14,17-éthano-19-norprégna-4-ène-3,20-dione ;
la 21-méthyl-14,17-éthano-19-norprégn-4,9-diène-3,20-dione ;
la 21-méthyl-14,17-éthano-19-norprégna-4,6-diène-3,20-dione ;
la 21-méthyl-14,17-éthano-19-norprégna-4,15-diène-3,20-dione ;
la 21-méthyl-14,17-éthano-19-norprégna-4,9,15-triène-3,20-dione ;
la 14,17-éthéno-19-norprégna-4-ène-3,20-dione ;
la 14,17-éthéno-19-norprégna-4,6-diène-3,20-dione ;
la 14,17-éthéno-19-norprégna-4,9-diène-3,20-dione ;
la 21-méthyl-14,17-éthéno-19-norprégn-4-ène-3,20-dione ;
la 21-méthyl-14,17-éthéno-19-norprégna-4,6-diène-3,20-dione ;
la 21-méthyl-14,17-éthéno-19-norprégna-4,9-diène-3,20-dione ;
la 21-méthyl-14,17-éthéno-19-norprégna-4,9,11-triène-3,20-dione ;
la 21-hydroxy-14,17-éthéno-19-norprégn-4-ène-3,20-dione ;
la 21-hydroxy-14,17-éthéno-19-norprégna-4,9-diène-3,20-dione ;
la 17¹-méthyl-14,17-éthéno-19-norprégn-4-ène-3,20-dione ;
la 17¹-méthyl-14,17-éthéno-19-norprégna-4,6-diène-3,20-dione ;
la 17²-méthyl-14,17-éthéno-19-norprégn-4-ène-3,20-dione ;
la 17²-méthyl-14,17-éthéno-19-norprégna-4,9-dièn-3,20-dione ;
la 15β,16α-diméthyl-14,17-éthano-19-norprégn-4-ène-3,20-dione ;
la 6-méthyl-14,17-éthano-19-norprégna-4,6-diène-3,20-dione ;
la 6-chloro-14,17-éthano-19-norprégna-4,6-diène-3,20-dione ;
la 6α-méthyl-14,17-éthano-19-norprégn-4-ène-3,20-dione ;
la 6,21-diméthyl-14,17-éthano-19-norprégna-4,6-diène-3,20-dione ;
la 15β,16α-diméthyl-14,17-éthano-19-norprégn-4-ène-3,20-dione ;
la 6-chloro-21-méthyl-14,17-éthano-19-norprégna-4,6-diène-3,20-dione ;
la 16α-méthyl-14,17-éthano-19-norprégn-4-ène-3,20-dione ;
la 16α-méthyl-14,17-éthano-19-norprégna-4,6-diène-3,20-dione ;
la 16α-méthyl-14,17-éthano-19-norprégna-4,9-diène-3,20-dione ;
la 16α,21-diméthyl-14,17-éthano-19-norprégna-4,9-diène-3,20-dione ;
la 21-hydroxy-16α-méthyl-14,17-éthano-19-norprégn-4-ène-3,20-dione ;
la 16α-éthyl-14,17-éthano-19-norprégn-4-ène-3,20-dione ;
la 16α-éthényl-14,17-éthano-19-norprégna-4-ène-3,20-dione ;
la 16-méthyl-14,17-éthano-19-norprégna-4,15-diène-3,20-dione ;
la (17¹*R*)-17¹-méthyl-14,17-éthano-19-norprégn-4-ène-3,20-dione ;
la (17¹*S*)-17¹-méthyl-14,17-éthano-19-norprégn-4-ène-3,20-dione ;
la (17¹*R*)-17¹-méthyl-14,17-éthano-19-norprégna-4,9-diène-3,20-dione ;
la (17¹*S*)-17¹-méthyl-14,17-éthano-19-norprégna-4,9-diène-3,20-dione ;
la (17²*R*)-17²-méthyl-14,17-éthano-19-norprégn-4-ène-3,20-dione ;
la (17²*R*)-17²-méthyl-14,17-éthano-19-norprégna-4,6-diène-3,20-dione ;
la (17²*R*)-17²-méthyl-14,17-éthano-19-norprégna-4,9-diène-3,20-dione ;
la (17²*R*)-17²,21-diméthyl-14,17-éthano-19-norprégna-4,6-diène-3,20-dione ;
la (17²*R*)-17²,21-diméthyl-14,17-éthano-19-norprégna-4,9-diène-3,20-dione ;
la (17²*R*)-17²,21-diméthyl-14,17-éthano-19-norprégna-4,9,11-triène-3,20-dione ;
la 16-méthylène-14,17-éthano-19-norprégn-4-ène-3,20-dione ;
la 16-méthylène-14,17-éthano-19-norprégna-4,6-diène-3,20-dione ;
la 16-méthylène-14,17-éthano-19-norprégna-4,9-diène-3,20-dione ;
la 21-hydroxy-14,17-éthano-19-norprégn-4-ène-3,20-dione ;
la 21-hydroxy-14,17-éthano-19-norprégna-4,6-diène-3,20-dione ;
la 21-hydroxy-14,17-éthano-19-norprégna-4,9-diène-3,20-dione ;
la 21-hydroxy-14,17-éthano-19-norprégna-4,9,15-triène-3,20-dione ;
la (21*R*)-21-hydroxy-21-méthyl-14,17-éthano-19-norprégn-4-ène-3,20-dione ;
la (21*S*)-21-hydroxy-21-méthyl-14,17-éthano-19-norprégn-4-ène-3,20-dione ;
la (21*R*)-21-hydroxy-21-méthyl-14,17-éthano-19-norprégna-4,9-diène-3,20-dione ;
la (21*S*)-21-hydroxy-21-méthyl-14,17-éthano-19-norprégna-4,9-diène-3,20-dione ;
la (21*R*)-21-hydroxy-21-méthyl-14,17-éthano-19-norprégna-4,6-diène-3,20-dione ;
la (21*S*)-21-hydroxy-21-méthyl-14,17-éthano-19-norprégna-4,6-diène-3,20-dione ;
la (21*R*) -21-hydroxy-21-méthyl-14,17-éthano-19-norprégna-4,9,15-triène-3,20-dione ;
la (21*S*)-21-hydroxy-21-méthyl-14,17-éthano-19-norprégna-4,9,15-triène-3,20-dione ;
la 14,17-éthano-18a-homo-19-norprégn-4-ène-3,20-dione ;
la 14,17-éthano-18a-homo-19-norprégna-4,6-diène-3,20-dione ;
la 14,17-éthano-18a-homo-19-norprégna-4,9-diène-3,20-dione ;
la 14,17-éthano-18a-homo-19-norprégna-4,15-diène-3,20-dione ;
la 21-méthyl-14,17-éthano-18a-homo-19-norprégn-4-ène-3,20-dione ;
la 21-méthyl-14,17-éthano-18a-homo-19-norprégna-4,6-diène-3,20-dione ;
la 21-méthyl-14,17-éthano-18a-homo-19-norprégna-4,9-diène-3,20-dione ;
la (21*R*)-21-hydroxy-21-méthyl-14,17-éthano-18a-homo-19-norprégn-4-ène-3,20-dione ;
la (21*S*)-21-hydroxy-21-méthyl-14,17-éthano-18a-homo-19-norprégn-4-ène-3,20-dione ;
la (21*R*)-21-hydroxy-21-méthyl-14,17-éthano-18a-homo-19-norprégna-4,9-ène-3,20-dione ;
la (21*S*)-21-hydroxy-21-méthyl-14,17-éthano-18a-homo-19-norprégna-4,9-ène-3,20-dione ;
la (21*R*)-21-hydroxy-21-méthyl-14,17-éthano-18a-homo-19-norprégna-4,6-ène-3,20-dione ;
la (21*S*)-21-hydroxy-21-méthyl-14,17-éthano-18a-homo-19-norprégn-4,6-ène-3,20-dione.

11. Préparations pharmaceutiques contenant au moins un composé de formule générale I selon la revendication 1 ainsi qu'un support pharmaceutiquement acceptable.

12. Utilisation des composés de formule générale I selon la revendication 1 pour la préparation de médicaments.

13. Intermédiaires de formule générale II 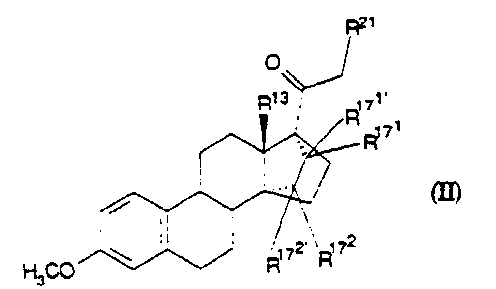 dans laquelle
R¹³ = -C₂H₅ ; R²¹ = un hydrogène, un alkyle en C₁-C₃, ou R¹³ = -CH₃ ; R²¹ = un alkyle en C₁-C₃, et
R^{17¹} et R^{17²} = indépendamment l'un de l'autre, un hydrogène ou un alkyle en C₁-C₃,
R^{17^{1'}} et R^{17^{2'}} = chacun un hydrogène ou conjointement une liaison.

14. Intermédiaires de formule générale III 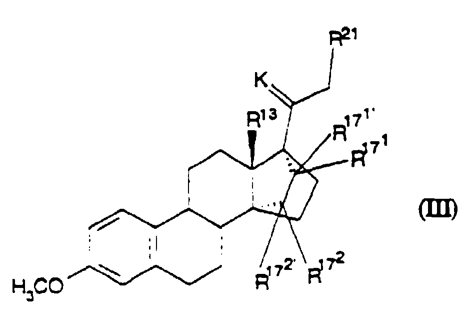 dans laquelle
R¹³ = -CH₃, -C₂H₅
R^{17¹} et R^{17²} = indépendamment l'un de l'autre, un hydrogène ou un alkyle en C₁-C₃,
R^{17^{1'}} et R^{17^{2'}} = chacun un hydrogène ou conjointement une liaison,
K = un groupe protecteur de cétal,
R²¹ = un hydrogène, un alkyle en C₁-C₃.

15. Intermédiaires de formule générale IV 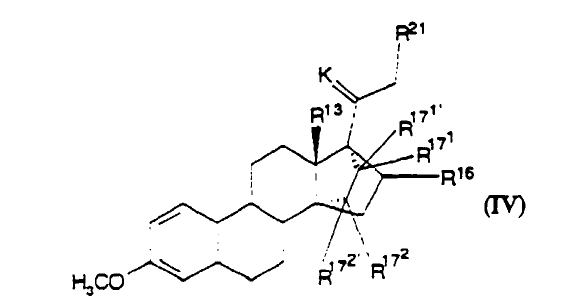 dans laquelle
R¹³ = -CH₃, -C₂H₅
R¹⁶ = -COOalkyle, où l'alkyle est un radical alkyle en C₁-C₄, ou -CH₂OH ou CHO ou un méthylène,
R^{17¹} et R^{17²} = indépendamment l'un de l'autre, un hydrogène ou un alkyle en C₁-C₃,
R^{17^{1'}} et R^{17^{2'}} = chacun un hydrogène ou conjointement une liaison,
K = un atome d'oxygène ou un groupe protecteur de cétal,
R²¹ = un hydrogène, un alkyle en C₁-C₃.

16. Intermédiaires de formule générale V 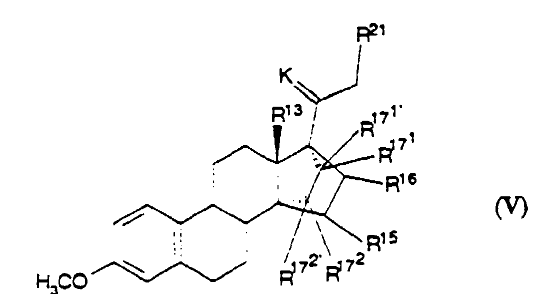 dans laquelle
R¹³ = -CH₃, -C₂H₅
R¹⁵ et R¹⁶ = conjointement un noyau de formules partielles 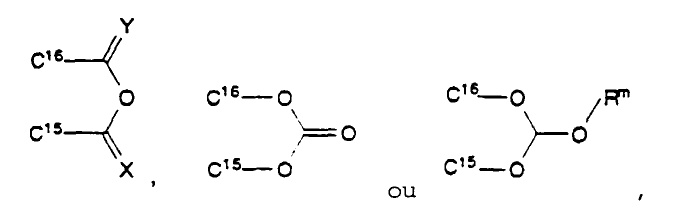 dans lesquelles
X et Y = chacun, indépendamment l'un de l'autre, un atome d'oxygène ou deux atomes d'hydrogène, et
R^{m} = un alkyle en C₁-C₃,
ou
R¹⁵ et R¹⁶ = chacun un groupe -OH, ou
R¹⁵ et R¹⁶ = conjointement une liaison,
et
R^{17¹} et R^{17²} = indépendamment l'un de l'autre, un hydrogène ou un alkyle en C₁-C₃,
R^{17^{1'}} et R^{17^{2'}} = chacun un hydrogène ou conjointement une liaison,
K = un atome d'oxygène ou un groupe protecteur de cétal, R²¹ = un hydrogène ou un alkyle en C₁-C₃.

17. Intermédiaires de formule générale VI 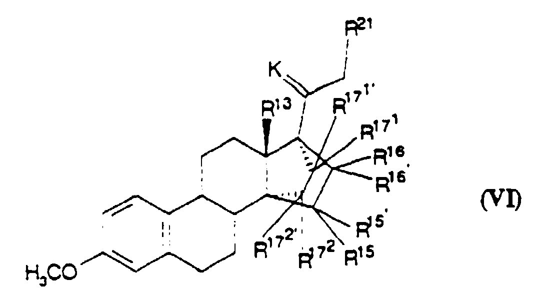 dans laquelle
R¹³ = -CH₃, -C₂H₅
R¹⁵ et R¹⁶ = chacun un hydrogène ou conjointement une liaison,
R¹⁵ = un hydrogène ou un alkyle en C₁-C₃,
R^{16'}= un -COOalkyle, où l'alkyle est un radical alkyle en C₁-C₄, ou CH₂OH, ou CHO, ou un radical alkyle en C₁-C₃,
R^{17¹} et R^{17²} = indépendamment l'un de l'autre, un hydrogène ou un alkyle en C₁-C₃,
R^{17^{1'}} et R^{17^{2'}} = chacun un hydrogène ou conjointement une liaison,
K = un atome d'oxygène ou un groupe protecteur de cétal,
R²¹ = un hydrogène ou un alkyle en C₁-C₃.

18. Intermédiaires de formule générale III selon la revendication 14, dans laquelle K désigne un groupe 1,2-éthanediylbis(oxy) ou 2,2-diméthyl-1,3-propanediylbis(oxy).

19. Intermédiaires de formule générale IV selon la revendication 15, dans laquelle K désigne, lorsqu'il s'agit d'un groupe protecteur de cétal, un groupe 1,2-éthanediylbis(oxy) ou 2,2-diméthyl-1,3-propanediylbis(oxy).

20. Intermédiaires de formule générale V selon la revendication 16, dans laquelle K désigne, lorsqu'il s'agit d'un groupe protecteur de cétal, un groupe 1,2-éthanediylbis(oxy) ou 2,2-diméthyl-1,3-propanediylbis(oxy).

21. Intermédiaires de formule générale VI selon la revendication 17, dans laquelle K désigne, lorsqu'il s'agit d'un groupe protecteur de cétal, un groupe 1,2-éthanediylbis(oxy) ou 2,2-diméthyl-1,3-propanediylbis (oxy).
